(19) **Europäisches Patentamt / European Patent Office / Office européen des brevets**

(11) **EP 3 221 469 B1**

(12) **EUROPEAN PATENT SPECIFICATION**

(45) Date of publication and mention of the grant of the patent:
**15.01.2020 Bulletin 2020/03**

(21) Application number: **15801655.0**

(22) Date of filing: **19.11.2015**

(51) Int Cl.:
*C12Q 1/6869* (2018.01)

(86) International application number:
**PCT/US2015/061615**

(87) International publication number:
**WO 2016/081740 (26.05.2016 Gazette 2016/21)**

(54) **ENZYME- AND AMPLIFICATION-FREE SEQUENCING**

ENZYM- UND AMPLIFIKATIONSFREIE SEQUENZIERUNG

SÉQUENÇAGE SANS ENZYME NI AMPLIFICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.11.2014 US 201462082883 P**

(43) Date of publication of application:
**27.09.2017 Bulletin 2017/39**

(73) Proprietor: **Nanostring Technologies, Inc Seattle, WA 98109 (US)**

(72) Inventors:
• **BEECHEM, Joseph M.**
**Eugene, Oregon 97405 (US)**
• **KHAFIZOV, Rustem**
**Seattle, Washington 98109 (US)**

(74) Representative: **Cooley (UK) LLP**
**Dashwood**
**69 Old Broad Street**
**London EC2M 1QS (GB)**

(56) References cited:
**WO-A2-2013/055995   US-A1- 2014 005 067**
**US-B1- 7 745 129**

SEQUENCE LISTING

<110> NanoString Technologies, Inc. BEECHEM, Joseph KHAFIZOV, Rustem

<120> ENZYME- AND AMPLIFICATION-FREE SEQUENCING

<130> NATE-025/001

<150> US 62/082,883
<151> 2014-11-21

<160> 104

<170> Patent In version 3.5

<210> 1
<211> 10
<212> DNA

<213> Artificial Sequence

<220>
<223> Synthetic Polynucleotide

<400> 1
atacatctag          10

<210> 2
<211> 10
<212> DNA
<213> Artificial Sequence

<220>
<223> Synthetic Polynucleotide

<400> 2
gatctacata          10

<210> 3
<211> 10
<212> DNA
<213> Artificial Sequence

<220>
<223> Synthetic Polynucleotide

<400> 3
ttaggtaaag          10

<210> 4
<211> 10
<212> DNA
<213> Artificial Sequence

<220>
<223> Synthetic Polynucleotide

<400> 4
tcttcattac          10

<210> 5
<211> 10
<212> DNA
<213> Artificial Sequence

<220>
<223> Synthetic Polynucleotide

<400> 5
atgaatctac          10

<210> 6
<211> 10
<212> DNA
<213> Artificial Sequence

<220>

<223> Synthetic Polynucleotide

<400> 6
tcaatgtatg          10

<210> 7
<211> 10
<212> DNA
<213> Artificial Sequence

<220>
<223> Synthetic Polynucleotide

<400> 7
aattgagtac          10

<210> 8
<211> 10
<212> DNA
<213> Artificial Sequence

<220>
<223> Synthetic Polynucleotide

<400> 8
atgttaatgg          10

<210> 9
<211> 10
<212> DNA
<213> Artificial Sequence

<220>
<223> Synthetic Polynucleotide

<400> 9
aattaggatg          10

<210> 10
<211> 10
<212> DNA
<213> Artificial Sequence

<220>
<223> Synthetic Polynucleotide

<400> 10
ataatggatc          10

<210> 11
<211> 10
<212> DNA
<213> Artificial Sequence

<220>
<223> Synthetic Polynucleotide

<400> 11

taataaggtg          10

<210> 12
<211> 10
<212> DNA
<213> Artificial Sequence

<220>
<223> Synthetic Polynucleotide

<400> 12
tagttagagc          10

<210> 13
<211> 10
<212> DNA
<213> Artificial Sequence

<220>
<223> Synthetic Polynucleotide

<400> 13
atagagaagg          10

<210> 14
<211> 10
<212> DNA
<213> Artificial Sequence

<220>
<223> Synthetic Polynucleotide

<400> 14
ttgatgatac          10

<210> 15
<211> 10
<212> DNA
<213> Artificial Sequence

<220>
<223> Synthetic Polynucleotide

<400> 15
atagtgattc          10

<210> 16
<211> 10
<212> DNA
<213> Artificial Sequence

<220>
<223> Synthetic Polynucleotide

<400> 16
tataacgatg          10

<210> 17

<211> 10
<212> DNA
<213> Artificial Sequence

<220>
<223> Synthetic Polynucleotide

<400> 17
ttaagtttag          10

<210> 18
<211> 10
<212> DNA
<213> Artificial Sequence

<220>
<223> Synthetic Polynucleotide

<400> 18
atacgttatg          10

<210> 19
<211> 10
<212> DNA
<213> Artificial Sequence

<220>
<223> Synthetic Polynucleotide

<400> 19
tgtactatag          10

<210> 20
<211> 10
<212> DNA
<213> Artificial Sequence

<220>
<223> Synthetic Polynucleotide

<400> 20
ttaacaagtg          10

<210> 21
<211> 10
<212> DNA
<213> Artificial Sequence

<220>
<223> Synthetic Polynucleotide

<400> 21
aactatgtac          10

<210> 22
<211> 10
<212> DNA
<213> Artificial Sequence

<220>
<223> Synthetic Polynucleotide

<400> 22
taactatgac          10

<210> 23
<211> 10
<212> DNA
<213> Artificial Sequence

<220>
<223> Synthetic Polynucleotide

<400> 23
actaatgttc          10

<210> 24
<211> 10
<212> DNA
<213> Artificial Sequence

<220>
<223> Synthetic Polynucleotide

<400> 24
tcattgaatg          10

<210> 25
<211> 14
<212> DNA
<213> Artificial Sequence

<220>
<223> Synthetic Polynucleotide

<400> 25
ctgtctcatc tctt          14

<210> 26
<211> 26
<212> DNA
<213> Artificial Sequence

<220>
<223> Synthetic Polynucleotide

<400> 26
ctgtctcatc tcttgctgca tcctgt          26

<210> 27
<211> 38
<212> DNA
<213> Artificial Sequence

<220>
<223> Synthetic Polynucleotide

<400> 27
ctgtctcatc tcttgctgca tcctgtcggt tcacgttg          38


<210> 28
<211> 36
<212> DNA
<213> Artificial Sequence


<220>
<223> Synthetic Polynucleotide


<400> 28
ctgtctcatc ttgctgcatc ctgtcggttc acgttg          36


<210> 29
<211> 36
<212> DNA
<213> Artificial Sequence


<220>
<223> Synthetic Polynucleotide


<400> 29
ctgtctcatt ttgctgcatc ctgtccgttc acgttg          36


<210> 30
<211> 43
<212> DNA
<213> Artificial Sequence


<220>
<223> Synthetic Polynucleotide


<400> 30
gactgtaccc acgcgatgac gttcgtcaag agtcgcataa tct          43


<210> 31
<211> 44
<212> DNA
<213> Artificial Sequence


<220>
<223> Synthetic Polynucleotide


<400> 31
agactgtacc acaagaatcc ctgctagctg aaggagggtc aaac          44


<210> 32
<211> 45
<212> DNA
<213> Artificial Sequence


<220>
<223> Synthetic Polynucleotide


<400> 32
gagactgtac cctacgtata tatccaagtg gttatgtccg acggc          45

<210> 33
<211> 46
<212> DNA
<213> Artificial Sequence

<220>
<223> Synthetic Polynucleotide

<400> 33
tgagactgta ccacccctcc aaacgcattc ttattggcaa atggaa        46

<210> 34
<211> 47
<212> DNA
<213> Artificial Sequence

<220>
<223> Synthetic Polynucleotide

<400> 34
ctgagactgt acccgggaat cggcatttcg cattcttagg atctaaa        47

<210> 35
<211> 44
<212> DNA
<213> Artificial Sequence

<220>
<223> Synthetic Polynucleotide

<400> 35
caatgtgagt ctcttggtac agtctcagtt agtcactccc taag        44

<210> 36
<211> 45
<212> DNA
<213> Artificial Sequence

<220>
<223> Synthetic Polynucleotide

<400> 36
gagacagtac cctggtctag gtatctaatt cgtgggtcgg gtact        45

<210> 37
<211> 45
<212> DNA
<213> Artificial Sequence

<220>
<223> Synthetic Polynucleotide

<400> 37
gagaccgtac cgctcatttt gaacatacga ttgcgattac ggaaa        45

<210> 38
<211> 45
<212> DNA

<213> Artificial Sequence

<220>
<223> Synthetic Polynucleotide

<400> 38
gagacggtac cttaaagcta tccacgaatg tcaaaaatgt ggttt        45

<210> 39
<211> 45
<212> DNA
<213> Artificial Sequence

<220>
<223> Synthetic Polynucleotide

<400> 39
gagagtgtac ccaatgcttg cagtatgtat cctgatcgtg cgtgc        45

<210> 40
<211> 45
<212> DNA
<213> Artificial Sequence

<220>
<223> Synthetic Polynucleotide

<400> 40
gagaatgtac cctcatacca atgtaaagta tagttaacgc cctgt        45

<210> 41
<211> 45
<212> DNA
<213> Artificial Sequence

<220>
<223> Synthetic Polynucleotide

<400> 41
gagattgtac cctacatata taggaaaagg gaaggtagaa gagct        45

<210> 42
<211> 12
<212> DNA
<213> Artificial Sequence

<220>
<223> Synthetic Polynucleotide

<400> 42
cacgaacgtc ag        12

<210> 43
<211> 12
<212> DNA
<213> Artificial Sequence

<220>

<223> Synthetic Polynucleotide

<400> 43
catcgcatgc ct          12

<210> 44
<211> 12
<212> DNA
<213> Artificial Sequence

<220>
<223> Synthetic Polynucleotide

<400> 44
gtcatctcct ac          12

<210> 45
<211> 12
<212> DNA
<213> Artificial Sequence

<220>
<223> Synthetic Polynucleotide

<400> 45
gtcatccgct ac          12

<210> 46
<211> 12
<212> DNA
<213> Artificial Sequence

<220>
<223> Synthetic Polynucleotide

<400> 46
gtcatcgact ac          12

<210> 47
<211> 12
<212> DNA
<213> Artificial Sequence

<220>
<223> Synthetic Polynucleotide

<400> 47
gtcatcttct ac          12

<210> 48
<211> 12
<212> DNA
<213> Artificial Sequence

<220>
<223> Synthetic Polynucleotide

<400> 48

gtcatcacct ac        12

<210> 49
<211> 12
<212> DNA
<213> Artificial Sequence

<220>
<223> Synthetic Polynucleotide

<400> 49
gtcatcactc ac        12

<210> 50
<211> 12
<212> DNA
<213> Artificial Sequence

<220>
<223> Synthetic Polynucleotide

<400> 50
gtcatcttcg ac        12

<210> 51
<211> 12
<212> DNA
<213> Artificial Sequence

<220>
<223> Synthetic Polynucleotide

<400> 51
gtcatcaact ac        12

<210> 52
<211> 12
<212> DNA
<213> Artificial Sequence

<220>
<223> Synthetic Polynucleotide

<400> 52
gtcatccgta ac        12

<210> 53
<211> 12
<212> DNA
<213> Artificial Sequence

<220>
<223> Synthetic Polynucleotide

<400> 53
gtcatccgaa ac        12

<210> 54

EP 3 221 469 B1

<211> 12
<212> DNA
<213> Artificial Sequence

<220>
<223> Synthetic Polynucleotide

<400> 54
gtcatcacaa ac          12

<210> 55
<211> 12
<212> DNA
<213> Artificial Sequence

<220>
<223> Synthetic Polynucleotide

<400> 55
gtcatcttgc ac          12

<210> 56
<211> 12
<212> DNA
<213> Artificial Sequence

<220>
<223> Synthetic Polynucleotide

<400> 56
gtcatcttgc ct          12

<210> 57
<211> 12
<212> DNA
<213> Artificial Sequence

<220>
<223> Synthetic Polynucleotide

<400> 57
gtcatccgtc ct          12

<210> 58
<211> 12
<212> DNA
<213> Artificial Sequence

<220>
<223> Synthetic Polynucleotide

<400> 58
cttttcacct ct          12

<210> 59
<211> 12
<212> DNA
<213> Artificial Sequence

32

<220>
<223> Synthetic Polynucleotide

<400> 59
cttttcctct ct          12

<210> 60
<211> 12
<212> DNA
<213> Artificial Sequence

<220>
<223> Synthetic Polynucleotide

<400> 60
cttttcgact ct          12

<210> 61
<211> 12
<212> DNA
<213> Artificial Sequence

<220>
<223> Synthetic Polynucleotide

<400> 61
cttttctgct ct          12

<210> 62
<211> 12
<212> DNA
<213> Artificial Sequence

<220>
<223> Synthetic Polynucleotide

<400> 62
cttttctgta ct          12

<210> 63
<211> 12
<212> DNA
<213> Artificial Sequence

<220>
<223> Synthetic Polynucleotide

<400> 63
cttttctgtg ct          12

<210> 64
<211> 12
<212> DNA
<213> Artificial Sequence

<220>
<223> Synthetic Polynucleotide

<400> 64
cttttctgtc ct       12

<210> 65
<211> 12
<212> DNA
<213> Artificial Sequence

<220>
<223> Synthetic Polynucleotide

<400> 65
cttttcactc ct       12

<210> 66
<211> 12
<212> DNA
<213> Artificial Sequence

<220>
<223> Synthetic Polynucleotide

<400> 66
cttttcgttc ct       12

<210> 67
<211> 12
<212> DNA
<213> Artificial Sequence

<220>
<223> Synthetic Polynucleotide

<400> 67
cttttcgtac ct       12

<210> 68
<211> 12
<212> DNA
<213> Artificial Sequence

<220>
<223> Synthetic Polynucleotide

<400> 68
cttttccgtc ct       12

<210> 69
<211> 12
<212> DNA
<213> Artificial Sequence

<220>
<223> Synthetic Polynucleotide

<400> 69
cttttctgac ct       12

<210> 70
<211> 12
<212> DNA
<213> Artificial Sequence

<220>
<223> Synthetic Polynucleotide

<400> 70
aggcatgcga tg            12

<210> 71
<211> 12
<212> DNA
<213> Artificial Sequence

<220>
<223> Synthetic Polynucleotide

<400> 71
aggcattgtg ct            12

<210> 72
<211> 12
<212> DNA
<213> Artificial Sequence

<220>
<223> Synthetic Polynucleotide

<400> 72
aggcattgct ct            12

<210> 73
<211> 12
<212> DNA
<213> Artificial Sequence

<220>
<223> Synthetic Polynucleotide

<400> 73
aggcatttct ac            12

<210> 74
<211> 12
<212> DNA
<213> Artificial Sequence

<220>
<223> Synthetic Polynucleotide

<400> 74
aggcatacct ac            12

<210> 75
<211> 12
<212> DNA

<213> Artificial Sequence

<220>
<223> Synthetic Polynucleotide

<400> 75
aggcatttgc ac        12

<210> 76
<211> 12
<212> DNA
<213> Artificial Sequence

<220>
<223> Synthetic Polynucleotide

<400> 76
aggcatcgtc ct        12

<210> 77
<211> 12
<212> DNA
<213> Artificial Sequence

<220>
<223> Synthetic Polynucleotide

<400> 77
tcctgtcggt tc        12

<210> 78
<211> 12
<212> DNA
<213> Artificial Sequence

<220>
<223> Synthetic Polynucleotide

<400> 78
gttcaatgct ct        12

<210> 79
<211> 12
<212> DNA
<213> Artificial Sequence

<220>
<223> Synthetic Polynucleotide

<400> 79
attcggtgct ct        12

<210> 80
<211> 12
<212> DNA
<213> Artificial Sequence

<220>

<223> Synthetic Polynucleotide

<400> 80
gatgcctgct ct          12

<210> 81
<211> 12
<212> DNA
<213> Artificial Sequence

<220>
<223> Synthetic Polynucleotide

<400> 81
tttgcttgct ct          12

<210> 82
<211> 100
<212> DNA
<213> Artificial Sequence

<220>
<223> Synthetic Polynucleotide

<400> 82

```
ttcactgtag ctgtctcatt ttgctgcatc ctgtccgttc acgttggagc ttgtcatccg          60

tcctcttttc actcctaggc atttgcctat tcggcgtcct                                100
```

<210> 83
<211> 10
<212> DNA
<213> Artificial Sequence

<220>
<223> Synthetic Polynucleotide

<400> 83
cgatctggtt          10

<210> 84
<211> 10
<212> DNA
<213> Artificial Sequence

<220>
<223> Synthetic Polynucleotide

<400> 84
cgatctggtt          10

<210> 85
<211> 10
<212> DNA
<213> Artificial Sequence

<220>
<223> Synthetic Polynucleotide

<400> 85
gctagaccaa          10


<210> 86
<211> 10
<212> DNA
<213> Artificial Sequence


<220>
<223> Synthetic Polynucleotide

<400> 86
gctggaccaa          10


<210> 87
<211> 10
<212> DNA
<213> Artificial Sequence


<220>
<223> Synthetic Polynucleotide

<400> 87
gctcgaccaa          10


<210> 88
<211> 10
<212> DNA
<213> Artificial Sequence


<220>
<223> Synthetic Polynucleotide

<400> 88
gcttgaccaa          10


<210> 89
<211> 10
<212> DNA
<213> Artificial Sequence


<220>
<223> Synthetic Polynucleotide

<400> 89
gagactgtac          10


<210> 90
<211> 10
<212> DNA
<213> Artificial Sequence


<220>
<223> Synthetic Polynucleotide

<400> 90
gagacagtac      10

<210> 91
<211> 10
<212> DNA
<213> Artificial Sequence

<220>
<223> Synthetic Polynucleotide

<400> 91
gagaccgtac      10

<210> 92
<211> 10
<212> DNA
<213> Artificial Sequence

<220>
<223> Synthetic Polynucleotide

<400> 92
gagacggtac      10

<210> 93
<211> 10
<212> DNA
<213> Artificial Sequence

<220>
<223> Synthetic Polynucleotide

<400> 93
gagagtgtac      10

<210> 94
<211> 10
<212> DNA
<213> Artificial Sequence

<220>
<223> Synthetic Polynucleotide

<400> 94
gagaatgtac      10

<210> 95
<211> 10
<212> DNA
<213> Artificial Sequence

<220>
<223> Synthetic Polynucleotide

<400> 95
gagattgtac      10

<210> 96
<211> 10
<212> DNA
<213> Artificial Sequence

<220>
<223> Synthetic Polynucleotide

<400> 96
gagactgtac          10

<210> 97
<211> 10
<212> DNA
<213> Artificial Sequence

<220>
<223> Synthetic Polynucleotide

<400> 97
gagattgtac          10

<210> 98
<211> 10
<212> DNA
<213> Artificial Sequence

<220>
<223> Synthetic Polynucleotide

<400> 98
gagaccgtac          10

<210> 99
<211> 10
<212> DNA
<213> Artificial Sequence

<220>
<223> Synthetic Polynucleotide

<400> 99
gagagtgtac          10

<210> 100
<211> 12
<212> DNA
<213> Artificial Sequence

<220>
<223> Synthetic Polynucleotide

<400> 100
ctgagactgt ac          12

<210> 101
<211> 11
<212> DNA

<213> Artificial Sequence

<220>
<223> Synthetic Polynucleotide

<400> 101
tgagactgta c          11

<210> 102
<211> 10
<212> DNA
<213> Artificial Sequence

<220>
<223> Synthetic Polynucleotide

<400> 102
gagactgtac          10

<210> 103
<211> 12
<212> DNA
<213> Artificial Sequence

<220>
<223> Synthetic Polynucleotide

<400> 103
catgtcagag tc          12

<210> 104
<211> 11
<212> DNA
<213> Artificial Sequence

<220>
<223> Synthetic Polynucleotide

<400> 104
catgtcagag t          11

**Description**

BACKGROUND OF THE INVENTION

There are currently a variety of methods for nucleic acid sequencing, *i.e.,* the process of determining the precise order of nucleotides within a nucleic acid molecule. Current methods require amplifying a nucleic acid enzymatically, *e.g.,* PCR, and/or by cloning. Further enzymatic polymerizations are required to produce a detectable signal by a light detection means. Such amplification and polymerization steps are costly and/or time-consuming. Thus, there is a need in the art for a method of nucleic acid sequencing that is amplification- and enzyme-free. WO 2013/055995 A2 discloses a sequencing probe having a hexamer target binding domain (N1-N6) and a template non-hybridizing nucleic acid structure (TNH) having six probe hybridization sites (B1-B6), wherein each of the B1-B6 in the TNH correspond to one of N1-N6. US 7 745 129 B1 discloses a sequencing probe having one or more probe domains that bind to a target nucleic acid and a label-assembly domain having a start indicator, base encoding regions, and an end indicator, wherein each of the base encoding regions in the label-assembly domain correspond to nucleotides within the probe domains. The base encoding regions can contain either protein binding sites or probe hybridization sites. The present invention addresses these needs.

SUMMARY OF THE INVENTION

The present invention, as defined by the appended claims, provides sequencing probes, methods, and kits that provide enzyme-free, amplification-free, and library-free nucleic acid sequencing that has long-read-lengths and with low error rate. Moreover, the methods and kits have rapid sample-to-answer capability. These features are particularly useful for sequencing in a clinical setting. Provided herein are sequencing probes comprising a target binding domain and a barcode domain. The target binding domain and the barcode domain may be operably linked, *e.g.,* covalently linked. A sequencing probe optionally comprises a spacer between the target binding domain and the barcode domain. The spacer can be any polymer with appropriate mechanical properties, for example, a single- or double-stranded DNA spacer (of 1 to 100 nucleotides, e.g., 2 to 50 nucleotides). Non-limiting examples of double-stranded DNA spacers include the sequences covered by SEQ ID NO: 25 to SEQ ID NO: 29.

The target binding domain comprises at least eight nucleotides (*e.g.,* 9, 10, 11, 12, or more) and is capable of binding a target nucleic acid (*e.g.,* DNA, RNA, and PNA), and wherein at least two nucleotides in the target binding domain are universal bases capable of binding to any base within the target nucleic acid. The barcode domain comprises a synthetic backbone, the barcode domain comprising at least six attachment positions, e.g., seven, eight, nine, ten, eleven, twelve, or more attachment positions; each attachment position having at least one or more (*e.g.,* one to fifty) attachment regions; said attachment region comprising at least one (*i.e.,* one to fifty, *e.g.,* ten to thirty copies of a) nucleic acid sequence(s)) capable of being bound by a complementary nucleic acid molecule (RNA or DNA). Each attachment position of the at least six attachment positions corresponds to one nucleotide in the target binding domain and each of the at least six attachment positions have a different nucleic acid sequence, wherein said nucleic acid sequence of each position of the at least six attachment positions determines the position and identity of the corresponding one nucleotide in said target nucleic acid that is bound by said target binding domain, and wherein the universal bases precede and follow the nucleotides corresponding to positions in the barcode domain. Certain positions in a barcode domain may have more attachment regions than other positions; alternately, each position in a barcode domain has the same number of attachment regions. The nucleic acid sequence of a first attachment region determines the position and identity of a first nucleotide in the target nucleic acid that is bound by a first nucleotide of the target binding domain, whereas the nucleic acid sequence of a second attachment region determines the position and identity of a second nucleotide in the target nucleic acid that is bound by a second nucleotide of the target binding domain. Likewise, the nucleic acid sequence of a sixth attachment region determines the position and identity of a sixth nucleotide in the target nucleic acid that is bound by a sixth nucleotide of the target binding domain. In embodiments, the synthetic backbone comprises a polysaccharide, a polynucleotide (e.g., single or double stranded DNA or RNA), a peptide, a peptide nucleic acid, or a polypeptide. The number of nucleotides in a target binding domain equals to or is greater than (*e.g.,* 1, 2, 3, 4, or more) the number of positions in the barcode domain. Each attachment region in a specific position of the barcode domain may include one copy of the same nucleic acid sequence and/or multiple copies of the same nucleic acid sequence. However, an attachment region will include a different nucleic acid sequence than an attachment region in a different position of the barcode domain, even when both attachment regions identify the same type of nucleotide, *e.g.,* adenine, thymine, cytosine, guanine, uracil, and analogs thereof. An attachment region may be linked to a modified monomer, *e.g.,* a modified nucleotide, in the synthetic backbone, thereby creating a branch relative to the backbone. An attachment region may be part of a synthetic backbone's polynucleotide sequence. One or more attachment regions may be adjacent to at least one flanking single-stranded polynucleotide, that is, an attachment region may be operably linked to a 5' flanking single-stranded polynucleotide and/or to a 3' flanking single-stranded polynucleotide. An attachment region with or without one or two flanking single-stranded polynucleotides may be hybridized to a hybridizing nucleic acid molecule lacking a detectable label. A hybridizing nucleic acid molecule lacking a detectable label may be between about 4 and about 20 nucleotides in length, e.g., 12 nucleotides, or longer.

An attachment region may be bound by a complementary nucleic acid comprising a detectable label. Each complementary nucleic acid may comprise a detectable label.

Alternately, an attachment region may be bound by a complementary nucleic acid that is part of a reporter complex (comprising detectable labels). A complementary nucleic acid (either comprising a detectable label or of a reporter complex) may be between about 4 and about 20 nucleotides in length, e.g., about 8, 10, 12, and 14 nucleotides, or more. In a reporter complex, a complementary nucleic acid is linked (directly or indirectly) to a primary nucleic acid molecule. A complementary nucleic acid may be indirectly linked to a primary nucleic acid molecule via a single or double-stranded nucleic acid linker (e.g., a polynucleotide comprising 1 to 100 nucleotides). A primary nucleic acid is hybridized to one or more (e.g., 1, 2, 3, 4, 5, 6, 7, 8, 9, 10, or more) secondary nucleic acids. Each secondary nucleic acid is hybridized to one or more (e.g., 1, 2, 3, 4, 5, 6, 7, 8, 9, 10, or more) tertiary nucleic acids; the tertiary nucleic acids comprise one or more detectable labels. A or each secondary nucleic acid may comprise a region that does not hybridize to a primary nucleic acid molecule and does not hybridize to a tertiary nucleic acid molecule (an "extra-handle"); this region may be four or more (e.g., about 6 to about 40, e.g., about 8, 10, 12, and 14) nucleotides in length. The region that does not hybridize to a primary nucleic acid molecule and does not hybridize to a tertiary nucleic acid molecule may comprise the nucleotide sequence of the complementary nucleic acid molecule that is linked to the primary nucleic acid molecule. This region may be located near the end of the secondary nucleic acid distal to its end that hybridizes to the primary nucleic acid. By having "extra-handles" comprising the nucleotide sequence of the complementary nucleic acid, the likelihood and speed at which a reporter complex binds to a sequencing probe is greatly increased. In any embodiment or aspect of the present invention, when a reporter complex comprises "extra-handles", the reporter complex can hybridize to a sequencing probe either via the reporter complex's complementary nucleic acid or via the "extra-handle." Thus, for example, the phrase "binding to the first attachment region .. . a first complementary nucleic acid molecule of a first reporter complex" would be understood according to its plain meaning and also understood to mean "binding to the first attachment region ... an 'extra handle' of a first reporter complex."

In embodiments, the terms "barcode domain" and "synthetic backbone" are synonymous.

Provided herein, as defined by the appended claims, is a method for sequencing a nucleic acid using a sequencing probe of the present invention.. Each (e.g., first, second, third, fourth, fifth, sixth, seventh, eighth, ninth, tenth, or higher) complementary nucleic acid molecule (having a detectable label or part of a reporter complex) has the same nucleic acid sequence as its corresponding (i.e., first, second, third, fourth, fifth, sixth, seventh, eighth, ninth, tenth, or higher) hybridizing nucleic acid molecule lacking a detectable label. The target nucleic acid is immobilized to a substrate by binding a first position and/or second position of the target nucleic acid with a first and/or a second capture probe; each capture probe comprises an affinity tag that selectively binds to a substrate. The first and/or second positions may be at or near a terminus of a target nucleic acid. The substrate can be any solid support known in the art, e.g., a coated slide and microfluidic device (e.g., coated with streptavidin). Other positons which are located distant from a terminus of a target nucleic acid may be selectively bound to the substrate. The nucleic acid may be elongated by applying a force (e.g., gravity, hydrodynamic force, electromagnetic force, flow-stretching, a receding meniscus technique, and combinations thereof) sufficient to extend the target nucleic acid.

Provided herein is a method for sequencing a nucleic acid using one population of probes of the present invention or a plurality of populations of probes of the present invention. The method comprises steps of: (1) hybridizing at least one first population of first sequencing probes comprising a plurality of the sequencing probe of the present invention to a target nucleic acid that is immobilized to a substrate, wherein the target nucleic acid is immobilized to the substrate at one or more positions (with each sequencing probe in the first population de-hybridizing from the immobilized target nucleic acid under about the same conditions, e.g., level of chaotropic agent, temperature, salt concentration, pH, and hydrodynamic force); (2) binding a first complementary nucleic acid molecule comprising a detectable label or a first complementary nucleic acid molecule of a first reporter complex comprising a detectable label to a first attachment position of the at least six attachment positions; (3) detecting the detectable label of the bound first complementary nucleic acid molecule or the detectable label of the bound first complementary nucleic acid molecule of the first reporter complex; (4) identifying the position and identity of a first nucleotide in the immobilized target nucleic acid; (5) binding to the first attachment position a first hybridizing nucleic acid molecule lacking a detectable label, thereby unbinding the first complementary nucleic acid molecules comprising a detectable label or the first complementary nucleic acid molecule of the first reporter complex comprising a detectable label; (6) binding a second complementary nucleic acid molecule comprising a detectable label or a second complementary nucleic acid molecule of a second reporter complex comprising a detectable label to a second attachment position of the at least six attachment positions; (7) detecting the detectable label of the bound second complementary nucleic acid molecule or the detectable label of the bound second complementary nucleic acid molecule of the second complementary nucleic acid molecule of the second reporter complex; (8) identifying the position and identity of a second nucleotide in the immobilized target nucleic acid; (9) repeating steps (5) to (8) until each attachment position of the at least six attachment positions have been bound by a complementary nucleic acid molecule comprising a detectable label or a complementary nucleic acid molecule of a reporter complex comprising a detectable label, and the detectable label of the bound complementary nucleic acid molecule or the detectable label of the bound complementary nucleic acid molecule of a reporter complex has been detected, thereby identifying the linear order of at least six nucleotides for at least a first region of the immobilized target nucleic acid that was hybridized by the target binding domain of the sequencing probe; and (10) de-hybridizing the at least one first population of first sequencing probes from the immobilized target nucleic acid. Steps (5) and (6) may occur concurrently or sequentially. Thereby, the linear order of nucleotides is identified for regions of the immobilized target nucleic acid that were hybridized by the target binding domain of sequencing probes in the first population of sequencing probes.

In embodiments, when a plurality of populations (i.e., more than one population) of probes are used, the method further comprises steps of: (11) hybridizing at least one second population of second sequencing probes comprising a plurality of the sequencing probes of the present invention to a target nucleic acid that is immobilized to a substrate, wherein the target nucleic acid is immobilized to the substrate at one or more positions, wherein the target binding domain of the first sequencing probes and the second sequencing probe are different, and wherein each sequencing probe in the second population de-hybridizes from the immobilized target nucleic acid under about the same conditions; and de-hybridizes from the immobilized target nucleic acid under different conditions from the sequencing probes in the first population; (12) binding a first complementary nucleic acid molecule comprising a detectable label or a first complementary nucleic acid molecule of a first reporter complex comprising a detectable label to a first attachment region of the at least six attachment positions; (13) detecting the detectable label of the bound first complementary nucleic acid molecule or the detectable label of the bound first complementary nucleic acid molecule of the first reporter complex; (14) identifying the position and identity of a first nucleotide in the immobilized target nucleic acid; (15) binding to the first attachment position a first hybridizing nucleic acid molecule lacking a detectable label, thereby unbinding the first complementary nucleic acid molecules (having a detectable label or from a reporter complex); (16) binding a second complementary nucleic acid molecule comprising a detectable label or a second complementary nucleic acid molecule of a second reporter complex comprising a detectable label to a second attachment position of the at least six attachment positions; (17) detecting the detectable label of the bound second complementary nucleic acid molecule or the detectable label of the bound second complementary nucleic acid molecule of the second reporter complex; (18) identifying the position and identity of a second nucleotide in the immobilized target nucleic acid; and (19) repeating steps (15) to (18) until each attachment position of the at least six attachment positions have been bound by a complementary nucleic acid molecule comprising a detectable label or a complementary nucleic acid molecule of a reporter complex comprising a detectable label, and the detectable label of the bound complementary nucleic acid molecule or the detectable label of the bound complementary nucleic acid molecule of a reporter complex has been detected, thereby identifying the linear order of at least six nucleotides for at least a second region of the immobilized target nucleic acid that was hybridized by the target binding domain of the sequencing probe; and (20) de-hybridizing the at least one second population of second sequencing probes from the immobilized target nucleic acid. Steps (16) and (17) may occur concurrently or sequentially.

Each sequencing probe in the second population may de-hybridize from the immobilized target nucleic acid at a different condition (*e.g.,* a higher temperature, higher level of chaotropic agent, higher salt concentration, higher flow rate, and different pH) than the average condition for which the sequencing probes in the first population de-hybridize from the target nucleic acid. However, when more than two populations of probes are used, then probes in two sequential populations may de-hybridize at different conditions and probes in non-sequential populations may de-hybridize at similar conditions. As an example, probes in a first population and third population may de-hybridize under similar conditions. In embodiments, sequential populations of probes de-hybridized at increasingly more stringent conditions (*e.g.,* higher levels of chaotropic agent, salt concentration, and temperature). For a microfluidic device, using temperature as an example, a first population of probes may remain hybridized at a first temperature but de-hybridize at a second temperature, which is higher than the first. A second population of probes may remain hybridized at the second temperature but de-hybridize at a third temperature, which is higher than the second. In this example, solutions (comprising reagents required by the present method) flowing over a target nucleic acid for initial probe populations are at a lower temperature than solutions flowing over the target nucleic acid for later probe populations.

In some embodiments, after a population of probes has been used, the population of probes is de-hybridized from the target nucleic acid and a new aliquot of the same population of probes is used. For example, after a first population of probes has been hybridized, detected, and de-hybridized, a subsequent aliquot of the first population of probes is hybridized. Alternately, as an example, a first population of probes may be de-hybridized and replaced with a second population of probes; once the second population has been detected and de-hybridized, a subsequent aliquot of the first population of probes is hybridized to the target nucleic acid. Thus, a probe in the subsequent population may hybridize to a region of the target nucleic acid that had been previously sequenced (thereby gaining duplicative and/or confirmatory sequence information) or a probe in the subsequent population may hybridize to a region of the target nucleic acid that had not previously been sequenced (thereby gaining new sequence information). Accordingly, a population of probes may be re-aliquoted when a prior read was unsatisfactory (for any reason) and/or to improve the accuracy of the alignment resulting from the sequencing reads.

The probes hybridizing and de-hybridizing under similar conditions may have similar lengths of their target binding domain, GC content, or frequency of repeated bases and combinations thereof. Relationships between Tm and length of an oligonucleotide are taught, for example, in Sugimoto et al., Biochemistry, 34, 11211-6.

When more than two populations of probes are used, steps, as described for the first and second populations of sequencing probes, are repeated with additional populations of probes (*e.g.,* 10 to 100 to 1000 populations). The number of populations of probes used will depend on a variety of factors, including but not limited to the size of the target nucleic acid, the number of unique probes in each population, the degree of overlap among sequencing probes desired, and the enrichment of probes to regions of interest.

A population of probes may contain extra sequencing probes directed to a specific region of interest in a target nucleic acid, *e.g.,* a region containing a mutation (*e.g.,* a point mutation) or a SNP allele. A population of probes may contain fewer sequencing probes directed to a specific region of less interest in a target nucleic acid.

A population of sequencing probes may be compartmentalized into discrete smaller pools of sequencing probes. The compartmentalization may be based upon predicted melting temperature of the target binding domain in the sequencing probes and/or upon sequence motif of the target binding domain in the sequencing probes. The compartmentalization may be based on empirically-derived rules. The different pools of sequencing probes can be reacted with the target nucleic acid using different reaction conditions, e.g., based on temperature, salt concentration, and/or buffer content. The compartmentalization may be performed to cover target nucleic acid with uniform coverage. The compartmentalization may be performed to cover target nucleic acid with known coverage profile.

The lengths of target binding domains in a population of sequencing probes may be reduced to increase coverage of probes in a specific region of a target nucleic acid. The lengths of target binding domains in a population of sequencing probes may be increased to decrease coverage of probes in a specific region of a target nucleic acid, *e.g.,* to above the resolution limit of the sequencing apparatus.

Alternately or additionally, the concentration of sequencing probes in a population may be increased to increase coverage of probes in a specific region of a target nucleic acid. The concentration of sequencing probes may be reduced to decrease coverage of probes in a specific region of a target nucleic acid, *e.g.,* to above the resolution limit of the sequencing apparatus. The methods for sequencing a nucleic acid further comprises steps of assembling each identified linear order of nucleotides for each region of the immobilized target nucleic acid, thereby identifying a sequence for the immobilized target nucleic acid. Steps of assembling use a non-transitory computer-readable storage medium with an executable program stored thereon which instructs a microprocessor to arrange each identified linear order of nucleotides, thereby obtaining the sequence of the nucleic acid. Assembling can occur in "real time", *i.e.,* while data is being collected from sequencing probes rather than after all data has been collected.

The target nucleic acid, *i.e.,* that is sequenced, may be between about 4 and 1,000,000 nucleotides in length. The target may include a whole, intact chromosome or a fragment thereof either of which is greater than 1,000,000 nucleotides in length.

Provided herein are kits including sequencing probes of the present invention and for performing methods of the present invention. In embodiments, the kits include a substrate capable of immobilizing a nucleic acid via a capture probe, a plurality of sequencing probes of the present invention, at least two capture probes, wherein a capture probe comprises a nucleic acid sequence that hybridizes to a region of a target nucleic acid that is different than the region of the target nucleic acid that hybridizes to a sequencing probe and an affinity tag, at least six complementary nucleic acid molecules comprising a detectable label, wherein a complementary nucleic acid comprising a detectable label comprises a nucleic acid sequence that hybridizes to one of the at least six attachment positions of a sequencing probe and a detectable label, at least six complementary nucleic acid molecules which lacks a detectable label, wherein a complementary nucleic acid which lacks a detectable label comprises a nucleic acid sequence that hybridizes to one of the at least six attachment positions of a sequencing probe, and instructions for use. In embodiments, the kit comprises about or at least 4096 unique sequencing probes. 4096 is the minimum number of unique probes necessary to include each possible hexameric combination (*i.e.,* for probes each having six attachment regions in the barcode domains). Here, "4096" is achieved since there are four nucleotides options for six positions: $4^6$. For a set of probes having four attachment regions in the barcode domains, only 256 (*i.e.,* $4^4$) unique probes will be needed. For a set of probes having eight nucleotides in their target binding domains, $4^8$ (*i.e.,* 65,536) unique probes will be needed. For a set of probes having ten nucleotides in their target binding domains, $4^{10}$ (*i.e.,* 1,048,576) unique probes will be needed.

In embodiments, the kit comprises about or at least twenty four distinct complementary nucleic acid molecule having a detectable label and about or at least twenty four distinct hybridizing nucleic acid molecule lacking a detectable label. A complementary nucleic acid may bind to an attachment region having a sequence of one of SEQ ID NO: 1 to 24, as non-limiting examples. Additional exemplary sequences that may be included in a barcode domain are listed in SEQ ID NO: 42 to SEQ ID NO: 81. Indeed, the nucleotide sequence is not limited; preferably it lacks substantial homology (e.g., 50% to 99.9%) with a known nucleotide sequence; this helps avoid undesirable hybridization of a complementary nucleic acid and a target nucleic acid.

Any of the above aspects and embodiments can be combined with any other aspect or embodiment. Unless otherwise defined, all technical and scientific terms used herein have the same meaning as commonly understood by one of ordinary skill in the art to which this invention belongs. In the Specification, the singular forms also include the plural unless the context clearly dictates otherwise; as examples, the terms "a," "an," and "the" are understood to be singular or plural and the term "or" is understood to be inclusive. By way of example, "an element" means one or more element. Throughout the specification the word "comprising," or variations such as "comprises" or "comprising," will be understood to imply the inclusion of a stated element, integer or step, or group of elements, integers or steps, but not the exclusion of any other element, integer or step, or group of elements, integers or steps. About can be understood as within 10%, 9%, 8%, 7%, 6%, 5%, 4%, 3%, 2%, 1%, 0.5%, 0.1%, 0.05%, or 0.01% of the stated value. Unless otherwise clear from the context, all numerical values provided herein are modified by the term "about."

BRIEF DESCRIPTION OF THE DRAWINGS

The patent or application file contains at least one drawing executed in color. Copies of this patent or patent application publication with color drawings will be provided by the Office upon request and payment of the necessary fee.

The above and further features will be more clearly appreciated from the following detailed description when taken in conjunction with the accompanying drawings.

Figure 1 to Figure 5 show schematics of exemplary sequencing probes of the disclosure.

Figure 6A to Figure 6D are schematics showing variants of a sequencing probe of the disclosure.

Figure 7 shows schematics of target binding domains of sequencing probes of the disclosure; the domains include zero, two, or four nucleotides having universal bases.

Figure 8A to Figure 8E illustrate steps of a sequencing method of the disclosure.

Figure 9A shows an initial step of a sequencing method of the disclosure.

Figure 9B shows a schematic of a reporter complex comprising detectable labels.

Figure 9C shows a plurality of reporter complexes each comprising detectable labels.

Figures 9D to 9G show further steps of the sequencing method begun in Figure 9A.

Figure 10 shows an alternate illustration of the steps shown in Figure 9D and Figure 9E and exemplary data obtained therefrom. The fragment of the sequencing probe shown has the sequence of SEQ ID NO: 82.

Figure 11 illustrates a variation of the method shown in Figure 10. The fragment of the sequencing probe shown likewise has the sequence of SEQ ID NO: 82.

Figure 12 illustrates a method of the present invention.

Figure 13 compares steps required in a sequencing method of the disclosure with steps required with other sequencing methods.

Figure 14 and Figure 15 exemplify performance measurements obtainable by the methods of the disclosure.

Figure 16 compares the sequencing rate, number of reads, and clinical utility for the present invention and various other sequencing methods/apparatuses.

Figure 17 demonstrates the low raw error rate of sequencing methods of the disclosure. The template sequence shown has the sequence of SEQ ID NO: 83.

Figure 18 compares sequencing data obtainable from the methods of the disclosure with other sequencing methods.

Figure 19 demonstrates single-base specificity of sequencing methods of the disclosure. The template and probe sequences shown (from top to bottom) have the sequences of SEQ ID NO: 84 to SEQ ID NO: 88.

Figure 20A shows various designs of reporter complexes of the disclosure.

Figure 20B shows fluorescent counts obtained from the reporter complexes shown in Figure 20A.

Figure 20C shows exemplary recipes for constructing reporter complexes of the disclosure.

Figure 21A shows designs of reporter complexes comprising "extra-handles".

Figure 21B shows fluorescent counts obtained from the reporter complexes having "extra-handles".

Figure 22A and Figure 22B show hybridization kinetics of two exemplary designs of reporter complexes of the disclosure.

Figure 23 shows a schematic of a sequencing probe of the disclosure used in a method distinct from that shown in Figure 8 through Figure 12.

Figure 24 shows a schematic of a consumable sequencing card useful in the disclosure.

Figure 25 shows the mismatch detection of a 10 mer, as described in Example 3. The nucleotides shown (top to bottom) have the sequences of SEQ ID NO: 89 to SEQ ID NO: 99.

Figure 26 shows hybridization ability depending on the size of a target binding domain, as described in Example 3. The background is high due to very high reporter concentration and there was no prior purification. The nucleotides shown (top to bottom) have the sequences of SEQ ID NO: 100 to SEQ ID NO: 104.

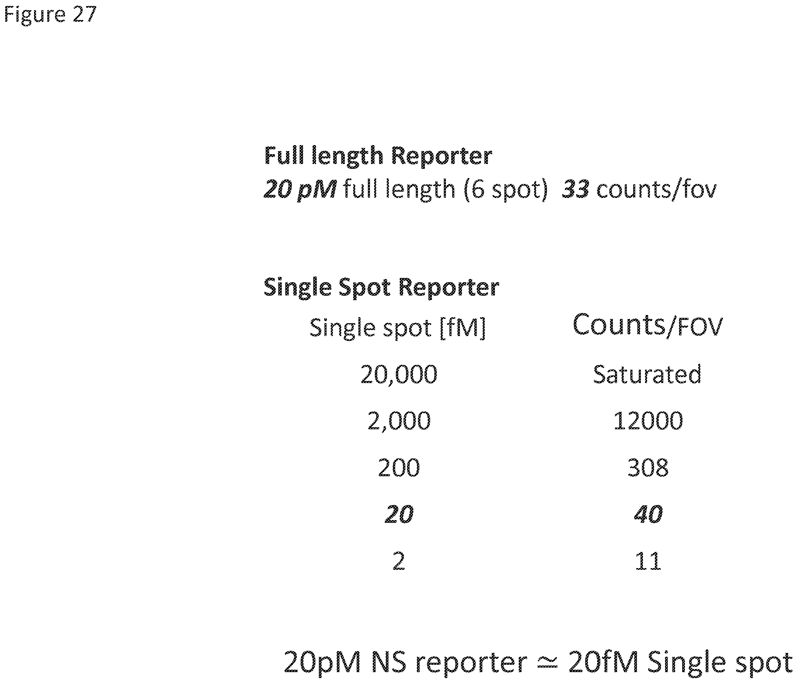

Figure 27 shows a comparison between a single spot vs a full-length reporter. Results for single spots show speed of hybridization is 1000x greater than for a full length barcode (Conditions 100nM target, 30 minute hybridization).

DETAILED DESCRIPTION OF THE INVENTION

The present invention provides sequencing probes, methods, kits, and apparatuses that provide enzyme-free, amplification-free, and library-free nucleic acid sequencing that has long-read-lengths and with low error rate.

**Sequencing Probe**

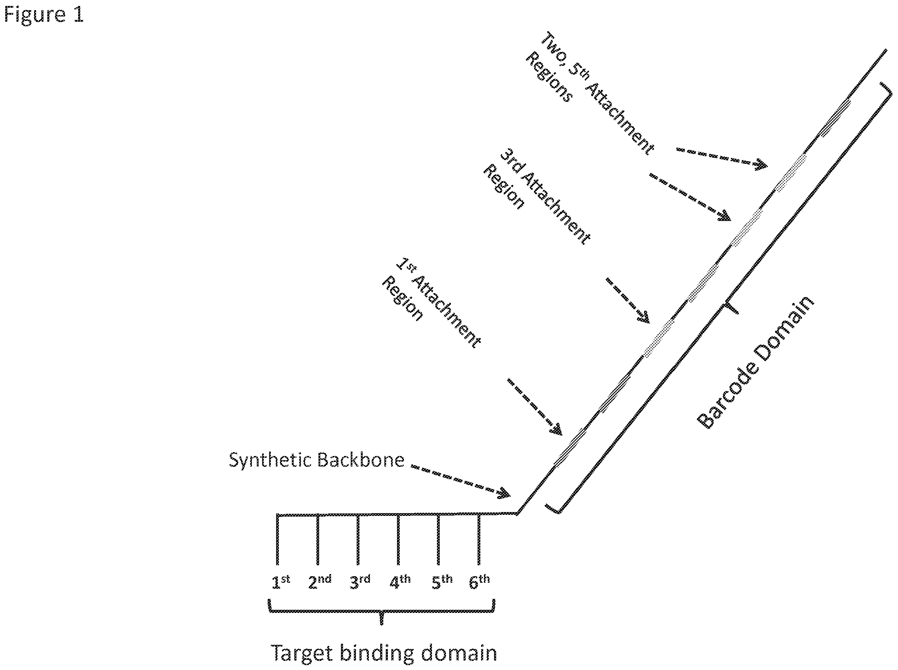

The present invention, as defined by the appended claims, and the disclosure relate to a sequencing probe comprising a target binding domain and a barcode domain. Examples of sequencing probes of the disclosure are shown in Figures 1 to 6.

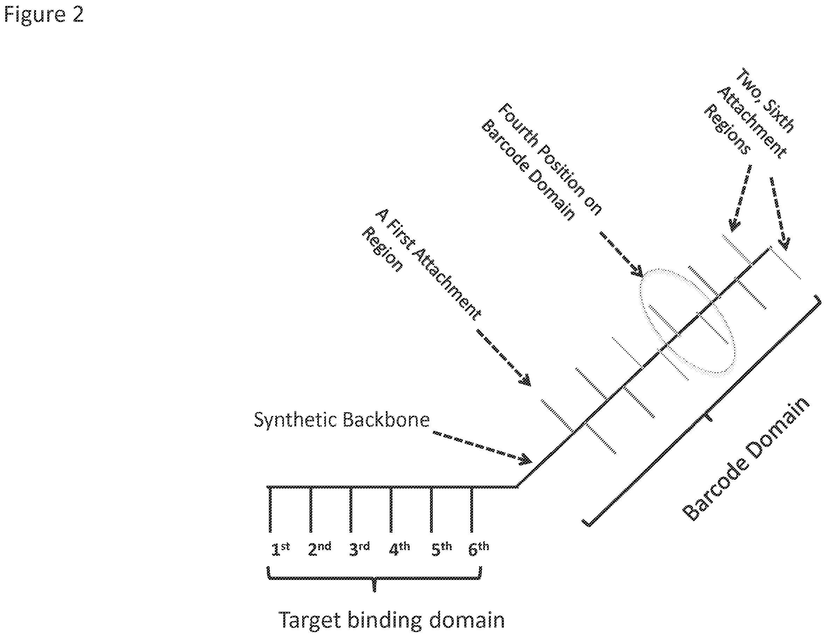

Figure 1 shows a schematic of a sequencing probe of the disclosure. This exemplary sequencing probe has a target binding domain of six nucleotides, each of which corresponds to a position in the barcode domain (which comprises one or more an attachment regions). A first attachment region is noted; it corresponds to the nucleotide of a target nucleic acid bound by a first nucleotide in the target binding domain. The third position on the barcode domain is noted. A fifth position comprising two attachment regions is noted. Each position on a barcode domain can have multiple attachment regions. For example, a position may have 1 to 50 attachment regions. Certain positions in a barcode domain may have more attachment regions than other positions (as shown here in position 5 relative to positions 1 to 4 and 6); alternately, each position in a barcode domain has the same number of attachment regions (see, e.g., Figures 2, 3, 5, and 6). Although not shown, each attachment region comprises at least one (*i.e.,* one to fifty, *e.g.,* ten to thirty) copies of a nucleic acid sequence(s) capable of reversibly binding to a complementary nucleic acid molecule (RNA or DNA). In Figure 1, the attachment regions are integral to the linear polynucleotide molecule that makes up the barcode domain.

Figure 2 shows a schematic of a sequencing probe of the disclosure. This exemplary sequencing probe has a target binding domain of six nucleotides, each of which corresponds to an attachment region in the barcode domain. A first attachment region is noted; it corresponds to the nucleotide of a target nucleic acid bound by a first nucleotide in the target binding domain. The fourth position on the barcode domain, which comprises a portion of the barcode domain and two fourth attachment regions are encircled. Two sixth attachments regions are noted. Here, each position has two attachment regions; however, each position on a barcode domain can have one attachment region or multiple attachment regions, e.g., 2 to 50 attachment regions. Although not shown, each attachment region comprises at least one (*i.e.,* one to fifty, *e.g.,* ten to thirty) copies of a nucleic acid sequence(s) capable of reversibly binding to a complementary nucleic acid molecule (RNA or DNA). In Figure 2, the barcode domain is a linear polynucleotide molecule to which the attachment regions are linked; the attachment regions are not integral to the polynucleotide molecule.

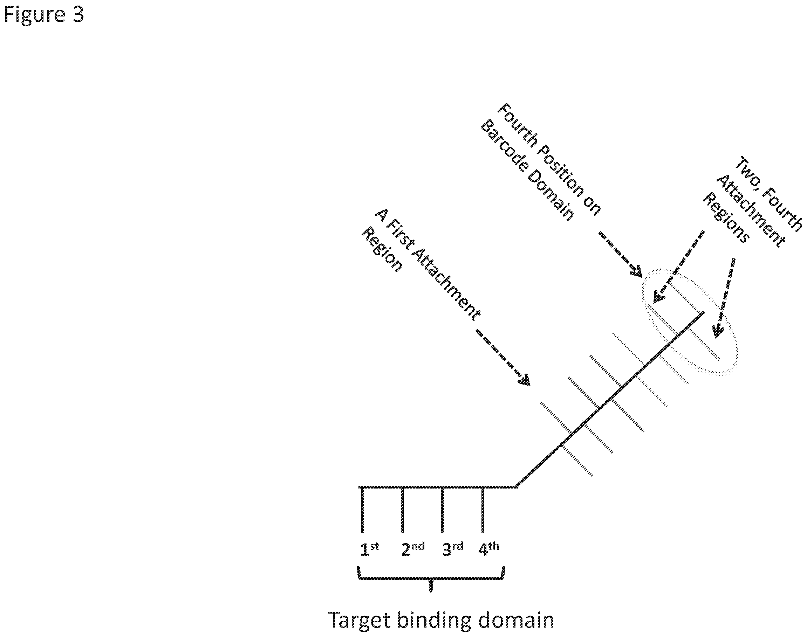

Figure 3 shows another a schematic of a sequencing probe of the disclosure. This exemplary sequencing probe has a target binding domain of four nucleotides, with these four nucleotides in the corresponding to four positions in the barcode domain. Each position is shown with three linked attachment regions.

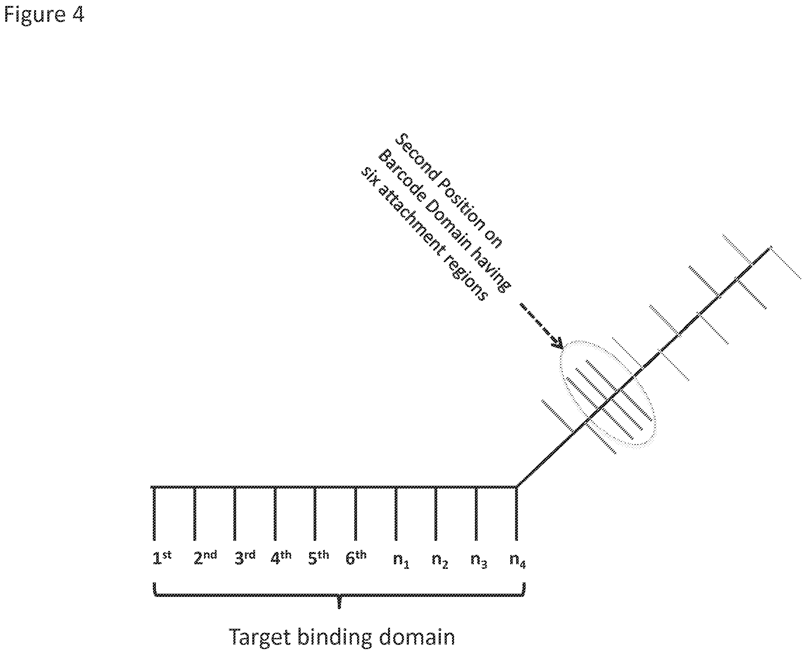

Figure 4 shows yet another schematic of a sequencing probe of the disclosure. This exemplary sequencing probe has a target binding domain of ten nucleotides. However, only the first six nucleotides correspond to six positions in the barcode domain. The seventh to tenth nucleotides (indicated by "$n_1$ to $n_4$") are added to increase the length of the target binding domain thereby affecting the likelihood that a probe will hybridize and remain hybridized to a target nucleic acid.

In embodiments, "n" nucleotides may precede the nucleotides corresponding to positions in the barcode domain. In embodiments, "n" nucleotides may follow the nucleotides corresponding to positions in the barcode domain. In Figure 4, four "n" nucleotides are shown; however, a target binding domain may include more than four "n" nucleotides. The "n" nucleotides may have universal bases (e.g., inosine, 2'-deoxyinosine (hypoxanthine deoxynucleotide) derivatives, nitroindole, nitroazole analogues, and hydrophobic aromatic non-hydrogen-bonding bases) which can base pair with any of the four canonical bases.

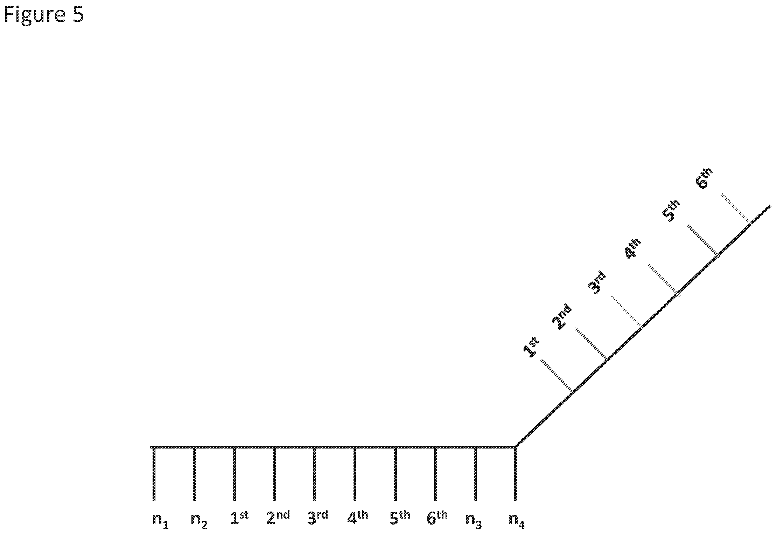

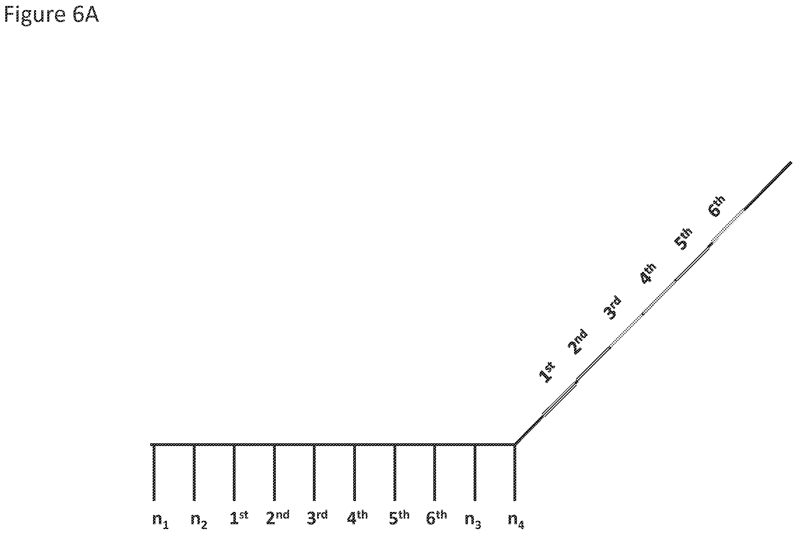

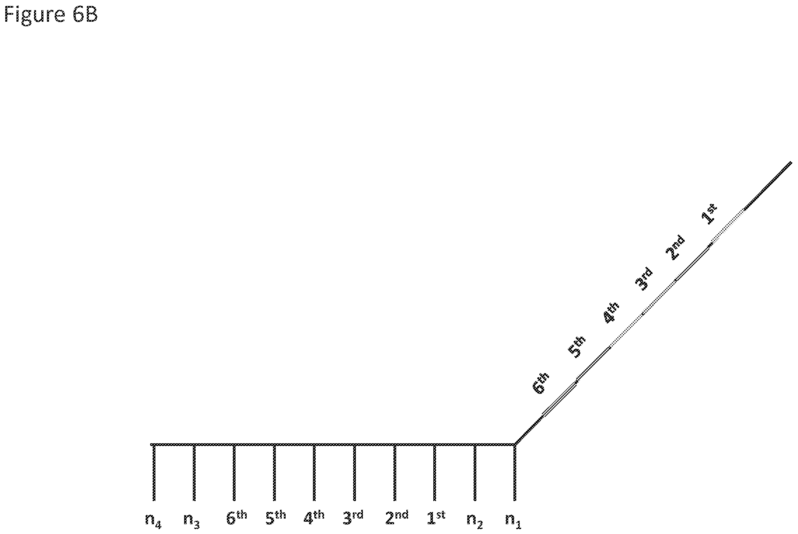

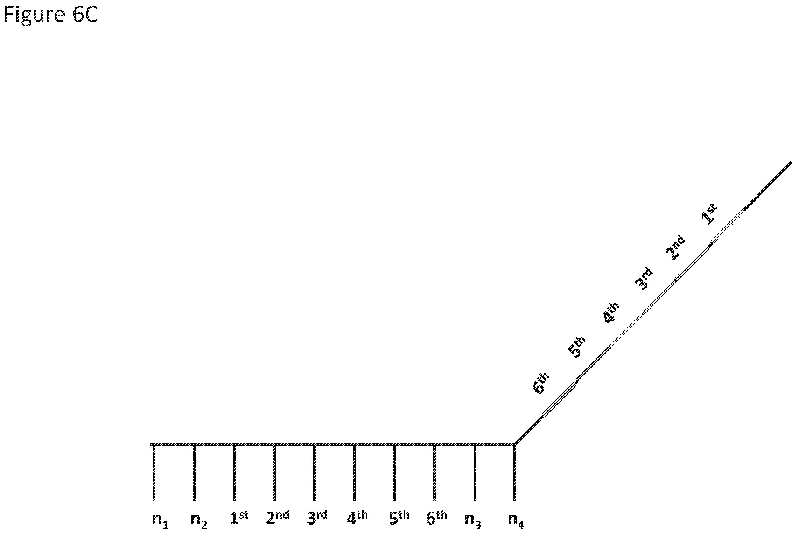

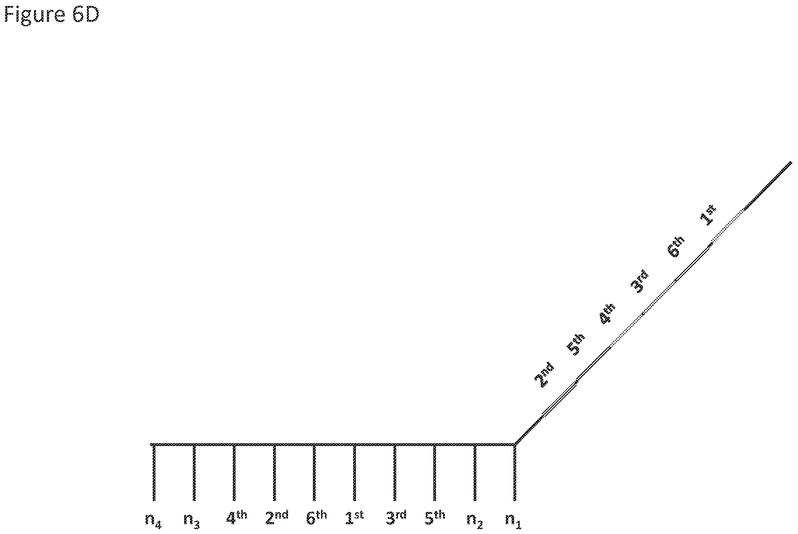

Another sequencing probe of the disclosure is shown in Figure 5. Here, the "n" nucleotides precede and follow the nucleotides corresponding to positions in the barcode domain. The exemplary sequencing probe shown has a target binding domain of ten nucleotides. However, only the third to eight nucleotides in the target binding domain correspond to six positions (first to sixth) in the barcode domain. The first, second, ninth, and tenth nucleotides (indicated by "$n_1$ to n4") are added to increase the length of the target binding domain. In Figure 5, four "n" nucleotides are shown; however, a target binding domain may include more or less than four "n" nucleotides. Figure 6A to Figure 6D show variants of a sequencing probe of Figure 1. In Figure 6A, the linear order of nucleotides in the target binding domain and linear order of attachment regions in the barcode domain progress from left to right (with respect to the illustration). In Figure 6B, the linear order of nucleotides in the target binding domain and linear order of attachment regions in the barcode domain progress from right to left (with respect to the illustration). In Figure 6C, the linear order of nucleotides in the target binding domain is reversed relative to the linear order of attachment regions in the barcode domain. In any probe of the disclosure, there may be a lack of strict order of the nucleotides in the target binding domain and of attachment regions in barcode domain as long as the probe is designed such that each nucleotide in the target binding domain corresponds to an attachment domain or attachment domains in the barcode domain; lacks of strict order is shown in Figure 6D. Any probe of the disclosure (e.g., those exemplified in Figures 1 to 5) may have an ordering of nucleotides and attachment regions as shown in Figure 6.

The target binding domain has at least four nucleotides, e.g., at least, 4, 5, 6, 7, 8, 9, 10, 11, 12, or more nucleotides. The target binding domain preferable is a polynucleotide. The target binding domain is capable of binding a target nucleic acid.

A probe may include multiple copies of the target binding domain operably linked to a synthetic backbone.

Probes can be designed to control the likelihood of hybridization and/or de-hybridization and the rates at which these occur. Generally, the lower a probe's Tm, the faster and more likely that the probe will de-hybridize to/from a target nucleic acid. Thus, use of lower Tm probes will decrease the number of probes bound to a target nucleic acid.

The length of a target binding domain, in part, affects the likelihood of a probe hybridizing and remaining hybridized to a target nucleic acid. Generally, the longer (greater number of nucleotides) a target binding domain is, the less likely that a complementary sequence will be present in the target nucleotide. Conversely, the shorter a target binding domain is, the more likely that a complementary sequence will be present in the target nucleotide. For example, there is a 1/256 chance that a four-mer sequence will be located in a target nucleic acid versus a 1/4096 chance that a six-mer sequence will be located in the target nucleic acid. Consequently, a collection of shorter probes will likely bind in more locations for a given stretch of a nucleic acid when compared to a collection of longer probes.

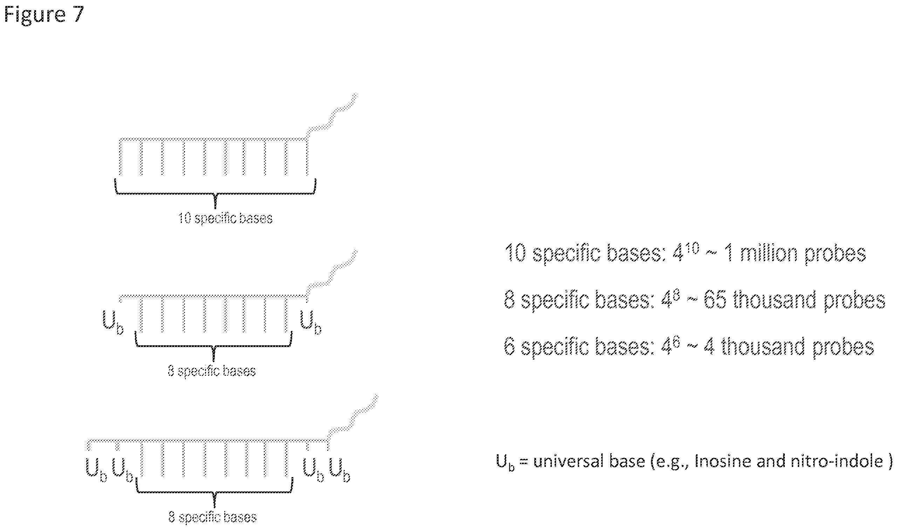

Figure 7 shows 10-mer target binding domains. In some embodiments, the target binding domain includes four universal bases (identified as "$U_b$") which base pair with any of the four canonical nucleotides (A, G, C, and T). In embodiments, the target binding domain includes one to six (e.g., 2 and 4) universal bases. A target binding domain may include no universal nucleotides. Figure 7 notes that a "complete" population of probes having 6 specific nucleotides in the target binding domain will require 4096 unique probes and a "complete" population of probes having 10 specific nucleotides will require ~1 million unique probes.

In circumstances, it is preferable to have probes having shorter target binding domains to increase the number of reads in the given stretch of the nucleic acid, thereby enriching coverage of a target nucleic acid or a portion of the target nucleic acid, especially a portion of particular interest, e.g., when detecting a mutation or SNP allele.

However, it may be preferable to have fewer numbers of probes bound to a target nucleic acid since there are occasions when too many probes in a region may cause overlap of their detectable label, thereby preventing resolution of two nearby probes. This is explained as follows. Given that one nucleotide is 0.34 nm in length and given that the lateral (x-y) spatial resolution of a sequencing apparatus is about 200nm, a sequencing apparatus's resolution limit is about 588 base pair (i.e., a 1 nucleotide/0.34nm x 200nm). That is to say, the sequencing apparatus mentioned above would be unable to resolve signals from two probes hybridized to a target nucleic acid when the two probes are within about 588 base pair of each other. Thus, two probes, depending on the resolution of the sequencing apparatus, will need be spaced approximately 600bp's apart before their detectable label can be resolved as distinct "spots". So, at optimal spacing, there should be a single probe per 600bp of target nucleic-acid. A variety of software approaches (e.g., utilize fluorescence intensity values and wavelength dependent ratios) can be used to monitor, limit, and potentially deconvolve the number of probes hybridizing inside a resolvable region of a target nucleic acid and to design probe populations accordingly. Moreover, detectable labels (e.g., fluorescent labels) can be selected that provide more discrete signals. Furthermore, methods in the literature (e.g., Small and Parthasarthy: "Superresolution localization methods." Annu. Rev. Phys Chem., 2014; 65:107-25) describe structured-illumination and a variety of super-resolution approaches which decrease the resolution limit of a sequencing microscope up to 10's-of-nanometers. Use of higher resolution sequencing apparatuses allow for use of probes with shorter target binding domains.

As mentioned above, designing the Tm of probes can affect the number of probes hybridized to a target nucleic acid. Alternately or additionally, the concentration of sequencing probes in a population may be increased to increase coverage of probes in a specific region of a target nucleic acid. The concentration of sequencing probes may be reduced to decrease coverage of probes in a specific region of a target nucleic acid, e.g., to above the resolution limit of the sequencing apparatus.

The term "target nucleic acid" shall mean a nucleic acid molecule (DNA, RNA, or PNA) whose sequence is to be determined by the probes, methods, and apparatuses of the invention. In general, the terms "target nucleic acid", "nucleic acid molecule,", "nucleic acid sequence," "nucleic acid", "nucleic acid fragment," "oligonucleotide" and "polynucleotide" are used interchangeably and are intended to include, but not limited to, a polymeric form of nucleotides that may have various lengths, either deoxyribonucleotides or ribonucleotides, or analogs thereof. Non- limiting examples of nucleic acids include a gene, a gene fragment, an exon, an intron, intergenic DNA (including, without limitation, heterochromatic DNA), messenger RNA (mRNA), transfer RNA, ribosomal RNA, ribozymes, small interfering RNA (siRNA), non-coding RNA (ncRNA), cDNA, recombinant polynucleotides, branched polynucleotides, plasmids, vectors, isolated DNA of a sequence, isolated RNA of a sequence, nucleic acid probes, and primers.

The present methods directly sequence a nucleic acid molecule obtained from a sample, *e.g.*, a sample from an organism, and, preferably, without a conversion (or amplification) step. As an example, for RNA-based sequencing, the present methods do not require conversion of an RNA molecule to a DNA molecule (*i.e.,* via synthesis of cDNA) before a sequence can be obtained. Since no amplification or conversion is required, a nucleic acid sequenced as described herein will retain any unique base and/or epigenetic marker present in the nucleic acid when the nucleic acid is in the sample or when it was obtained from the sample. Such unique bases and/or epigenetic markers are lost in sequencing methods known in the art.

The target nucleic acid can be obtained from any sample or source of nucleic acid, *e.g.,* any cell, tissue, or organism, in vitro, chemical synthesizer, and so forth. The target nucleic acid can be obtained by any art-recognized method. In embodiments, the nucleic acid is obtained from a blood sample of a clinical subject. The nucleic acid can be extracted, isolated, or purified from the source or samples using methods and kits well known in the art.

A nucleic acid molecule comprising the target nucleic acid may be fragmented by any means known in the art. Preferably, the fragmenting is performed by an enzymatic or a mechanical means. The mechanical means may be sonication or physical shearing. The enzymatic means may be performed by digestion with nucleases (*e.g.,* Deoxyribonuclease I (DNase I)) or one or more restriction endonucleases.

When a nucleic acid molecule comprising the target nucleic acid is an intact chromosome, steps should be taken to avoid fragmenting the chromosome.

The target nucleic acid can include natural or non-natural nucleotides, comprising modified nucleotides, as well-known in the art.

Probes may have overall lengths (including target binding domain, barcode domain, and any optional domains) of about 20 nanometers to about 50 nanometers. A probe's backbone may a polynucleotide molecule comprising about 120 nucleotides.

The barcode domain comprises a synthetic backbone. The synthetic backbone and the target binding domain are operably linked, *e.g.,* are covalently attached or attached via a linker. The synthetic backbone can comprise any material, *e.g.,* polysaccharide, polynucleotide, polymer, plastic, fiber, peptide, peptide nucleic acid, or polypeptide. Preferably, the synthetic backbone is rigid. In embodiments, the backbone comprises "DNA origami" of six DNA double helices (*See, e.g.,* Lin et al, "Submicrometre geometrically encoded fluorescent barcodes self-assembled from DNA." Nature Chemistry; 2012 Oct; 4(10): 832-9). A barcode can be made of DNA origami tiles (Jungmann et al, "Multiplexed 3D cellular super-resolution imaging with DNA-PAINT and Exchange-PAINT", Nature Methods, Vol. 11, No. 3, 2014).

The barcode domain comprises a plurality of positions, e.g., one, two, three, four, five, six, seven, eight, nine, ten, or more positions. The number of positions may be less than, equal to, or more than the number of nucleotides in the target binding domain. It is preferable to include additional nucleotides in a target binding domain than number of positions in the backbone domain, *e.g.,* one, two, three, four, five, six, seven, eight, nine, ten, or more nucleotides. The length of the barcode domain is not limited as long as there is sufficient space for at least four positions, as described above.

Each position in the barcode domain corresponds to a nucleotide in the target binding domain and, thus, to a nucleotide in the target nucleic acid. As examples, the first position in the barcode domain corresponds to the first nucleotide in the target binding domain and the sixth position in the barcode domain corresponds to the sixth nucleotide in the target binding domain.

Each position in the barcode domain comprises at least one attachment region, *e.g.,* one to 50, or more, attachment regions. Certain positions in a barcode domain may have more attachment regions than other positions (e.g., a first position may have three attachment regions whereas a second position may have two attachment positions); alternately, each position in a barcode domain has the same number of attachment regions. Each attachment region comprises at least one (*i.e.,* one to fifty, *e.g.,* ten to thirty) copies of a nucleic acid sequence(s) capable of being reversibly bound by a complementary nucleic acid molecule (*e.g.,* DNA or RNA). In examples, the nucleic acid sequence in a first attachment region determines the position and identity of a first nucleotide in the target nucleic acid that is bound by a first nucleotide of the target binding domain. Each attachment region may be linked to a modified monomer (*e.g.,* modified nucleotide) in the synthetic backbone such that the attachment region branches from the synthetic backbone. In embodiments, the attachment regions are integral to a polynucleotide backbone; that is to say, the backbone is a single polynucleotide and the attachment regions are parts of the single polynucleotide's sequence. In embodiments, the terms "barcode domain" and "synthetic backbone" are synonymous.

The nucleic acid sequence in an attachment region identifies the position and identity of a nucleotide in the target nucleic acid that is bound by a nucleotide in the target binding domain of a sequencing probe. In a probe, each attachment region will have a unique overall sequence. Indeed, each position on a barcode domain can have an attachment region comprising a nucleic acid sequence that encodes one of four nucleotides, *i.e.,* specific to one of adenine, thymine/uracil, cytosine, and guanine. Also, the attachment region of a first position (and encoding cytosine, for example) will include a nucleic acid sequence different from the attachment region of a second position (and encoding cytosine, for example). Thus, to a nucleic acid sequence in an attachment region in a first position that encodes a thymine, there will be no binding of a complementary nucleic acid molecule that identifies an adenine in a target nucleic acid corresponding to the first nucleotide of a target binding domain. Also, to an attachment region in a second position, there will be no binding of a complementary nucleic acid molecule that identifies an adenine in a target nucleic acid corresponding to the first nucleotide of a target binding domain.

Each position on a barcode domain may include one or more (up to fifty, preferably ten to thirty) attachment region; thus, each attachment region may bind one or more (up to fifty, preferably ten to thirty) complementary nucleic acid molecules. As examples, the probe in Figure 1 has a fifth position comprising two attachment regions and the probe in Figure 2 has a second position having six attachment regions. In embodiments, the nucleic acid sequences of attachment regions at a position are identical; thus, the complementary nucleic acid molecules that bind those attachment regions are identical. In alternate embodiments, the nucleic acid sequences of attachment regions at a position are not identical; thus, the complementary nucleic acid molecules that bind those attachment regions are not identical, *e.g.,* each comprises a different nucleic acid sequence and/or detectable label. Therefore, in the alternate embodiment, the combination of non-identical nucleic acid molecules (*e.g.,* their detectable labels) attached to an attachment region together provides a code for identifying a nucleotide in the target nucleic acid.

Table 1 provides exemplary sequences, for illustration purposes only, for attachments regions for sequencing probes having up to six positions in its barcode domain and detectable labels on complementary nucleic acid that bind thereto.

Table 1:

| Nucleotide in target binding domain/position in barcode domain | Nucleotide | Nucleic Acid Sequence (5' to 3') in Attachment Region | Detectable label of complementary nucleic acid | SEQ ID NO |
|---|---|---|---|---|
| 1 | A | ATACATCTAG | GFP | 1 |
| 1 | G | GATCTACATA | RFP | 2 |
| 1 | C | TTAGGTAAAG | CFP | 3 |
| 1 | U/T | TCTTCATTAC | YFP | 4 |
| 2 | A | ATGAATCTAC | GFP | 5 |
| 2 | G | TCAATGTATG | RFP | 6 |
| 2 | c | AATTGAGTAC | CFP | 7 |
| 2 | U/T | ATGTTAATGG | YFP | 8 |
| 3 | A | AATTAGGATG | GFP | 9 |
| 3 | G | ATAATGGATC | RFP | 10 |
| 3 | C | TAATAAGGTG | CFP | 11 |
| 3 | U/T | TAGTTAGAGC | YFP | 12 |
| 4 | A | ATAGAGAAGG | GFP | 13 |
| 4 | G | TTGATGATAC | RFP | 14 |
| 4 | C | ATAGTGATTC | CFP | 15 |
| 4 | U/T | TATAACGATG | YFP | 16 |
| 5 | A | TTAAGTTTAG | GFP | 17 |
| 5 | G | ATACGTTATG | RFP | 18 |
| 5 | C | TGTACTATAG | CFP | 19 |
| 5 | U/T | TTAACAAGTG | YFP | 20 |
| 6 | A | AACTATGTAC | GFP | 21 |
| 6 | G | TAACTATGAC | RFP | 22 |
| 6 | C | ACTAATGTTC | CFP | 23 |
| 6 | U/T | TCATTGAATG | YFP | 24 |

As seen in Table 1, the nucleic acid sequence of a first attachment region may be one of SEQ ID NO: 1 to SEQ ID NO: 4 and the nucleic acid sequence of a second attachment may be one of SEQ ID NO: 5 to SEQ ID NO: 8. When the first nucleotide in the target nucleic acid is adenine, the nucleic acid sequence of the first attachment region would have the sequence of SEQ ID NO: 1 and when the second nucleotide in the target nucleic acid is adenine, the nucleic acid sequence of the second attachment region would have the sequence of SEQ ID NO: 5.

In embodiments, a complementary nucleic acid molecule may be bound by a detectable label. In alternate embodiments, a complementary nucleic acid is associated with a reporter complex comprising detectable labels.

The nucleotide sequence of a complementary nucleic acid is not limited; preferably it lacks substantial homology (e.g., 50% to 99.9%) with a known nucleotide sequence; this helps avoid undesirable hybridization of a complementary nucleic acid and a target nucleic acid.

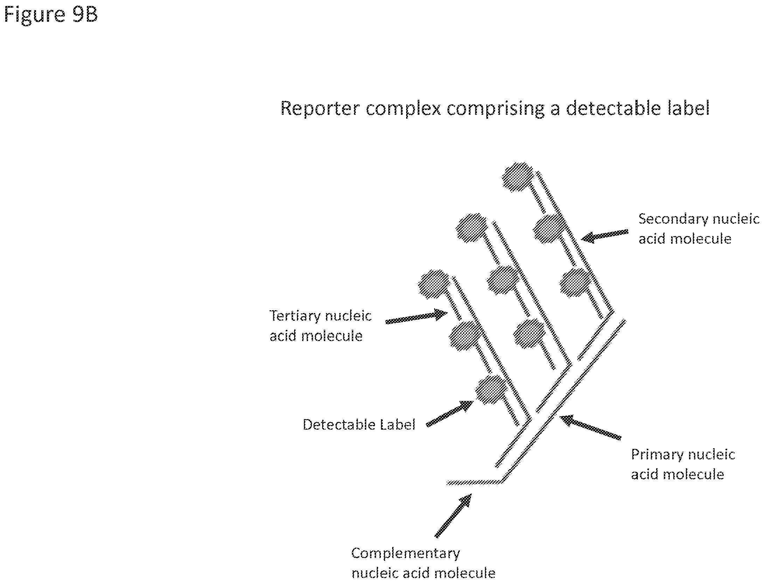

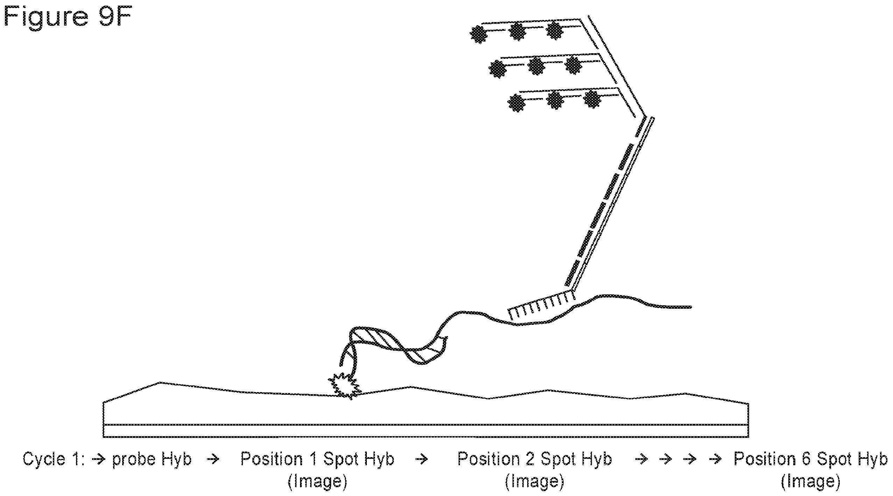

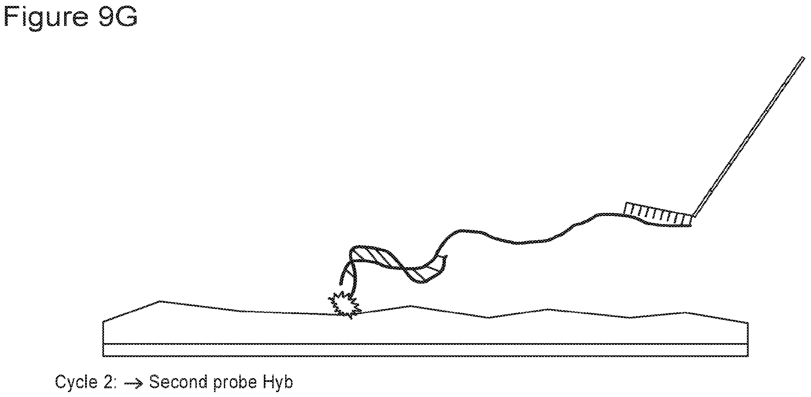

An example of the reporter complex useful in the disclosure is shown in Figure 9B. In this example, a complementary nucleic acid is linked to a primary nucleic acid molecule, which in turn is hybridized to a plurality of secondary nucleic acid molecules, each of which is in turn hybridized to a plurality of tertiary nucleic acid molecules having attached thereto one or more detectable labels.

In embodiments, a primary nucleic acid molecule may comprise about 90 nucleotides. A secondary nucleic acid molecule may comprise about 87 nucleotides. A tertiary nucleic acid molecule may comprise about 15 nucleotides.

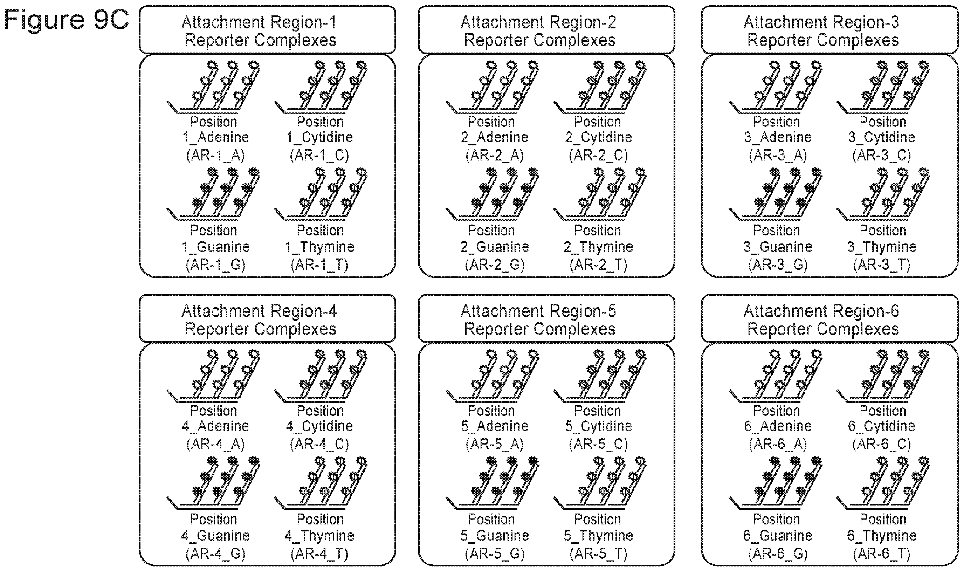

Figure 9C shows a population of exemplary reporter complexes. Included in the top left panel of Figure 9C are the four complexes that hybridize to attachment region 1 of a probe. There is one type of reporter complex for each possible nucleotide that can be present in nucleotide position 1 of a probe's target binding domain. Here, while performing a sequence method of the disclosure, if the position 1 of a probe's reporter domain is bound by a reporter complex having a "blue-colored" detectable label, then the first nucleotide in the target binding domain is identified as Adenine. Alternately, if the position 1 is bound by a reporter complex having a "green-colored" detectable label, then the first nucleotide in the target binding domain is identified as Thymine.

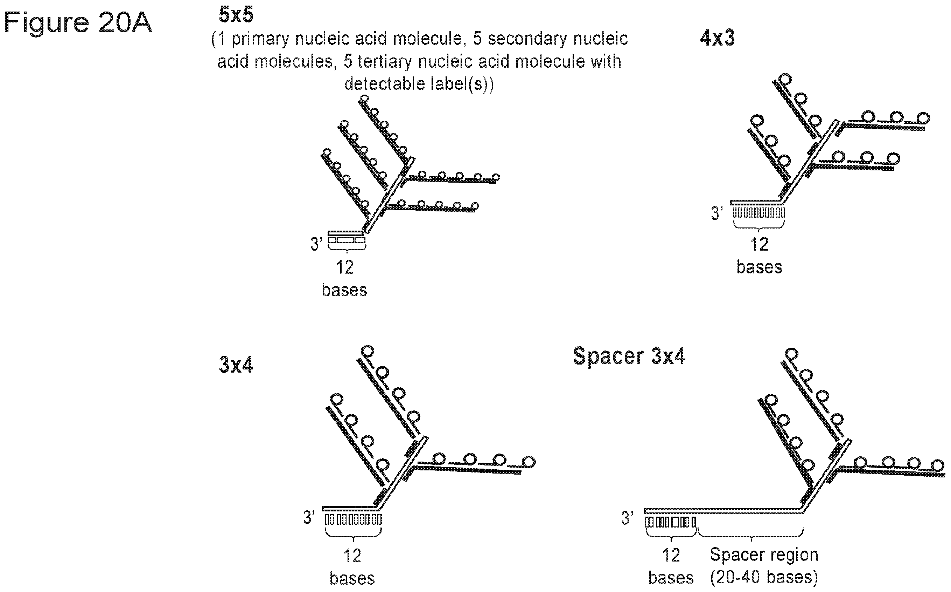

Reporter complexes can be of various designs. For example, a primary nucleic acid molecule can be hybridized to at least one (e.g., 1, 2, 3, 4, 5, 6, 7, 8, 9, 10, or more) secondary nucleic acid molecules. Each secondary nucleic acid molecule may be hybridized to at least one (e.g., 1, 2, 3, 4, 5, 6, 7, 8, 9, 10, or more) tertiary nucleic acid molecules. Exemplary reporter complexes are shown in Figure 20A. Here, the "4x3" reporter complex has one primary nucleic acid molecule (that is linked to a complementary nucleic acid molecule) hybridized to four secondary nucleic acid molecules, each of which is hybridized to three tertiary nucleic acid molecules (each comprising a detectable label). In this figure, each complementary nucleic acid of a complex is 12 nucleotides long ("12 bases"); however, the length of the complementary nucleic is non-limited and can be less than 12 or more than 12 nucleotides. The bottom-right complex includes a spacer region between its complementary nucleic acid and its primary nucleic acid molecule. The spacer is identified as 20 to 40 nucleotides long; however, the length of a spacer is non-limiting and it can be shorter than 20 nucleotides or longer than 40 nucleotides.

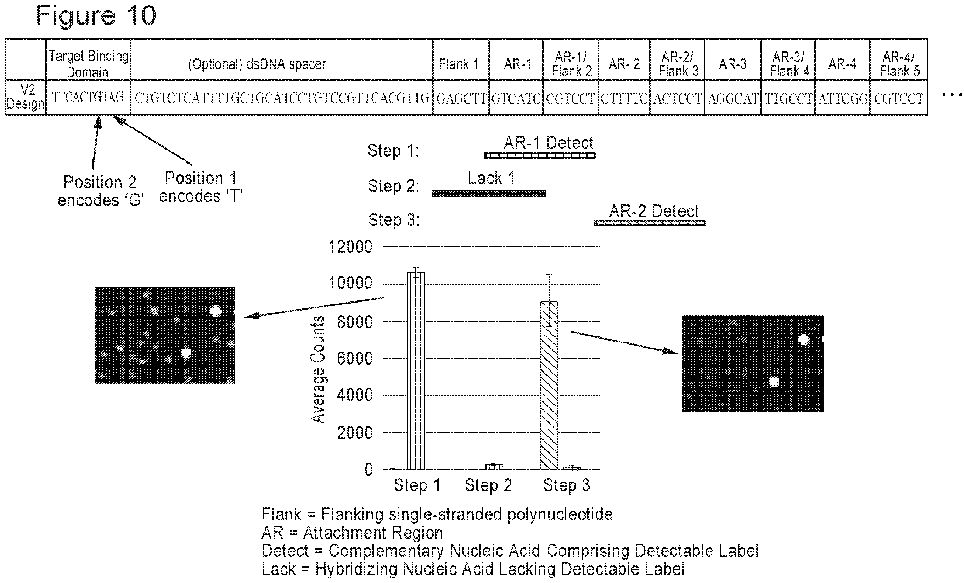

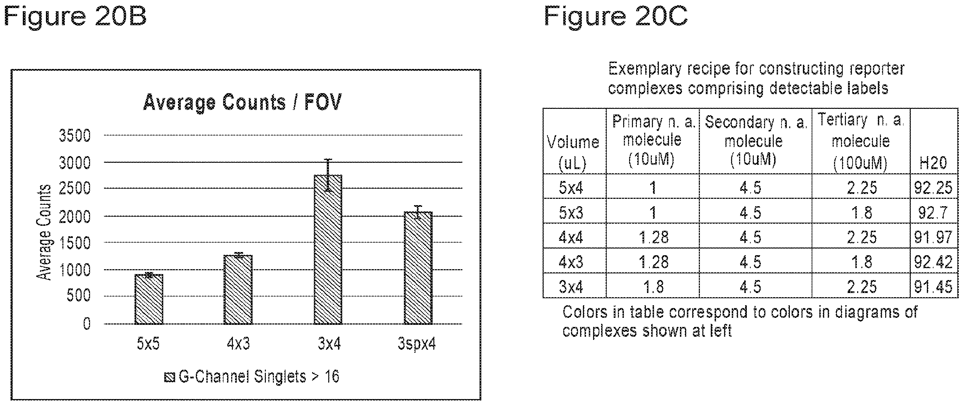

Figure 20B shows variable average (fluorescent) counts obtained from the four exemplary reporter complexes shown in Figure 20A. In Figure 20B, 10pM of biotinylated target template was attached onto a streptavidin-coated flow-cell surface, 10nM of a reporter complex was flowed onto the flow-cell; after a one minute incubation, the flow-cell was washed, the flow-cell was imaged, and fluorescent features were counted.

In embodiments, the reporter complexes are "pre-constructed". That is, each polynucleotide in the complex is hybridized prior to contacting the complex with a probe. An exemplary recipe for pre-constructing five exemplary reporter complexes is shown in Figure 20C.

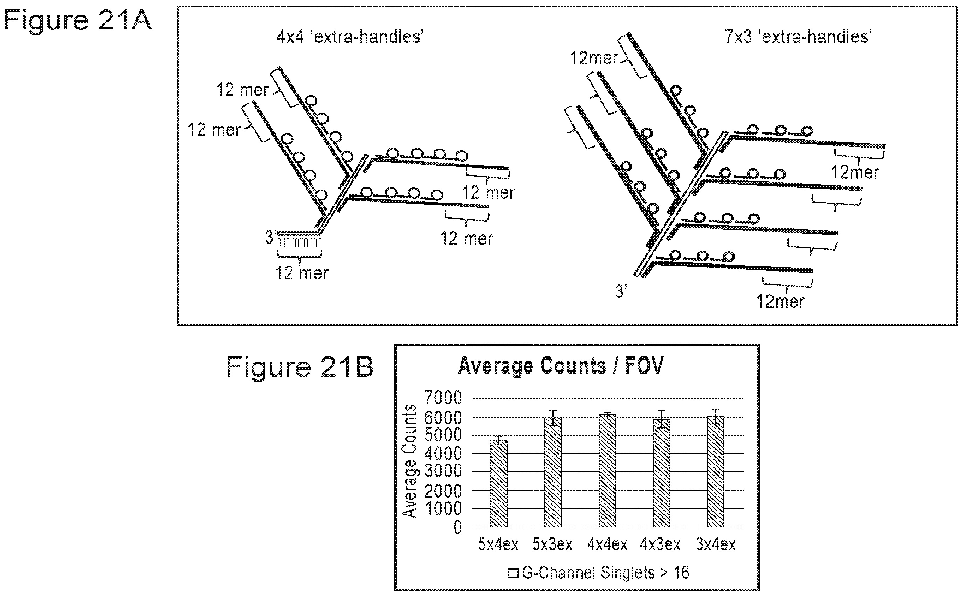

Figure 21A shows alternate reporter complexes in which the secondary nucleic acid molecules have "extra-handles" that are not hybridized to a tertiary nucleic acid molecule and are distal to the primary nucleic acid molecule. In this figure, each "extra-handle" is 12 nucleotides long ("12 mer"); however, their lengths are non-limited and can be less than 12 or more than 12 nucleotides. In embodiments, the "extra-handles" each comprise the nucleotide sequence of the complementary nucleic acid; thus, when a reporter complex comprises "extra-handles", the reporter complex can hybridize to a sequencing probe either via the reporter complex's complementary nucleic acid or via an "extra-handle." Accordingly, the likelihood that a reporter complex binds to a sequencing probe is increased. The "extra-handle" design may also improve hybridization kinetics. Without being bound to theory, the "extra-handles" essentially increase the effective concentration of the reporter complex's complementary nucleic acid.

Figure 21B shows variable average (fluorescent) counts obtained from the five exemplary reporter complexes having "extra-handles" using the procedure described for Figure 20B.

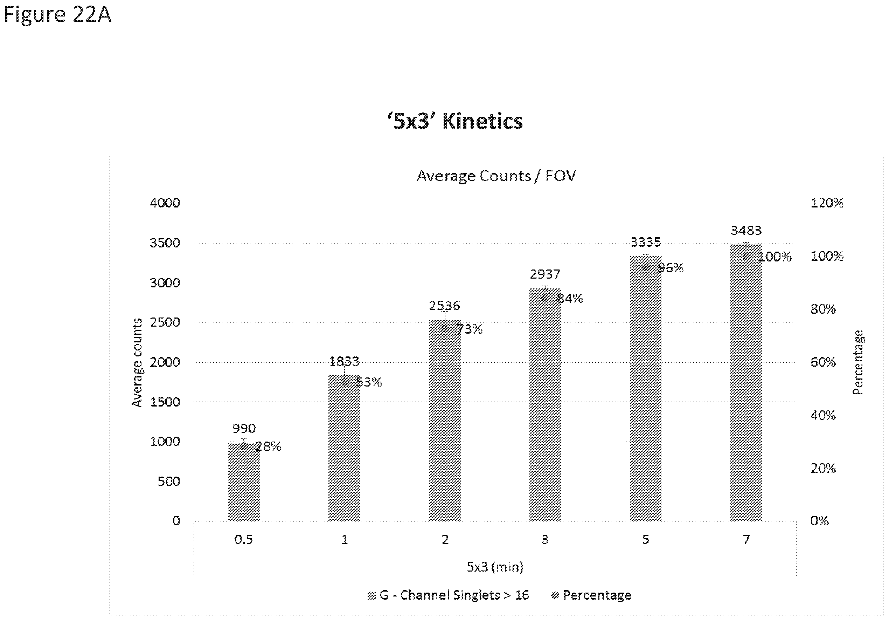

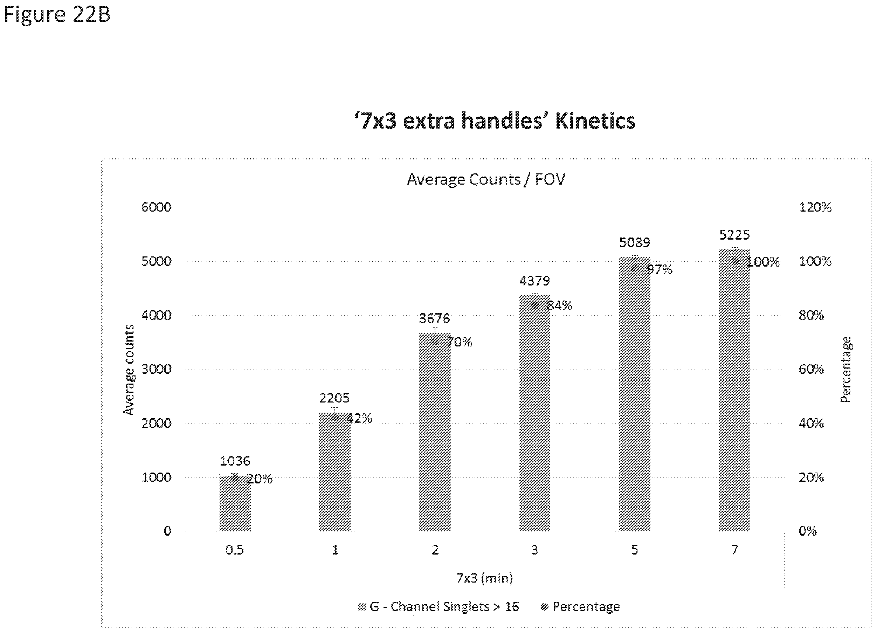

Figure 22A and 22B show hybridization kinetics and fluorescent intensities for two exemplary reporter complexes. By about 5 minutes, total counts start to plateau indicating that most reporter complex added have found an available target.

A detectable moiety, label or reporter can be bound to a complementary nucleic acid or to a tertiary nucleic acid molecule in a variety of ways, including the direct or indirect attachment of a detectable moiety such as a fluorescent moiety, colorimetric moiety and the like. One of skill in the art can consult references directed to labeling nucleic acids.

Examples of fluorescent moieties include, but are not limited to, yellow fluorescent protein (YFP), green fluorescent protein (GFP), cyan fluorescent protein (CFP), red fluorescent protein (RFP), umbelliferone, fluorescein, fluorescein isothiocyanate, rhodamine, dichlorotriazinylamine fluorescein, cyanines, dansyl chloride, phycocyanin, phycoerythrin and the like. Fluorescent labels and their attachment to nucleotides and/or oligonucleotides are described in many reviews, including Haugland, Handbook of Fluorescent Probes and Research Chemicals, Ninth Edition (Molecular Probes, Inc., Eugene, 2002); Keller and Manak, DNA Probes, 2nd Edition (Stockton Press, New York, 1993); Eckstein, editor, Oligonucleotides and Analogues: A Practical Approach (IRL Press, Oxford, 1991); and Wetmur, Critical Reviews in Biochemistry and Molecular Biology, 26:227-259 (1991). Particular methodologies applicable to the invention are disclosed in the following sample of references: U.S. Patent Nos. 4,757,141; 5,151,507; and 5,091,519. In one aspect, one or more fluorescent dyes are used as labels for labeled target sequences, *e.g.,* as disclosed by U.S. Patent Nos. 5,188,934 (4,7-dichlorofluorescein dyes); 5,366,860 (spectrally resolvable rhodamine dyes); 5,847,162 (4,7-dichlororhodamine dyes); 4,318,846 (ether-substituted fluorescein dyes); 5,800,996 (energy transfer dyes); Lee et al. 5,066,580 (xanthine dyes); 5,688,648 (energy transfer dyes); and the like. Labelling can also be carried out with quantum dots, as disclosed in the following patents and patent publications: U.S. Patent Nos. 6,322,901; 6,576,291; 6,423,551; 6,251,303; 6,319,426; 6,426,513; 6,444,143; 5,990,479; 6,207,392; 2002/0045045; and 2003/0017264. As used herein, the term "fluorescent label" comprises a signaling moiety that conveys information through the fluorescent absorption and/or emission properties of one or more molecules. Such fluorescent properties include fluorescence intensity, fluorescence lifetime, emission spectrum characteristics, energy transfer, and the like.

Commercially available fluorescent nucleotide analogues readily incorporated into nucleotide and/or oligonucleotide sequences include, but are not limited to, Cy3-dCTP, Cy3-dUTP, Cy5-dCTP, Cy5-dUTP (Amersham Biosciences, Piscataway, NJ), fluorescein- 12-dUTP, tetramethylrhodamine-6-dUTP, TEXAS RED™-5-dUTP, CASCADE BLUE™-7-dUTP, BODIPY TMFL-14-dUTP, BODIPY TMR-14-dUTP, BODIPY TMTR-14-dUTP, RHODAMINE GREEN™-5-dUTP, OREGON GREENR™ 488-5-dUTP, TEXAS RED™- 12-dUTP, BODIPY TM 630/650- 14-dUTP, BODIPY TM 650/665- 14-dUTP, ALEXA FLUOR™ 488-5-dUTP, ALEXA FLUOR™ 532-5-dUTP, ALEXA FLUOR™ 568-5-dUTP, ALEXA FLUOR™ 594-5-dUTP, ALEXA FLUOR™ 546- 14-dUTP, fluorescein- 12-UTP, tetramethylrhodamine-6-UTP, TEXAS RED™-5-UTP, mCherry, CASCADE BLUE™-7-UTP, BODIPY TM FL-14-UTP, BODIPY TMR-14-UTP, BODIPY TM TR-14-UTP, RHODAMINE GREEN™-5-UTP, ALEXA FLUOR™ 488-5-UTP, LEXA FLUOR™ 546- 14-UTP (Molecular Probes, Inc. Eugene, OR) and the like. Alternatively, the above fluorophores and those mentioned herein may be added during oligonucleotide synthesis using for example phosphoroamidite or NHS chemistry. Protocols are known in the art for custom synthesis of nucleotides having other fluorophores (*See,* Henegariu et al. (2000) Nature Biotechnol. 18:345). 2-Aminopurine is a fluorescent base that can be incorporated directly in the oligonucleotide sequence during its synthesis. Nucleic acid could also be stained, a priori, with an intercalating dye such as DAPI, YOYO- 1 , ethidium bromide, cyanine dyes (*e.g.,* SYBR Green) and the like.

Other fluorophores available for post-synthetic attachment include, but are not limited to, ALEXA FLUOR™ 350, ALEXA FLUOR™ 405, ALEXA FLUOR™ 430, ALEXA FLUOR™ 532, ALEXA FLUOR™ 546, ALEXA FLUOR™ 568, ALEXA FLUOR™ 594, ALEXA FLUOR™ 647, BODIPY 493/503, BODIPY FL, BODIPY R6G, BODIPY 530/550, BODIPY TMR, BODIPY 558/568, BODIPY 558/568, BODIPY 564/570, BODIPY 576/589, BODIPY 581/591, BODIPY TR, BODIPY 630/650, BODIPY 650/665, Cascade Blue, Cascade Yellow, Dansyl, lissamine rhodamine B, Marina Blue, Oregon Green 488, Oregon Green 514, Pacific Blue, Pacific Orange, rhodamine 6G, rhodamine green, rhodamine red, tetramethyl rhodamine, Texas Red (available from Molecular Probes, Inc., Eugene, OR), Cy2, Cy3, Cy3.5, Cy5, Cy5.5, Cy7 (Amersham Biosciences, Piscataway, NJ) and the like. FRET tandem fluorophores may also be used, including, but not limited to, PerCP-Cy5.5, PE-Cy5, PE-Cy5.5, PE-Cy7, PE-Texas Red, APC-Cy7, PE-Alexa dyes (610, 647, 680), APC-Alexa dyes and the like.

Metallic silver or gold particles may be used to enhance signal from fluorescently labeled nucleotide and/or oligonucleotide sequences (Lakowicz et al. (2003) BioTechniques 34:62).

Other suitable labels for an oligonucleotide sequence may include fluorescein (FAM, FITC), digoxigenin, dinitrophenol (DNP), dansyl, biotin, bromodeoxyuridine (BrdU), hexahistidine (6xHis), phosphor-amino acids (*e.g.,* P-tyr, P-ser, P-thr) and the like. In one embodiment the following hapten/antibody pairs are used for detection, in which each of the antibodies is derivatized with a detectable label: biotin/a-biotin, digoxigenin/a-digoxigenin, dinitrophenol (DNP)/a-DNP, 5-Carboxyfluorescein (FAM)/a-FAM.

Detectable labels described herein are spectrally resolvable. "Spectrally resolvable" in reference to a plurality of fluorescent labels means that the fluorescent emission bands of the labels are sufficiently distinct, *i.e.,* sufficiently non-overlapping, that molecular tags to which the respective labels are attached can be distinguished on the basis of the fluorescent signal generated by the respective labels by standard photodetection systems, *e.g.,* employing a system of band pass filters and photomultiplier tubes, or the like, as exemplified by the systems described in U.S. Patent Nos. 4,230,558; 4,811,218; or the like, or in Wheeless et al., pgs. 21-76, in Flow Cytometry: Instrumentation and Data Analysis (Academic Press, New York, 1985). In one aspect, spectrally resolvable organic dyes, such as fluorescein, rhodamine, and the like, means that wavelength emission maxima are spaced at least 20 nm apart, and in another aspect, at least 40 nm apart. In another aspect, chelated lanthanide compounds, quantum dots, and the like, spectrally resolvable means that wavelength emission maxima are spaced at least 10 nm apart, and in a further aspect, at least 15 nm apart.

## Sequencing Method

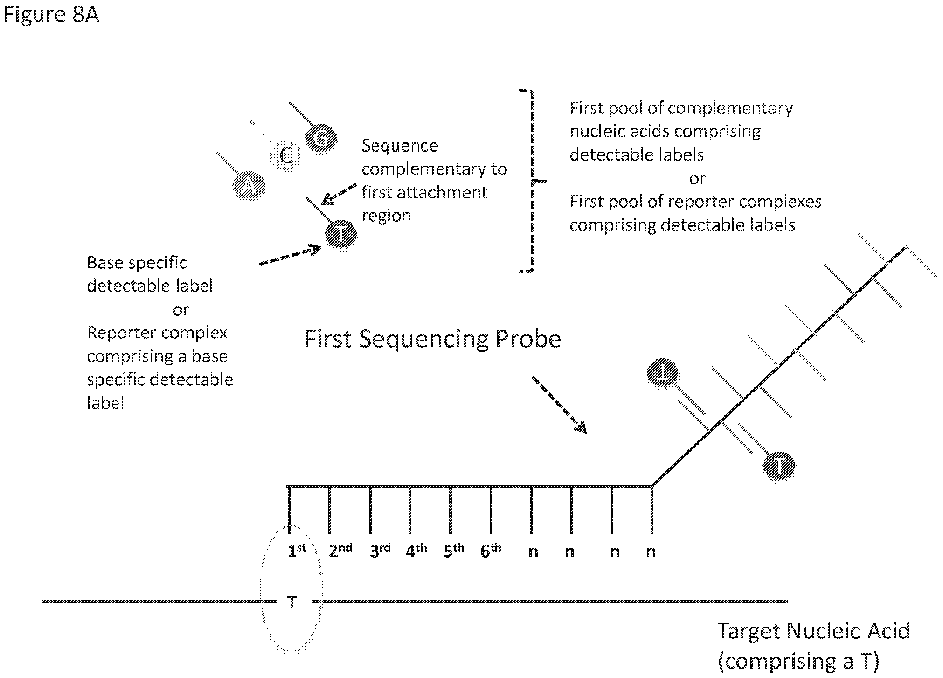

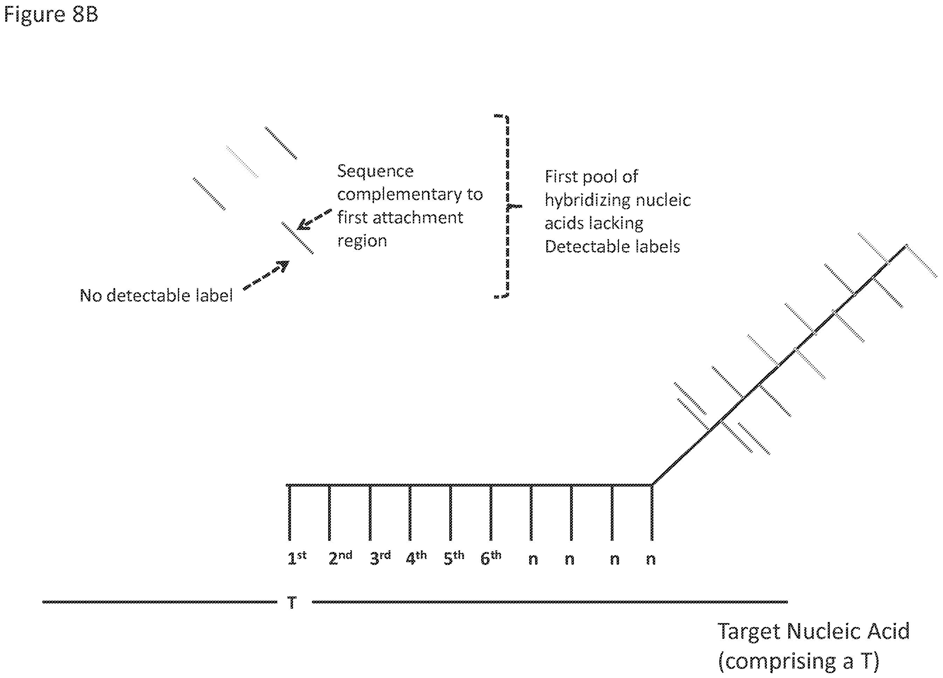

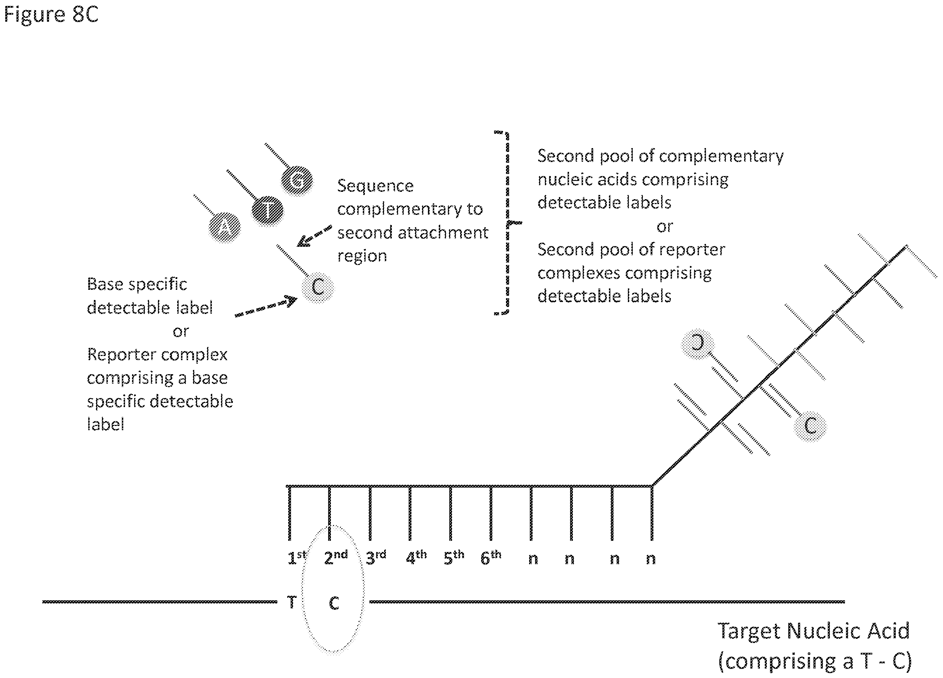

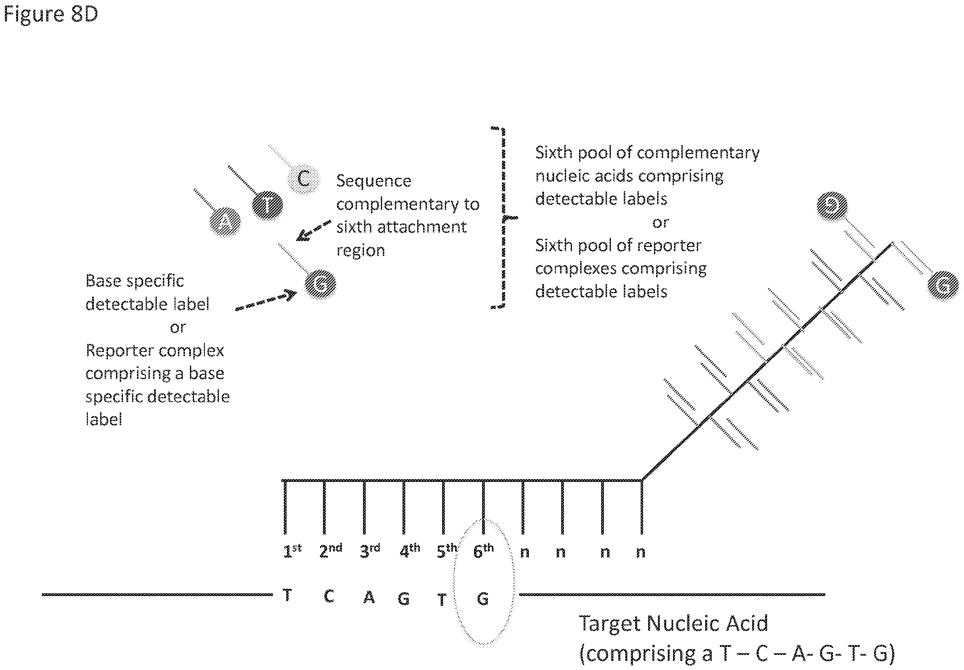

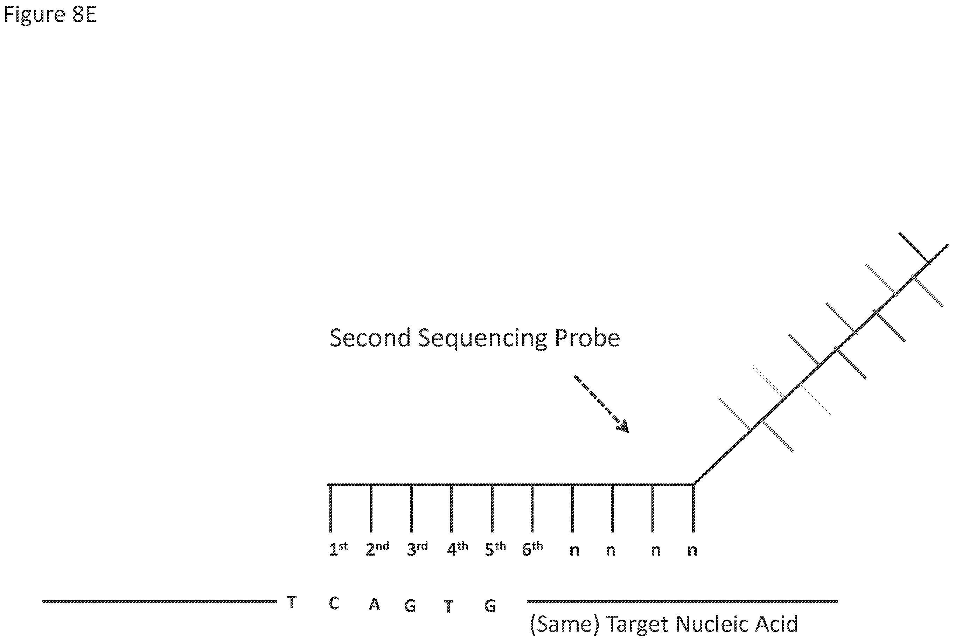

The present invention, as defined by the appended claims, and the disclosure relate to methods for sequencing a nucleic acid using a sequencing probe of the present invention or disclosure, respectively. Examples of the method are shown in Figures 8 to 12.

The method comprises reversibly hybridizing at least one sequencing probe to a target nucleic acid that is immobilized (*e.g.,* at one, two, three, four, five, six, seven, eight, nine, ten, or more positions) to a substrate.

The substrate can be any solid support known in the art, *e.g.,* a coated slide and a microfluidic device, which is capable of immobilizing a target nucleic acid. In certain embodiments, the substrate is a surface, membrane, bead, porous material, electrode or array. The target nucleic acid can be immobilized onto any substrate apparent to those of skill in the art.

In embodiments, the target nucleic acid is bound by a capture probe which comprises a domain that is complementary to a portion of the target nucleic acid. The portion may be an end of the target nucleic acid or not towards an end.

Exemplary useful substrates include those that comprise a binding moiety selected from the group consisting of ligands, antigens, carbohydrates, nucleic acids, receptors, lectins, and antibodies. The capture probe comprises a binding moiety capable of binding with the binding moiety of the substrate. Exemplary useful substrates comprising reactive moieties include, but are not limited to, surfaces comprising epoxy, aldehyde, gold, hydrazide, sulfhydryl, NHS-ester, amine, thiol, carboxylate, maleimide, hydroxymethyl phosphine, imidoester, isocyanate, hydroxyl, pentafluorophenyl-ester, psoralen, pyridyl disulfide or vinyl sulfone, polyethylene glycol (PEG), hydrogel, or mixtures thereof. Such surfaces can be obtained from commercial sources or prepared according to standard techniques. Exemplary useful substrates comprising reactive moieties include, but are not limited to, OptArray-DNA NHS group (Accler8), Nexterion Slide AL (Schott) and Nexterion Slide E (Schott).

In embodiments, the capture probe's binding moiety is biotin and the substrate comprises avidin (e.g., streptavidin). Useful substrates comprising avidin are commercially available including TB0200 (Accelr8), SAD6, SAD20, SAD100, SAD500, SAD2000 (Xantec), SuperAvidin (Array-It), streptavidin slide (catalog #MPC 000, Xenopore) and STREPTAVIDINnslide (catalog #439003, Greiner Bio-one).

In embodiments, the capture probe's binding moiety is avidin (*e.g.,* streptavidin) and the substrate comprises biotin. Useful substrates comprising biotin that are commercially available include, but are not limited to, Optiarray-biotin (Accler8), BD6, BD20, BD100, BD500 and BD2000 (Xantec). In embodiments, the capture probe's binding moiety can comprise a reactive moiety that is capable of being bound to the substrate by photoactivation. The substrate could comprise the photoreactive moiety, or the first portion of the nanoreporter could comprise the photoreactive moiety. Some examples of photoreactive moieties include aryl azides, such as N((2-pyridyldithio)ethyl)-4-azidosalicylamide; fluorinated aryl azides, such as 4-azido-2,3,5,6-tetrafluorobenzoic acid; benzophenone-based reagents, such as the succinimidyl ester of 4-benzoylbenzoic acid; and 5-Bromo-deoxyuridine.

In embodiments, the capture probe's binding moiety can be immobilized to the substrate via other binding pairs apparent to those of skill in the art.

After binding to the substrate, the target nucleic acid may be elongated by applying a force (*e.g.,* gravity, hydrodynamic force, electromagnetic force "electrostretching", flow-stretching, a receding meniscus technique, and combinations thereof) sufficient to extend the target nucleic acid.

The target nucleic acid may be bound by a second capture probe which comprises a domain that is complementary to a second portion of the target nucleic acid. The portion may be an end of the target nucleic acid or not towards an end. Binding of a second capture probe can occur after or during elongation of the target nucleic acid or to a target nucleic acid that has not been elongated. The second capture probe can have a binding as described above.

A capture probe may comprise or be associated with a detectable label, *i.e.,* a fiducial spot.

The capture probe is capable of isolating a target nucleic acid from a sample. Here, a capture probe is added to a sample comprising the target nucleic acid. The capture probe binds the target nucleic acid via the region of the capture probe that his complementary to a region of the target nucleic acid. When the target nucleic acid contacts a substrate comprising a moiety that binds the capture probe's binding moiety, the nucleic acid becomes immobilized onto the substrate.

To ensure that a user "captures" as many target nucleic acid molecules as possible from high fragmented samples, it is helpful to include a plurality of capture probes, each complementary to a different region of the target nucleic acid. For example, there may be three pools of capture probes, with a first pool complementary to regions of the target nucleic acid near its 5' end, a second pool complementary to regions in the middle of the target nucleic acid, and a third pool near its 3' end. This can be generalized to "n-regions-of-interest" per target nucleic acid. In this example, each individual pool of fragmented target nucleic acid bound to a capture probe comprising or bound to a biotin tag.

1/nth of input sample (where n = the number of distinct regions in target nucleic acid) is isolated for each pool chamber. The capture probe binds the target nucleic acid of interest. Then the target nucleic acid is immobilized, via the capture probe's biotin, to an avidin molecule adhered to the substrate. Optionally, the target nucleic acid is stretched, *e.g.,* via flow or electrostatic force. All n-pools can be stretched-and-bound simultaneously, or, in order to maximize the number of fully stretched molecules, pool 1 (which captures most 5' region) can be stretched and bound first; then pool 2, (which captures the middle-of-target region) is then can be stretched and bound; finally, pool 3 is can be stretched and bound.

The number of distinct capture probes required is inversely related to the size of target nucleic acid fragment. In other word, more capture probes will be required for a highly-fragmented target nucleic acid. For sample types with highly fragmented and degraded target nucleic acids (*e.g.,* Formalin-Fixed Paraffin Embedded Tissue) it may be useful to include multiple pools of capture probes. On the other hand, for samples with long target nucleic acid fragments, *e.g.,* in vitro obtained isolated nucleic acids, a single capture probe at a 5' end may be sufficient.

The region of the target nucleic acid between to two capture probes or after one capture probe and before a terminus of the target nucleic acid is referred herein as a "gap". The gap is a portion of the target nucleic acid that is available to be bound by a sequencing probe. The minimum gap is a target binding domain length (*e.g.,* 4 to 10 nucleotides) and a maximum gap is the majority of a whole chromosome.

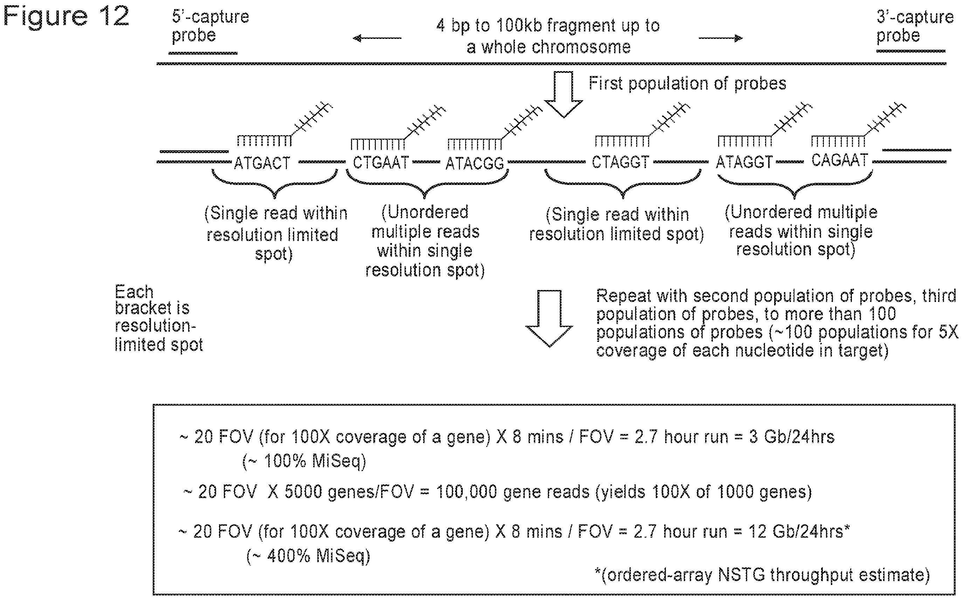

An immobilized target nucleic acid is shown in Figure 12. Here, the two capture probes are identified as "5' capture probe" and "3' capture probe"..

Figure 8A shows a schematic of a sequencing probe bound to a target nucleic acid. Here, the target nucleic acid has a thymidine (T). A first pool of complementary nucleic acids comprising a detectable label or reporter complexes is shown at the top, each member of the pool has a different detectable label (*e.g.,* thymidine is identified by a green signal) and a different nucleotide sequence. The first nucleotide in the target binding domain binds the T in the target nucleic acid. The first attachment regions of the probe include one or more nucleotide sequence(s) that specifies that the first nucleotide in the probe's target binding domain binds a thymidine. Thus, only the complementary nucleic acid for thymidine binds the first position of the barcode domain. As shown, a thymidine-encoding first complementary nucleic acid comprising a detectable label or reported complexes comprising detectable labels are bound to attachment regions in the first position of the probe's barcode domain.

The number of pools of complementary nucleic acids or reporter complexes is identical to the number of positions in the barcode domain. Thus, for a barcode domain having six positions, six pools will be cycled over the probes.

Alternately, prior to contacting a target nucleic acid with a probe, the probe may be hybridized at its first position to a complementary nucleic acid comprising a detectable label or a reporter complex. Thus, when contacted with its target nucleic acid, the probe is capable of emitting a detectable signal from its first position and it is unnecessary to provide a first pool of complementary nucleic acids or reporter complexes that are directed to the first position on the barcode domain.

Figure 8B continues the method shown in Figure 8A. Here, the first complementary nucleic acids (or reporter complexes) for thymidine that were bound to attachment regions in the first position of the barcode domain have been replaced with a first hybridizing nucleic acid for thymidine and lacking a detectable label. The first hybridizing nucleic acid for thymidine and lacking a detectable label displaces the previously-bound complementary nucleic acids comprising a detectable label or the previously-bound reporter complexes. Thereby, position 1 of the barcode domain no longer emits a detectable signal.

In embodiments, the complementary nucleic acids comprising a detectable label or reporter complexes may be removed from the attachment region but not replaced with a hybridizing nucleic acid lacking a detectable label. This can occur, for example, by adding a chaotropic agent, increasing the temperature, changing salt concentration, adjusting pH, and/or applying a hydrodynamic force. In these embodiments fewer reagents (*i.e.,* hybridizing nucleic acids lacking detectable labels) are needed.

Figure 8C continues the method of the claimed invention. Here, the target nucleic acid has a cytidine (C) following its thymidine (T). A second pool of complementary nucleic acids or reporter complexes is shown at the top, each member of the pool has a different detectable label and a different nucleotide sequence. Moreover, the nucleotide sequences for the complementary nucleic acids or complementary nucleic acids of the reporter complexes of the first pool are different from the nucleotide sequences for those of the second pool. However, the base specific detectable labels are common to the pools of complementary nucleic acids, *e.g.,* thymidines are identified by green signals. Here, the second nucleotide in the target binding domain binds the C in the target nucleic acid. The second attachment regions of the probe have a nucleotide sequence that specifies that the second nucleotide in the probe's target binding domain binds a cytidine. Thus, only the complementary nucleic acids comprising a detectable label or reporter complexes from the second pool and for cytidine binds the second position of the barcode domain. As shown, the cytidine-encoding second complementary nucleic acid or reporter complex is bound at the second position of the probe's barcode domain.

In embodiments, the steps shown in Figure 8C are subsequent to steps shown in Figure 8B. Here, once the first pool of complementary nucleic acids or reporter complexes (of Figure 8A) has been replaced with first hybridizing nucleic acids lacking a detectable label (in Figure 8B), then a second pool of complementary nucleic acids or reporter complexes is provided (as shown in Figure 8C). Alternately, the steps shown in Figure 8C are concurrent with steps shown in Figure 8B. Here, the first hybridizing nucleic acids lacking a detectable label (in Figure 8B) are provided simultaneously with a second pool of complementary nucleic acids or reporter complexes (as shown in Figure 8C).

Figure 8D continues the method shown in Figure 8C. Here, the first through fifth positions on the barcode domain were bound by complementary nucleic acids comprising a detectable labels or reporter complexes and have been replaced with hybridizing nucleic acids lacking detectable labels. The sixth position of the barcode domain is currently bound by a complementary nucleic acid comprising a detectable label or reporter complex, which identifies the sixth position in the target binding domain as being bound to a guanine (G).

As mentioned above, complementary nucleic acids comprising detectable labels or reporter complexes can be removed from attachment regions but not replaced with hybridizing nucleic acid lacking detectable labels.

If needed, the rate of detectable label exchange can be accelerated by incorporating small single-stranded oligonucleotides that accelerate the rate of exchange of detectable labels (*e.g.,* "Toe-Hold" Probes; *see, e.g.,* Seeling et al., "Catalyzed Relaxation of a Metastable DNA Fuel"; J. Am. Chem. Soc. 2006, 128(37), pp12211-12220).

It is possible to replace the complementary nucleic acids or reporter complexes on a final position on a barcode domain (the sixth position in Figure 8D); however, this may be unnecessary when a sequencing probe is to be replaced with another sequencing probe. Indeed, the sequencing probe of Figure 8D can now be de-hybridized and removed from the target nucleic acid and replaced with a second (overlapping or non-overlapping) sequencing probe that has not yet been bound by any complementary nucleic acids, as shown in Figure 8E. The probe in Figure 8E may be included in a second population of probes.

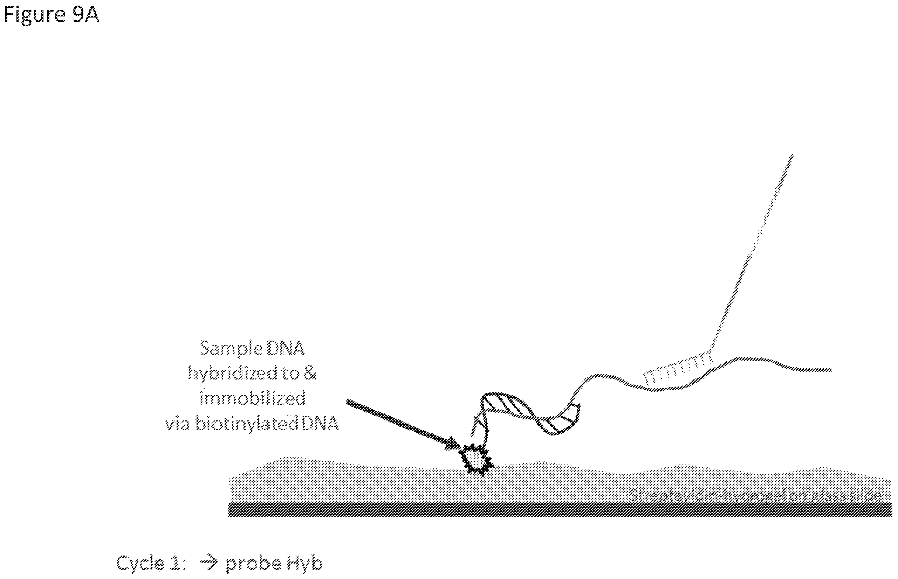

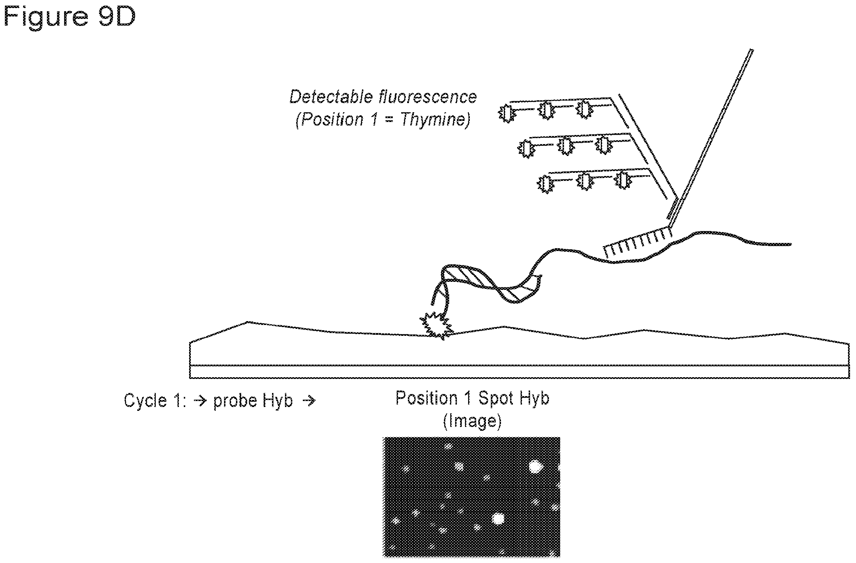

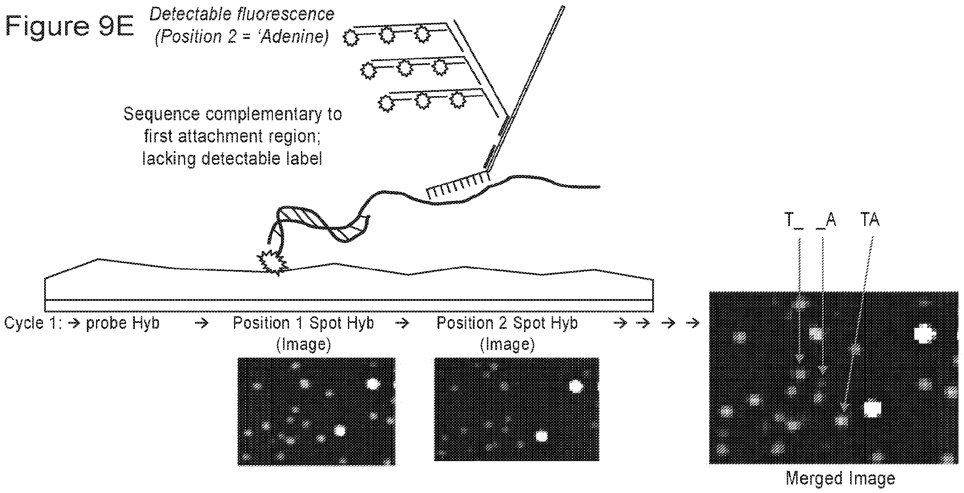

Like Figures 8A to 8E, Figures 9A and 9D to 9G show method steps; however, Figures 9A and 9D to 9G clearly show that reporter complexes (comprising detectable labels) are bound to attachment regions of sequencing probes. Figures 9D and 9E show fluorescent signals emitted from probes hybridized to reporter complexes. Figures 9D and 9E show that the target nucleic acid has a sequence of "T-A".

Figure 10 summarizes the steps shown in Figures 9D and 9E. At the top of the figure is shown the nucleotide sequence of an exemplary probe and identifies significant domains of the probe. The probe includes an optional double-stranded DNA spacer between its target binding domain and its barcode domain. The barcode domain comprises, in order, a "Flank 1" portion, an "AR-1" portion, an "AR-1/Flank 2" portion, an "AR-2" portion, and an "AR-2/Flank 3" portion. In Step 1, the "AR-1 Detect" is hybridized to the probe's "AR-1" and "AR-1/Flank 2" portions. "AR-1 Detect" corresponds to a reporter complex or complementary nucleic acid comprising a detectable label that encodes a first position thymidine. Thus, Step 1 corresponds to Figure 9D. In Step 2, the "Lack 1" is hybridized to the probe's "Flank 1" and "AR-1" portions. "Lack 1" corresponds to the hybridizing nucleic acid lacking a detectable label that is specific to the probe's first attachment region (as shown in Figure 9E as a black bar covering the first attachment region). By hybridizing to the "Flank 1" position, which is 5' to the reporter complex or complementary nucleic acid, the hybridizing nucleic acid more efficiently displaces the reporter complex/complementary nucleic acid from the probe. The "Flank" portions are also known as "Toe-Holds". In Step 3, the "AR-2 Detect" is hybridized to the probe's "AR-2" and "AR-2/Flank 3" portions. "AR-2 Detect" corresponds to a reporter complex or complementary nucleic acid comprising a detectable label that encodes a second position Guanine. Thus, Step 3 corresponds to Figure 9E. In this embodiment, hybridizing nucleic acid lacking a detectable label and complementary nucleic acids comprising detectable labels/reporter complexes are provided sequentially.

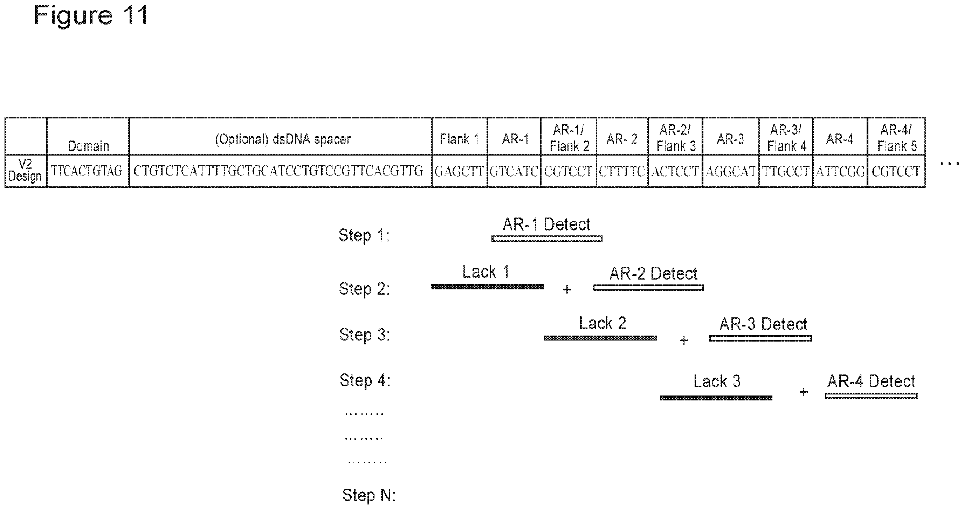

Alternately, hybridizing nucleic acid lacking a detectable label and complementary nucleic acids comprising detectable labels/reporter complexes are provided concurrently. This alternate embodiment is shown in Figure 11. In Step 2, the "Lack 1" (hybridizing nucleic acid lacking a detectable label) is provided along with the "AR-2 Detect" (reporter complex that encodes a second position Guanine). This alternate embodiment may be more time effective that the embodiment illustrated in Figure 10 because it combines two steps into one.

Figure 12 illustrates the methods herein described. Here, a target nucleic acid is captured and immobilized at two positions, thereby producing a "gap" to which a probe is able to bind. A first population of probes is hybridized onto the target nucleic acid and detectable labels are detected. The initial steps are repeated with a second population of probes, a third population of probes, to more than 100 populations of probes. Use of about 100 populations of probes provides about 5X coverage of each nucleotide in a target nucleic acid. Figure 12 provides estimated rates of read times based on the time required to detect signals from one Field of View (FOV).

The distribution of probes along a length of target nucleic acid is critical for resolution of detectable signal. As discussed above, the resolution limit for two detectable labels is about 600 nucleotides. Preferably, each sequencing probe in a population of probes will bind no closer than 600 nucleotides from each other. As discussed above, 600 nucleotides is the resolution limit of a typical sequencing apparatus. In this case, a sequencing probe will provide a single read; this is shown in Figure 12 in the left-most resolution-limited spot.

Randomly, but in part depending on the length of the target binding domain, the Tm of the probes, and concentration of probes applied, it is possible for two distinct sequencing probes in a population to bind within 600 nucleotides of each other. In this case, unordered multiple reads will emit from a single resolution-limited spot; this is shown in Figure 12 in the second resolution-limited spot.

Alternately or additionally, the concentration of sequencing probes in a population may be reduced to decrease coverage of probes in a specific region of a target nucleic acid, *e.g.,* to above the resolution limit of the sequencing apparatus, thereby producing a single read from a resolution-limited spot.

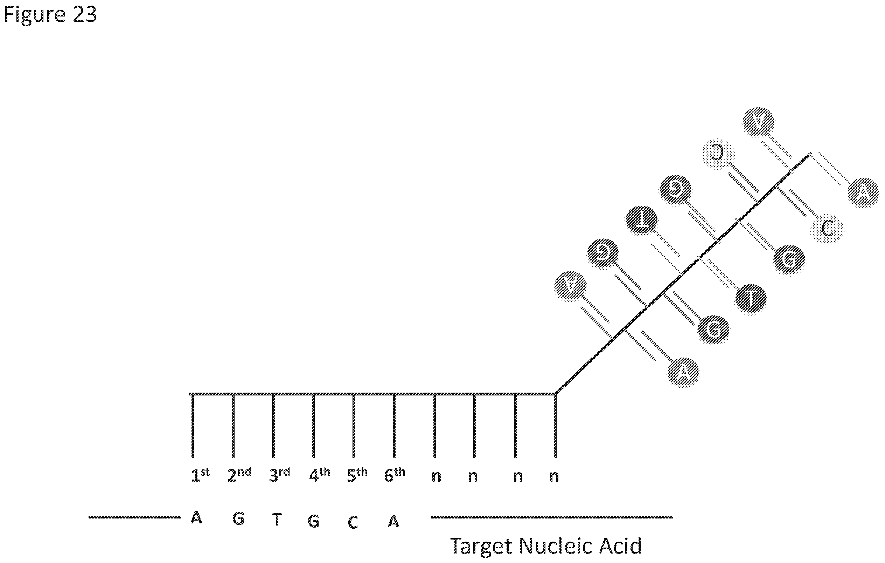

Figure 23 shows a schematic of a sequencing probe distinct from that used in Figures 8 through 12. Here, each position on a barcode domain is bound by complementary nucleic acids comprising detectable labels or by reporter complexes. Thus, in this example, a six nucleotide sequence can be read without needing to sequentially replace complementary nucleic acids. Use of this sequencing probe would reduce the time to obtain sequence information since many steps of the described method are omitted. However, this probe would benefit from detectable labels that are non-overlapping, *e.g.,* fluorophores are excited by non-overlapping wavelengths of light or the fluorophores emit non-overlapping wavelengths of light.

The method further comprising steps of assembling each identified linear order of nucleotides for each region of the immobilized target nucleic acid, thereby identifying a sequence for the immobilized target nucleic acid. The steps of assembling uses a non-transitory computer-readable storage medium with an executable program stored thereon. The program instructs a microprocessor to arrange each identified linear order of nucleotides for each region of the target nucleic acid, thereby obtaining the sequence of the nucleic acid. Assembling can occur in "real time", *i.e.,* while data is being collected from sequencing probes rather than after all data has been collected.

Any of the above aspects and embodiments can be combined with any other aspect or embodiment as disclosed here in the Summary and/or Detailed Description sections.

**Definitions:**

In certain exemplary embodiments, the terms "annealing" and "hybridization," as used herein, are used interchangeably to mean the formation of a stable duplex. In one aspect, stable duplex means that a duplex structure is not destroyed by a stringent wash under conditions such as a temperature of either about 5 °C below or about 5 °C above the Tm of a strand of the duplex and low monovalent salt concentration, *e.g.,* less than 0.2 M, or less than 0.1 M or salt concentrations known to those of skill in the art. The term "perfectly matched," when used in reference to a duplex means that the polynucleotide and/or oligonucleotide strands making up the duplex form a double stranded structure with one another such that every nucleotide in each strand undergoes Watson-Crick base pairing with a nucleotide in the other strand. The term "duplex" comprises, but is not limited to, the pairing of nucleoside analogs, such as deoxyinosine, nucleosides with 2-aminopurine bases, PNAs, and the like, that may be employed. A "mismatch" in a duplex between two oligonucleotides means that a pair of nucleotides in the duplex fails to undergo Watson-Crick bonding.

As used herein, the term "hybridization conditions," will typically include salt concentrations of less than about 1 M, more usually less than about 500 mM and even more usually less than about 200 mM. Hybridization temperatures can be as low as 5 °C, but are typically greater than 22 °C, more typically greater than about 30 °C, and often in excess of about 37 °C. Hybridizations are usually performed under stringent conditions, *e.g.,* conditions under which a probe will specifically hybridize to its target subsequence. Stringent conditions are sequence-dependent and are different in different circumstances. Longer fragments may require higher hybridization temperatures for specific hybridization. As other factors may affect the stringency of hybridization, including base composition and length of the complementary strands, presence of organic solvents and extent of base mismatching, the combination of parameters is more important than the absolute measure of any one alone.

Generally, stringent conditions are selected to be about 5 °C lower than the Tm for the specific sequence at a defined ionic strength and pH. Exemplary stringent conditions include salt concentration of at least 0.01 M to no more than 1 M Na ion concentration (or other salts) at a pH 7.0 to 8.3 and a temperature of at least 25 °C. For example, conditions of 5X SSPE (750 mM NaCl, 50 mM Na phosphate, 5 mM EDTA, pH 7.4) and a temperature of 25-30 °C are suitable for allele-specific probe hybridizations. For stringent conditions, see for example, Sambrook, Fritsche and Maniatis, "Molecular Cloning A Laboratory Manual, 2nd Ed." Cold Spring Harbor Press (1989) and Anderson Nucleic Acid Hybridization, 1st Ed., BIOS Scientific Publishers Limited (1999). As used herein, the terms "hybridizing specifically to" or "specifically hybridizing to" or similar terms refer to the binding, duplexing, or hybridizing of a molecule substantially to a particular nucleotide sequence or sequences under stringent conditions.

Detectable labels associated with a particular position of a probe can be "readout" (e.g., its fluorescence detected) once or multiple times; a "readout" may be synonymous with the term "basecall". Multiple reads improve accuracy. A target nucleic acid sequence is "read" when a contiguous stretch of sequence information derived from a single original target molecule is detected; typically, this is generated via multi-pass consensus (as defined below). As used herein, the term "coverage" or "depth of coverage" refers to the number of times a region of target has been sequenced (via discrete reads) and aligned to a reference sequence. Read coverage is the total number of reads that map to a specific reference target sequence; base coverage is the total number of basecalls made at a specific genomic position.

As used in herein, a "hybe and seq cycle" refers to all steps required to detect each attachment region on a particular probe or population of probes. For example, for a probe capable of detecting six positions on a target nucleic acid, one "hybe and seq cycle" will include, at least, hybridizing the probe to the target nucleic acid, hybridizing complementary nucleic acids/reporter complexes to attachment region at each of the six positions on the probe's barcode domain, and detecting the detectable labels associated with each of the six positions.

The term "k-mer probe" is synonymous with a probe of the present invention or disclosure.

When two or more sequences from discrete reads are aligned, the overlapping portions can be combined to create a single consensus sequence. In positions where overlapping portions have the same base (a single column of the alignment), those bases become the consensus. Various rules may be used to generate the consensus for positions where there are disagreements among overlapping sequences. A simple majority rule uses the most common base in the column as the consensus. A "multi-pass consensus" is an alignment of all discrete probe readouts from a single target molecule. Depending on the total number of cycles of probe populations/polls applied, each base position within a single target molecules can be queried with different levels of redundancy or overlap; generally, redundancy increases the confidence level of a basecall.

The "Raw Accuracy" is a measure of system's inherent ability to correctly identify a base. Raw accuracy is dependent on sequencing technology. "Consensus Accuracy" is a measure of system's ability to correctly identify a base with the use of additional reads and statistical power. "Specificity" refers to the percentage of reads that map to the intended targets out of total reads per run. "Uniformity" refers to the variability in sequence coverage across target regions; high uniformity correlates with low variability. This feature is commonly reported as the fraction of targeted regions covered by ≥20% of the average coverage depth across all targeted regions. Stochastic errors (i.e., intrinsic sequencing chemistry errors) can be readily corrected with 'multi-pass' sequencing of same target nucleic acid; given a sufficient number of passes, substantially 'perfect consensus' or 'error-free' sequencing can be achieved. The methods described herein may be implemented and/or the results recorded using any device capable of implementing the methods and/or recording the results. Examples of devices that may be used include but are not limited to electronic computational devices, including computers of all types. When the methods described herein are implemented and/or recorded in a computer, the computer program that may be used to configure the computer to carry out the steps of the methods may be contained in any computer readable medium capable of containing the computer program. Examples of computer readable medium that may be used include but are not limited to diskettes, CD-ROMs, DVDs, ROM, RAM, non-transitory computer-readable media, and other memory and computer storage devices. The computer program that may be used to configure the computer to carry out the steps of the methods, assemble sequence information, and/or record the results may also be provided over an electronic network, for example, over the internet, an intranet, or other network.

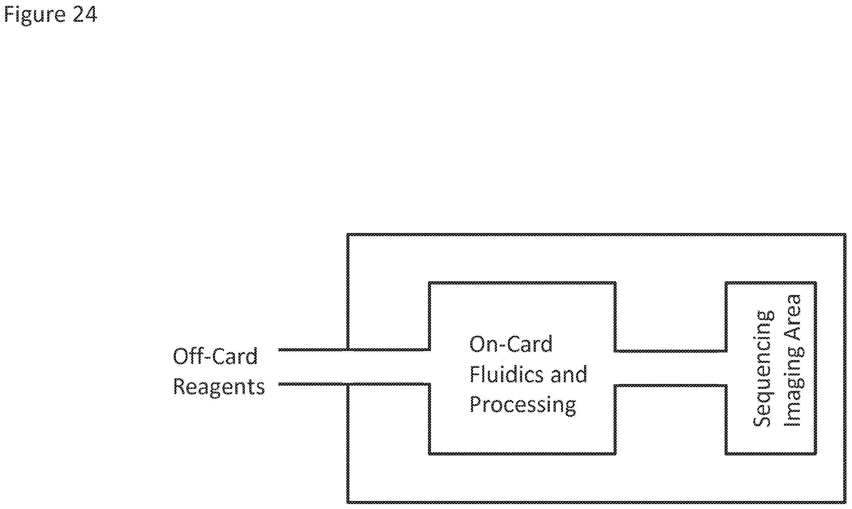

A "Consumable Sequencing Card" (Figure 24) can be incorporated into a fluorescence imaging device known in the art. Any fluorescence microscope with a number of varying features is capable of performing this sequencing readout. For instance: wide-field lamp, laser, LED, multi-photon, confocal or total-internal reflection illumination can be used for excitation and/or detection. Camera (single or multiple) and/or Photomultiplier tube (single or multiple) with either filter-based or grating-based spectral resolution (one or more spectrally resolved emission wavelengths) are possible on the emission-detection channel of the fluorescence microscope. Standard computers can control both the Consumable Sequencing Card, the reagents flowing through the Card, and detection by the fluorescence microscope.

The sequencing data can be analyzed by any number of standard next-generation-sequencing assemblers (*see, e.g.,* Wajid and Serpedin, "Review of general algorithmic features for genome assemblers for next generation sequencers" Genomics, proteomics & bioinformatics, 10 (2), 58-73, 2012). The sequencing data obtained within a single diffraction limited region of the microscope is "locally-assembled" to generate a consensus sequence from the multiple reads within a diffraction spot. The multiple diffraction spot assembled reads are then mapped together to generate contiguous sequences representing the entire targeted gene set, or a de-novo assembly of entire genome(s).

Additional teaching relevant to the present invention and disclosure are described in one or more of the following: U.S. 8,148,512, U.S. 7,473,767, U.S. 7,919,237, U.S. 7,941,279, U.S. 8,415,102, U.S. 8,492,094, U.S. 8,519,115, U.S. 2009/0220978, U.S. 2009/0299640, U.S. 2010/0015607, U.S. 2010/0261026, U.S. 2011/0086774, U.S. 2011/0145176, U.S. 2011/0201515, U.S. 2011/0229888, U.S. 2013/0004482, U.S. 2013/0017971, U.S. 2013/0178372, U.S. 2013/0230851, U.S. 2013/0337444, U.S. 2013/0345161, U.S. 2014/0005067, U.S. 2014/0017688, U.S. 2014/0037620, U.S. 2014/0087959, U.S. 2014/0154681, and U.S. 2014/0162251.

## EXAMPLES

### Example 1: The method of sequencing a target nucleic acid is rapid

Below is described the timing for steps in the methods of the present invention and as shown in Figures 8 to 12.

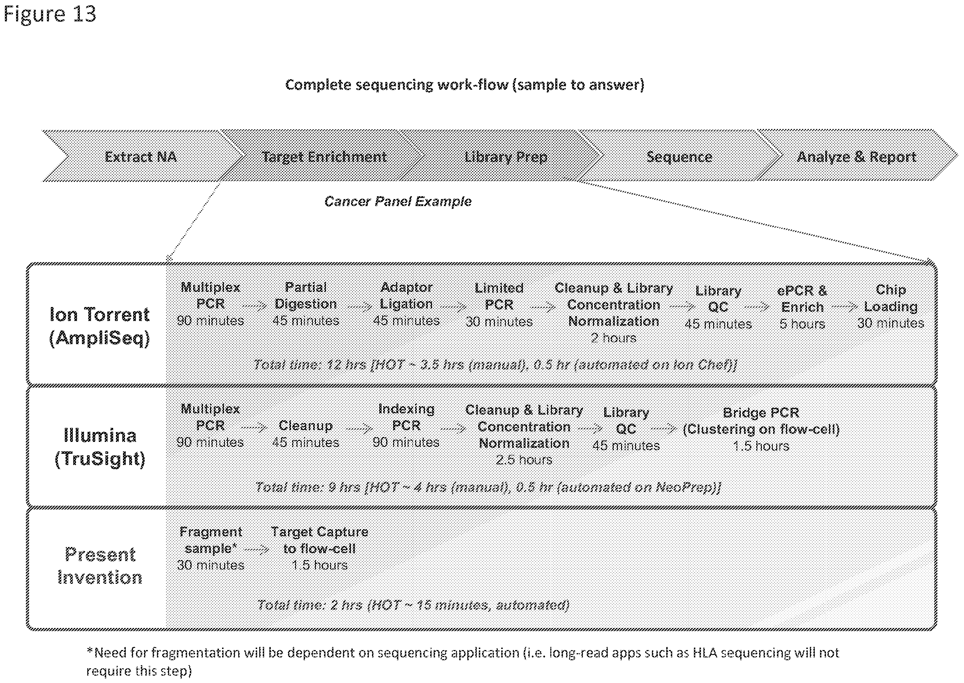

The present invention requires minimal sample preparation. For example, as shown in Figure 13, nucleic acids in a sample can begin to be read after 2 hours or less or preparation time; this is significantly less time required for Ion Torrent (AmpliSeq™) or Illumina (TruSight) sequencing, which, respectively, require about 12 or 9 hours of preparation time.

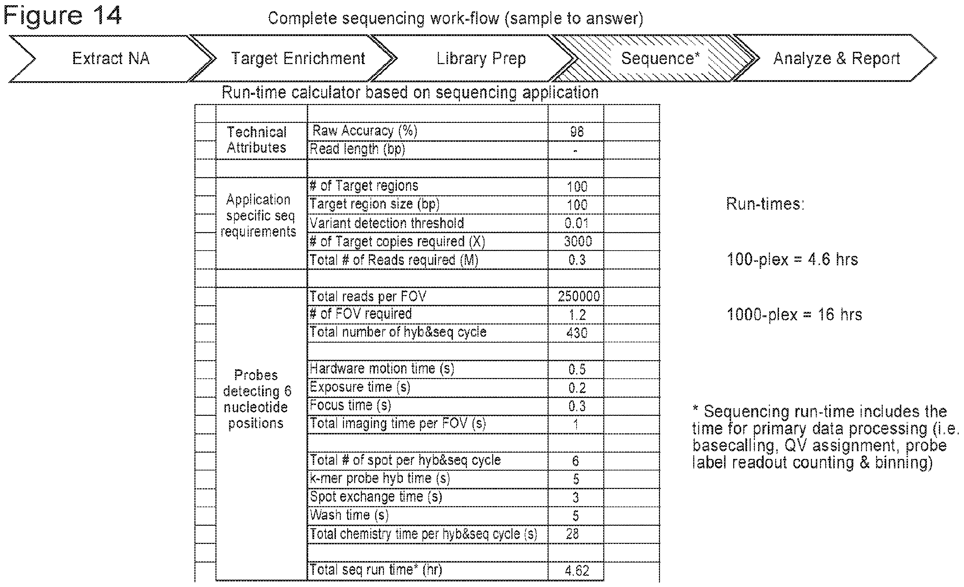

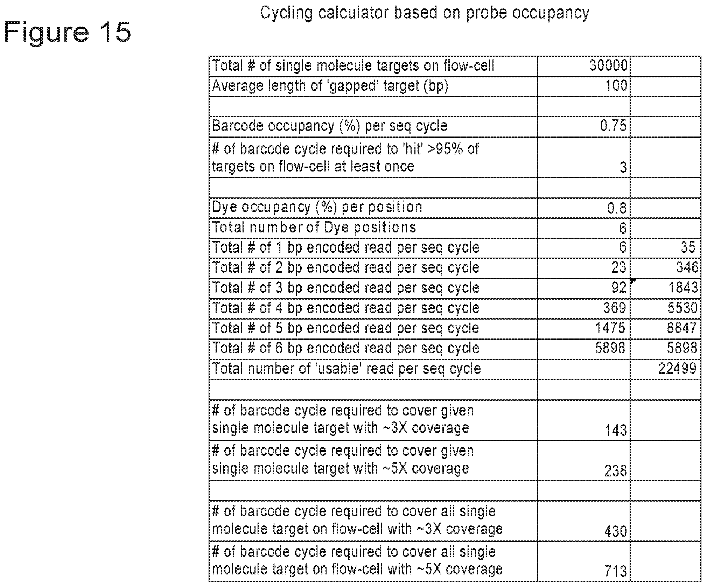

Calculations for an exemplary run are shown in Figure 14 and calculations for cycling times are shown in Figure 15.

Binding a population of probes to an immobilized target nucleic acid takes about sixty seconds. This reaction can be accelerated by utilizing multiple copies of the target binding domain on the synthetic backbone. With microfluidic-controlled fluid exchange device, washing away un-bound probes takes about a half a second.

Adding a first pool of complementary nucleic acids (comprising a detectable label) and binding them to attachment regions in the first position of the barcode domain takes about fifteen seconds. Each field of view (FOV) is imaged for four different colors, each color representing a single-base. Fiducial spots placed on a 5' capture probe or 3' capture probe (or both) may be helpful for reading only those optical barcodes in-a-line (consistent with the presence of gapped target nucleic acid) between the two locations. Fiducial spots can also be added to each field of view in order to generate equal alignment of images upon successive steps in the sequencing process. All four images can be obtained at a single FOV and then the optical reading device may move to a new FOV, or take all FOV in one color then reimage in a second color. A single FOV can be read in about a half a second. It takes about a half a second to move to a next FOV. Therefore, the time to read "n" FOV's equals "n" times 1 sec).

The complementary nucleic acids having detectable labels are removed from the first position of the barcode domain by addition of heat or washing with excess of complementary nucleic acids lacking detectable labels. If needed, the rate of detectable label exchange can be accelerated by incorporating small single-stranded oligonucleotides that accelerate the rate of exchange of detectable labels (*e.g.,* "Toe-Hold" Probes; *see, e.g.,* Seeling et al., "Catalyzed Relaxation of a Metastable DNA Fuel"; J. Am. Chem. Soc. 2006, 128(37), pp 12211-12220). A FOV can be reimaged to confirm that all complementary nucleic acids having detectable labels are removed before moving continuing. This takes about fifteen seconds. This step can be repeated until background signal levels are reached.

The above steps are repeated or the remaining positions in the probes' barcode domain.

The total time to read equals m (bases read) *times* (15 sec + n FOVs *times* 1 sec + 15 sec). For example, when the number of positions in the barcode domain is 6 and 20 FOVs, the time to read equals 6 X (30 + 20 + 15) or 390 seconds.

Probes of the first population are de-hybridized. This takes about sixty seconds.

The above steps are repeated for second and subsequent populations of probes. If populations of sequencing probes are organized by melting temperature (Tm), each population of probes will require multiple hybridizations to ensure that each base is covered to required depth (this is driven by error rate). Moreover, by analyzing the hybridization reads during a run, it is possible to recognize each individual gene that is being sequenced well before the entire sequence is actually determined. Hence cycling can be repeated until a particular desired error-frequency (or coverage) is met.

Using the timing described above, together with some gapped-nucleic acid binding density estimates, throughput of a Nanostring (NSTG)-Next Generation Sequencer of the present invention can be estimated.

Net throughput of sequencer is given by:

$$\text{Fractional-Base-Occupancy} \ X \ \langle\text{gap-length}\rangle \ X \ \text{number-of-gaps-per-FOV} \ X$$
$$\text{number-of-bases-per-optical-barcode} \ / \ [ \ 60 \ \text{sec (hybridizing probes to target}$$
$$\text{nucleic acid)} + 0.5 \ \text{sec (wash)} + \text{m: positions in the barcode domain} \ X \ (15 \ \text{sec}$$
$$\text{(binding complementary nucleic acids)} + \text{nfovsX1} + 15 \ \text{sec (unbinding}$$
$$\text{complementary nucleic acids))} + 60 \ \text{sec (de-hybridizing probes to target nucleic}$$
$$\text{acid)} \ ]$$

Therefore, in an example, a total "cycle" for a single gapped-nucleic acid (adding together from the method shown in Figure 10):
60 sec (hybridizing probes to target nucleic acid) + 0.5 sec (wash) + m-bases X (15 sec (binding complementary nucleic acids) + nFOVs *times* 1 + 15 sec (unbinding complementary nucleic acids)) + 60 sec (de-hybridizing probes to target nucleic acid). Using m = 6, nFOVs = 20, yields time = 60 + 0.5 + 390 + 60 = 510.5 sec.

Assuming: 1% occupancy of the gapped-nucleic acid region, 4000 bases per gap, and 5000 gapped nucleic-acid fragments per FOV and an m of 6 and nFOVs of 20 (as described above) yields a net throughput of:

$$0.01 X \ 4000 \ X \ 5000 \ X \ 20 = 4,000,000 \ \text{6-base reads per } 510.5 \ \text{secs} = 47,012.73$$
$$\text{bases/sec.}$$

Therefore, in this example, a net throughput per 24 hours of continuous measurement = 4.062 Gigabases (Gb) per day. Alternate estimates up to 12 Gb per day. *See* Figure 12.

As shown in Figure 14, the run-time required to sequence 100 different target nucleic acids (a "100-plex") is about 4.6 hours; the run-time required to sequence 1000 different target nucleic acids (a "1000-plex") is about 16 hours.

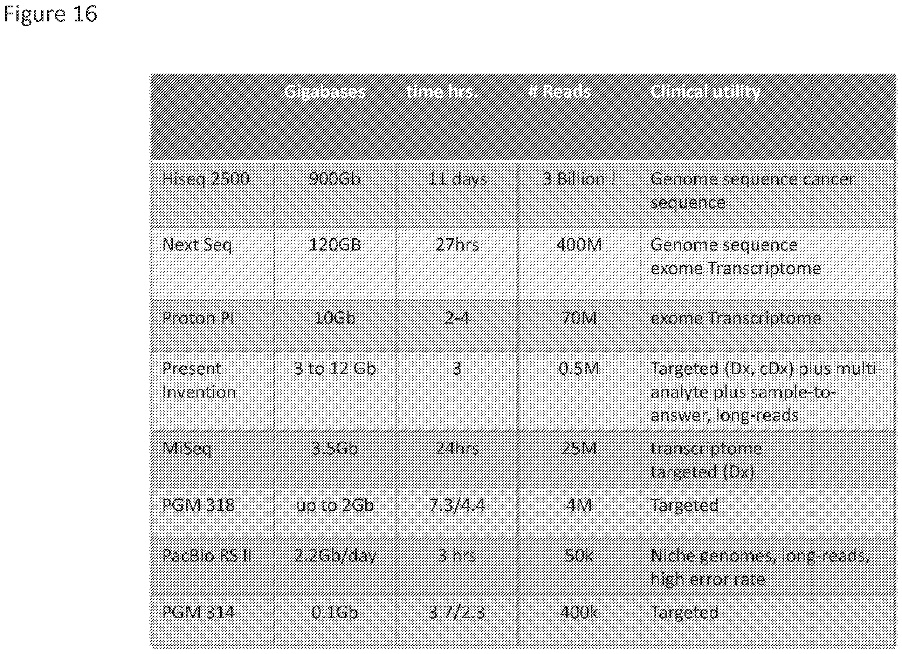

Figure 16 compares the sequencing rate, number of reads, and clinical utility for the present invention and various other sequencing methods/ apparatuses.

**Example 2: The method has a low error rate**

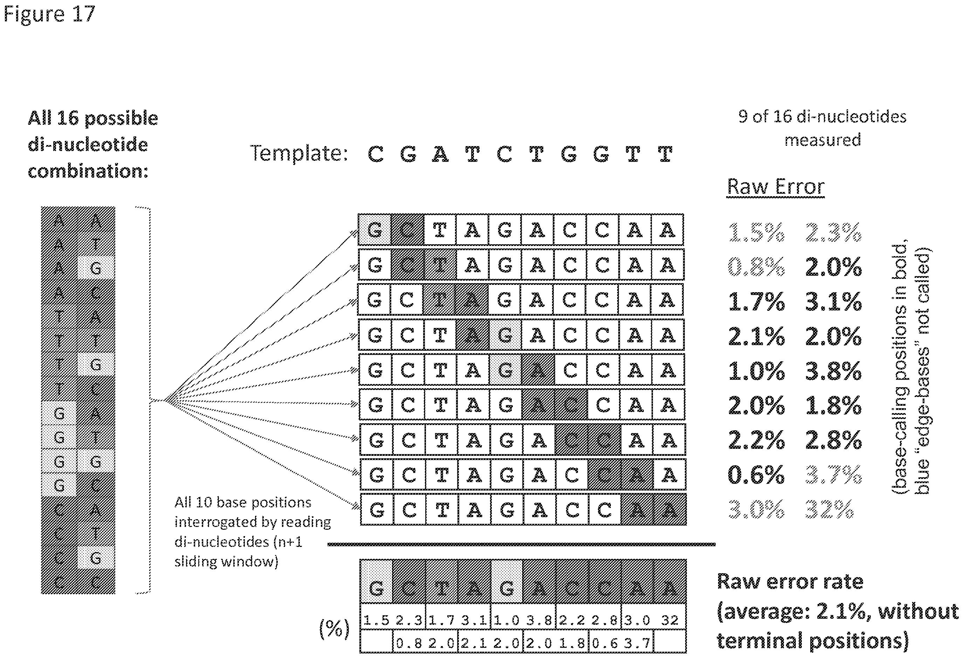

Figure 17 shows that the present invention has a raw error rate of about 2.1%, when terminal positions are omitted.

For the claimed invention, an error rate associated with sequencing is related to the free-energy difference between a fully-matched (m+n)-mer and a single-base mismatch (m-1+n)-mer. The sum of m+n is the number of nucleotides in a target binding domain and m represents the number of positions in a barcode domain. An estimate of the selectivity of hybridization can be made using the equation *(See,* Owczarzy, R. (2005), Biophys. Chem., 117:207-215 and Integrated DNA Technologies website: at the World Wide Web (www) idtdna.com/analyzer/Applications/Instructions/Default.aspx?AnalyzerDefinitions=true#Mismatc hMeltTemp):

$$\theta = 1 - \left( \frac{K_a([strand2] - [strand1]) - 1}{2K_a[strand2]} + \frac{\sqrt{K_a^2([strand1] - [strand2])^2 + 2K_a([strand1] + [strand2]) + 1}}{2K_a[strand2]} \right)$$

where $K_a$ is the association equilibrium constant obtained from predicted thermodynamic parameters,

$$K_a = \exp\left( \frac{-(\Delta H° - T\Delta S°)}{RT} \right)$$

Theta represents the percent bound of the exact complement and the single base mismatch sequences, which are expected to be annealed to target at the specified hybridization temperature. The T is the hybridization temperature in Kelvins, $\Delta H°$ (enthalpy) and $\Delta S°$ (entropy) are the melting parameters calculated from the sequence and the published nearest neighbor thermodynamic parameters, R is the ideal gas constant (1.987 cal $K^{-1}mole^{-1}$), [strand1/2] is the molar concentration of an oligonucleotide, and the constant of -273.15 converts temperature from Kelvin to degrees of Celsius. The most accurate, nearest-neighbor parameters were obtained from the following publications for DNA/DNA base pairs *(See,* Allawi,H., SantaLucia, J. Biochemistry, 36, 10581), RNA/DNA base pairs *(See,* Sugimoto et al., Biochemistry, 34, 11211-6), RNA/RNA base pairs *(See,* Xia,T. et al., Biochemistry, 37, 14719),

As example of an estimate of the approximate error-rate expected from the NSTG-sequencer follows. For (m + n) equals 8'mer. Consider the following 8-mer barcode and its single-base mismatch.

5'ATCGTACG3'
(region to sequence)
3'TAGCATGC5'
(sequencing optical barcode with perfect match)
3'TAGTATGC5'
(sequencing optical barcode with single-base mismatch (G-T) pairing)

Using the IDT calculator based upon the above equations yields:

At 17.4°C (the Tm of the perfect match case), (50% / 0.3%) would be the ratio of the correct optical barcode hybridized to that sequence versus the incorrect barcode at the Tm, yielding an estimated error rate for that sequence to be 0.6%.

A very high GC content sequencing calculation yields:

5'CGCCGGCC3'
(region to sequence)
3'GCGGCCGG5'
(sequencing optical barcode with perfect match)
3'GCGGACGG5'
(sequencing optical barcode with single-base mismatch (G-A) mis-pairing)

At 41.9°C (the Tm of the perfect match case), (50% / 0.4%) would be the ratio of the correct optical barcode hybridized to that sequence versus the incorrect barcode at the Tm, yielding an estimated error rate for that sequence to be 0.8%.

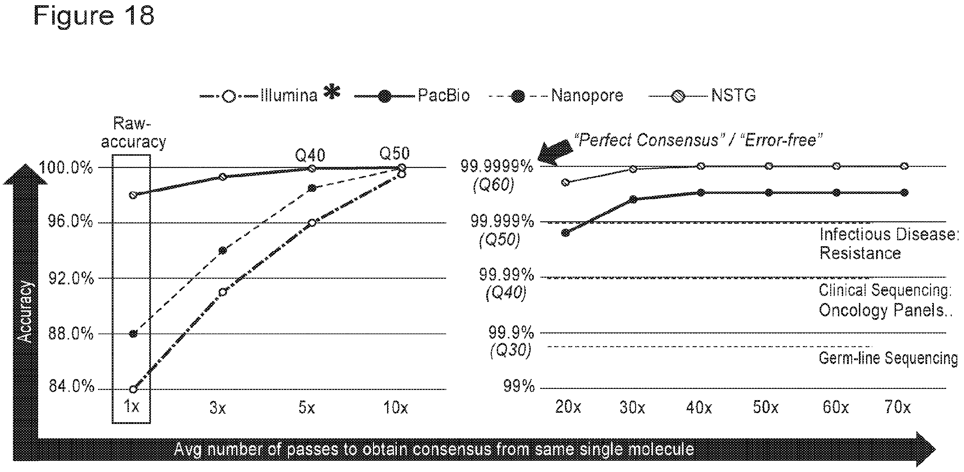

Examination of a number of 8-mer pairs yields a distribution of error rates, in the range of 0.2% to 1%. While the above calculations will not be identical to the conditions used, these calculations provide an indication that the method of the present invention will have a relatively low intrinsic error rate, when compared to other single-molecule sequencing technologies, such as Pacific Biosciences and Oxford Nanopore Technologies where error rates can be significant (>> 10%). Figure 18 demonstrates that the present invention's raw accuracy is higher than other sequencing methods. Thus, the present invention provides a consensus sequence from a single target after fewer passes than required for other sequencing methods. Additionally, the present invention may obtain "perfect consensus"/"error-free" sequencing (i.e., 99.9999%/Q60) after 30 or more passes whereas the PacBio sequencing methods (for example) cannot attain such a consensus after 70 passes.

**Example 3: Single base-pair resolution ability**

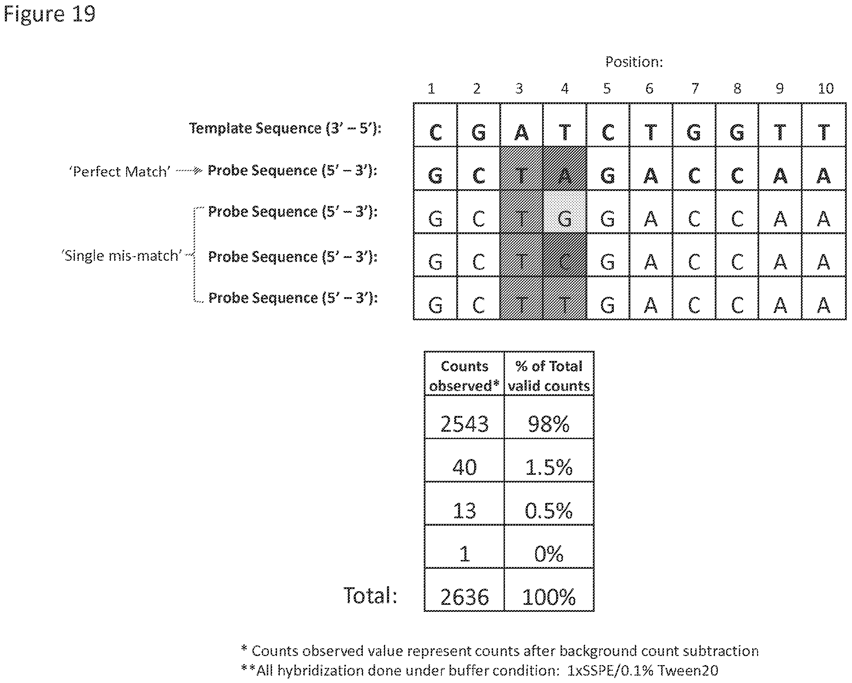

Figure 19 shows that the present invention has single-base resolution and with low error rates (ranging from 0% to 1.5% depending on a specific nucleotide substitution).

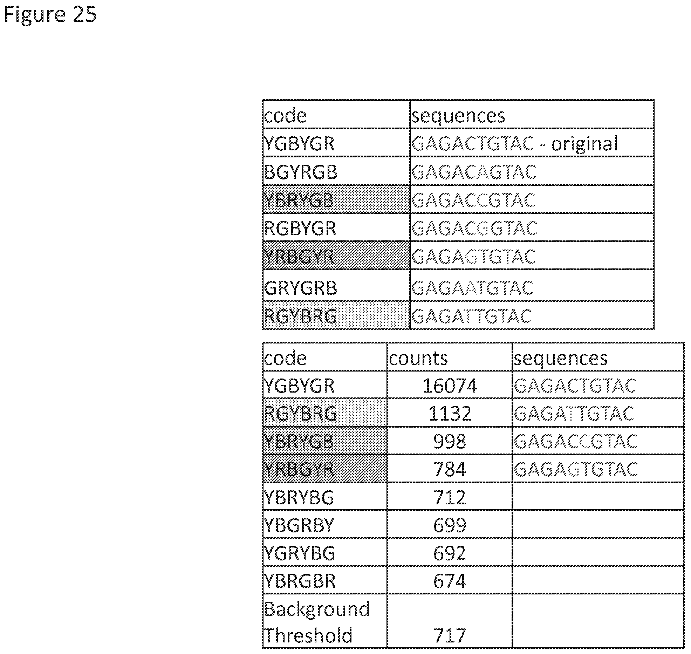

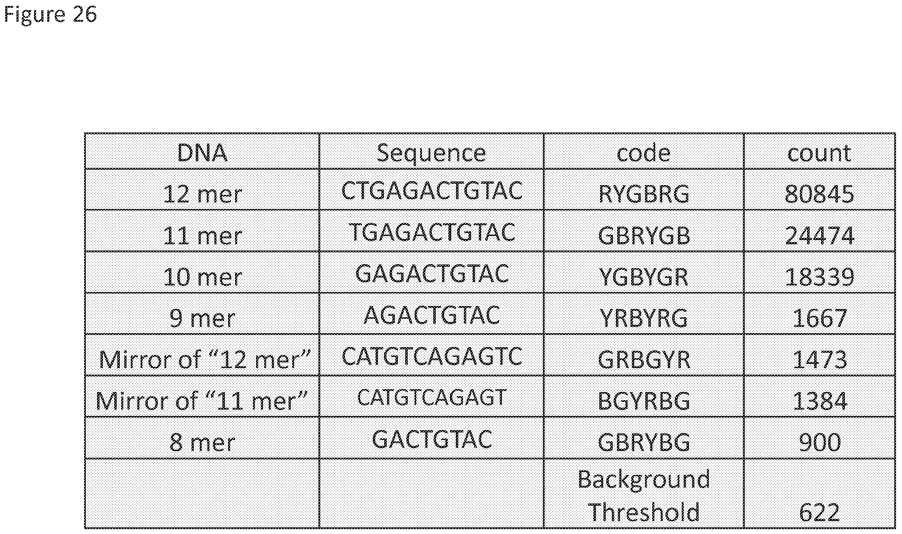

Additional experiments were performed using a target RNA hybridized with barcode and immobilized to the surface of cartridge using normal NanoString gene-expression binding technology (*see, e.g.,* Geiss et al, "Direct multiplexed measurement of gene expression with color-coded probe pairs"; Nature Biotechnology, 26, 317 - 325 (2008)). The ability of a barcode with different target binding domain length and with a perfect match (YGBYGR-2um optical bar code connected to perfect 10-mer match sequence) to hybridize to RNA-target was measured (Figure 26). Longer length of target binding domain gives higher counts. It also shows that 10-mer target binding domain is enough to register the sequence above background. Each of the individual single-base altered matches was synthesized with alternate optical bar codes. The ratio of correct to incorrect optical barcodes was counted (Figures 24 and 25).

Ability of 10mer to detect a SNP the real sequence is >15000 counts over background, whilst incorrect sequences are at most > 400 over background. In the presence of correct probe, error rates are expected to be <3% of real sequence. Note that this data is (in essence) a worse-case scenario. Having only a 10-base-pair hybridization sequence attached to a 6.6 Kilobase optical barcode reporter (Gen2 style). No specific condition optimizations were performed. This data, however, does reveal that the NanoString Next-Generation Sequencing approach is capable of resolving single-base pairs of sequence.

The detailed materials and methods utilized in the above study are as follows:

Hybridization Protocol Probe B plus codeset

- Take 25ul elements (194 codeset)
- Add 5ul Probe B+ complimentary sequence to target (100uM)
- Add 15ul Hyb Buffer (14.56 X SSPE 0.18% Tween 20)
  SSPE (150mM NaCl, NaH$_2$PO$_4$xH$_2$O 10mM, Na2EDTA 10mM)
- Incubate on ice for 10 min
- Add 150ul G beads(40ul G beads at 10mg/ml plus 110 ul 5x SSPE 0.1% Tween 20)
- Incubate for 10 min at RT
- Wash three times with 0.1SSPE 0.1% Tween 20 using magnet collector
- Elute in 100ul 0.1x SSPE for 10 min at 45C.

Target Hybridization protocol (750mM NaCl)

- Take 20 ul above eluted sample
- Add 10 ul hyb buffer
- Add 1ul Target (100nM biotinylated RNA)
- Incubate on ice for 30 min

Take 15ul and Bind to streptavidin slide for 20 min, flow stretch with G hooks, count using nCounter

Materials

Elements 194 codeset
Oligos bought from IDT
SSPE (150mM NaCl, NaH$_2$PO$_4$xH$_2$O 10mM, Na$_2$EDTA 10mM)

Hyb buffer (14.56 X SSPE 0.18% Tween 20)

Table 2: Probe B Sequences for 12, 11, .., 8 mers. (SEQ ID NO: 30 to SEQ ID NO: 34)

| GBRYBG | 5 | GACTGTACCCACGCGATGACGTTCGTCAAGAGTCGCATAATCT | 3 |
| YRBYRG | 5 | AGACTGTACCACAAGAATCCCTGCTAGCTGAAGGAGGGTCAAAC | 3 |
| YGBYGR | 5 | GAGACTGTACCCTACGTATATATCCAAGTGGTTATGTCCGACGGC | 3 |
| GBRYGB | 5 | TGAGACTGTACCACCCCTCCAAACGCATTCTTATTGGCAAATGGAA | 3 |
| RYGBRG | 5 | CTGAGACTGTACCCGGGAATCGGCATTTCGCATTCTTAGGATCTAAA | 3 |

Table 3: Target Sequence (in **Bold**; SEQ ID NO: 35)

| RNA | 5 | CAATGTGAGTCTCTTG**GTACAGTCTCAG**TTAGTCACTCCCTAAG\Bio TEG\ | 3 |

Table 4: Probe B Sequences for 10mer mismatches (in **Bold**; SEQ ID NO: 36 to SEQ ID NO: 41)

| 10mermis2A | GAGAC**A**GTACCCTGGTCTAGGTATCTAATTCGTGGGTCGGGTACT |
| 10mermis2C | GAGAC**C**GTACCGCTCATTTTGAACATACGATTGCGATTACGGAAA |
| 10mermis2G | GAGAC**G**GTACCTTAAAGCTATCCACGAATGTCAAAAATGTGGTTT |
| 1 Omermis 1G | GAGA**G**TGTACCCAATGCTTGCAGTATGTATCCTGATCGTGCGTGC |
| 10mermis1A | GAGA**A**TGTACCCTCATACCAATGTAAAGTATAGTTAACGCCCTGT |
| 1 0mermis 1T | GAGA**T**TGTACCCTACATATATAGGAAAAGGGAAGGTAGAAGAGCT |

## Claims

1. A sequencing probe comprising a target binding domain and a barcode domain;
    wherein said target binding domain comprises at least eight nucleotides and is capable of binding a target nucleic acid, and wherein at least two nucleotides in the target binding domain are universal bases capable of binding to any base within the target nucleic acid;
    wherein said barcode domain comprises a synthetic backbone, said barcode domain comprising at least six attachment positions, each attachment position comprising at least one attachment region, said attachment region comprising at least one nucleic acid sequence capable of being bound by a complementary nucleic acid molecule,

    wherein each attachment position of the at least six attachment positions corresponds to one nucleotide in the target binding domain and each of the at least six attachment positions have a different nucleic acid sequence, and
    wherein said nucleic acid sequence of each position of the at least six attachment positions determines the position and identity of the corresponding one nucleotide in said target nucleic acid that is bound by said target binding domain, and
    wherein the universal bases precede and follow the nucleotides corresponding to positions in the barcode domain.

2. The sequencing probe of claim 1, wherein said synthetic backbone comprises single-stranded DNA.

3. The sequencing probe of claim 1, wherein said sequencing probe comprises a doublestranded DNA spacer between the target binding domain and the barcode domain.

4. The sequencing probe of claim 1, wherein the number of nucleotides in a target binding domain is at least two more than the number of attachment regions in the barcode domain.

5. The sequencing probe of claim 1, wherein each position in a barcode domain has: (a) the same number of attachment regions; (b) one attachment region; or (c) more than one attachment region.

6. The sequencing probe of claim 1, wherein each complementary nucleic acid molecule;

   (a) for each attachment position comprises a detectable label;
   (b) or is indirectly linked to a primary nucleic acid molecule via a nucleic acid spacer; or
   (c) for each attachment position comprises between about 8 nucleotides and about 20, nucleotides, preferably, wherein each complementary nucleic acid molecule comprises about 12 nucleotides.

7. The sequencing probe of claim 6(b), wherein each primary nucleic acid molecule is hybridized to at least one, two, three, four or five secondary nucleic acid molecule.

8. The sequencing probe of claim 7, wherein the secondary nucleic acid molecule or molecules comprise at least one detectable label.

9. The sequencing probe of claim 7, wherein each secondary nucleic acid molecule is hybridized to at least one, two, three, four, five, six or seven tertiary nucleic acid molecule comprising at least one detectable label.

10. The sequencing probe of claim 1, wherein one or more attachment positions in the barcode domain are adjacent to at least one flanking single-stranded polynucleotide.

11. A population of sequencing probes comprising a plurality of the sequencing probe of claim 1.

12. A method for sequencing a nucleic acid comprising steps of:

   (1) hybridizing at least one first population of first sequencing probes comprising a plurality of the sequencing probe of any one of claims 1 to 10 to a target nucleic acid that is immobilized to a substrate, wherein the target nucleic acid is immobilized to the substrate at one or more positions;
   (2) binding a first complementary nucleic acid molecule comprising a detectable label or a first complementary nucleic acid molecule of a first reporter complex comprising a detectable label to a first attachment position of the at least six attachment positions;
   (3) detecting the detectable label of the bound first complementary nucleic acid molecule or the detectable label of the bound first complementary nucleic acid molecule of the first reporter complex;
   (4) identifying the position and identity of a first nucleotide in the immobilized target nucleic acid;
   (5) binding to the first attachment position a first hybridizing nucleic acid molecule lacking a detectable label, thereby unbinding the first complementary nucleic acid molecule comprising a detectable label or the first complementary nucleic acid molecule of the first reporter complex comprising a detectable label;
   (6) binding a second complementary nucleic acid molecule comprising a detectable label or a second complementary nucleic acid molecule of a second reporter complex comprising a detectable label to a second attachment position of the at least six attachment positions;
   (7) detecting the detectable label of the bound second complementary nucleic acid molecule or the detectable label of the bound second complementary nucleic acid molecule of the second reporter complex;
   (8) identifying the position and identity of a second nucleotide in the immobilized target nucleic acid;
   (9) repeating steps (5) to (8) until each attachment position of the at least six attachment positions have been bound by a complementary nucleic acid molecule comprising a detectable label or a complementary nucleic acid molecule of a reporter complex comprising a detectable label, and the detectable label of the bound complementary nucleic acid molecule or the detectable label of the bound complementary nucleic acid molecule of a reporter complex has been detected, thereby identifying the linear order of at least six nucleotides for at least a first region of the immobilized target nucleic acid that was hybridized by the target binding domain of the sequencing probe; and

(10) de-hybridizing the at least one first population of first sequencing probes from the immobilized target nucleic acid.

13. The method of claim 12, wherein steps (5) and (6) occur sequentially or concurrently.

14. The method of claim 12, wherein the first complementary nucleic acid molecule and the first hybridizing nucleic acid molecule lacking a detectable label comprise the same nucleic acid sequence.

15. The method of claim 12, wherein the first hybridizing nucleic acid molecule lacking a detectable label comprises a nucleic acid sequence complementary to a flanking single-stranded polynucleotide adjacent to the first attachment position.

16. The method of claim 12, further comprising

(11) hybridizing at least one second population of second sequencing probes comprising a plurality of the sequencing probes of any one of claims 1 to 10 to a target nucleic acid that is immobilized to a substrate, wherein the target nucleic acid is immobilized to the substrate at one or more positions, wherein the target binding domain of the first sequencing probe and the second sequencing probe are different, and wherein each sequencing probe in the second population de-hybridizes from the immobilized target nucleic acid under about the same conditions; and de-hybridizes from the immobilized target nucleic acid under different conditions than the sequencing probes in the first population;

(12) binding a first complementary nucleic acid molecule comprising a detectable label or a first complementary nucleic acid molecule of a first reporter complex comprising a detectable label to a first attachment position of the at least six attachment positions;

(13) detecting the detectable label of the bound first complementary nucleic acid molecule or the detectable label of the bound first complementary nucleic acid molecule of the first reporter complex;

(14) identifying the position and identity of a first nucleotide in the immobilized target nucleic acid;

(15) binding to the first attachment position a first hybridizing nucleic acid molecule lacking a detectable label, thereby unbinding the first complementary nucleic acid molecule comprising a detectable label or the first complementary nucleic acid molecule of the first reporter complex comprising a detectable label;

(16) binding a second complementary nucleic acid molecule comprising a detectable label or a second complementary nucleic acid molecule of a second reporter complex comprising a detectable label to a second attachment position of the at least six attachment positions;

(17) detecting the detectable label of the bound second complementary nucleic acid molecule or the detectable label of the bound second complementary nucleic acid molecule of the second reporter complex;

(18) identifying the position and identity of a second nucleotide in the immobilized target nucleic acid;

(19) repeating steps (15) to (18) until each attachment position of the at least six attachment positions have been bound by a complementary nucleic acid molecule comprising a detectable label or a complementary nucleic acid molecule of a reporter complex comprising a detectable label, and the detectable label of the bound complementary nucleic acid molecule or the detectable label of the bound complementary nucleic acid molecule of a reporter complex has been detected, thereby identifying the linear order of at least six nucleotides for at least a second region of the immobilized target nucleic acid that was hybridized by the target binding domain of the sequencing probe; and

(20) de-hybriding the at least one second population of second sequencing probes from the immobilized target nucleic acid.

17. The method of claim 16, further comprising steps of assembling each identified linear order of nucleotides in the at least first region and at least second region of the immobilized target nucleic acid, thereby identifying a sequence for the immobilized target nucleic acid.

18. A kit comprising a substrate, a plurality of sequencing probes of any of claims 1 to 10, at least two capture probes, wherein a capture probe comprises a nucleic acid sequence that hybridizes to a region of a target nucleic acid that is different than the region of the target nucleic acid that hybridizes to a sequencing probe and an affinity tag, at least six complementary nucleic acid molecules comprising a detectable label, wherein a complementary nucleic acid comprising a detectable label comprises a nucleic acid sequence that hybridizes to one of the at least six attachment positions of a sequencing probe and a detectable label,
at least six complementary nucleic acid molecules which lack a detectable label, wherein a complementary nucleic acid which lacks a detectable label comprises a nucleic acid sequence that hybridizes to one of the at least six attachment positions of a sequencing probe,
and instructions for use.

## Patentansprüche

1. Sequenzierungssonde, die eine Zielbindungsdomäne und eine Barcode-Domäne umfasst;
   wobei die Zielbindungsdomäne mindestens acht Nukleotide umfasst und eine Zielnukleinsäure binden kann und wobei mindestens zwei Nukleotide in der Zielbindungsdomäne universelle Basen sind, die jedwede Base innerhalb der Zielnukleinsäure binden können;
   wobei die Barcode-Domäne eine synthetische Hauptkette umfasst, die Barcode-Domäne mindestens sechs Anbringungspositionen umfasst, wobei jede Anbringungsposition mindestens eine Anbringungsregion umfasst, wobei die Anbringungsregion mindestens eine Nukleinsäuresequenz umfasst, die durch ein komplementäres Nukleinsäuremolekül gebunden werden kann,

   wobei jede Anbringungsposition der mindestens sechs Anbringungspositionen einem Nukleotid in der Zielbindungsdomäne entspricht und jede der mindestens sechs Anbringungspositionen eine unterschiedliche Nukleinsäuresequenz aufweist und
   wobei die Nukleinsäuresequenz von jeder Position der mindestens sechs Anbringungspositionen die Position und Identität des entsprechenden einen Nukleotids in der Zielnukleinsäure, die durch die Zielbindungsdomäne gebunden ist, bestimmt und
   wobei die universellen Basen den Nukleotiden, die den Positionen in der Barcode-Domäne entsprechen, vorausgehen und folgen.

2. Sequenzierungssonde nach Anspruch 1, wobei die synthetische Hauptkette einzelsträngige DNA umfasst.

3. Sequenzierungssonde nach Anspruch 1, wobei die Sequenzierungssonde einen doppelsträngigen DNA-Spacer zwischen der Zielbindungsdomäne und der Barcode-Domäne umfasst.

4. Sequenzierungssonde nach Anspruch 1, wobei die Anzahl an Nukleotiden in einer Zielbindungsdomäne mindestens zwei mehr als die Anzahl an Anbringungsregionen in der Barcode-Domäne beträgt.

5. Sequenzierungssonde nach Anspruch 1, wobei jede Position in einer Barcode-Domäne Folgendes aufweist: (a) die gleiche Anzahl an Anbringungsregionen; (b) eine Anbringungsregion; oder (c) mehr als eine Anbringungsregion.

6. Sequenzierungssonde nach Anspruch 1, wobei jedes komplementäre Nukleinsäuremolekül

   (a) für jede Anbringungsposition eine nachweisbare Markierung umfasst;
   (b) oder indirekt mit einem primären Nukleinsäuremolekül über einen Nukleinsäure-Spacer verknüpft ist; oder
   (c) für jede Anbringungsposition zwischen etwa 8 Nukleotide und etwa 20 Nukleotide umfasst, bevorzugt wobei jedes komplementäre Nukleinsäuremolekül etwa 12 Nukleotide umfasst.

7. Sequenzierungssonde nach Anspruch 6 (b), wobei jedes primäre Nukleinsäuremolekül mit mindestens einem, zwei, drei, vier oder fünf sekundären Nukleinsäuremolekülen hybridisiert ist.

8. Sequenzierungssonde nach Anspruch 7, wobei das sekundäre Nukleinsäuremolekül oder die sekundären Nukleinsäuremoleküle mindestens eine nachweisbare Markierung umfassen.

9. Sequenzierungssonde nach Anspruch 7, wobei jedes sekundäre Nukleinsäuremolekül mit mindestens einem, zwei, drei, vier, fünf, sechs oder sieben tertiären Nukleinsäuremolekülen hybridisiert ist, die mindestens eine nachweisbare Markierung umfassen.

10. Sequenzierungssonde nach Anspruch 1, wobei eine oder mehrere Anbringungspositionen in der Barcode-Domäne mindestens einem flankierenden einzelsträngigen Polynukleotid benachbart sind.

**11.** Population von Sequenzierungssonden, die eine Vielzahl der Sequenzierungssonde nach Anspruch 1 umfassen.

**12.** Verfahren zur Sequenzierung einer Nukleinsäure, das die folgenden Schritte umfasst:

(1) Hybridisieren von mindestens einer ersten Population von ersten Sequenzierungssonden, die eine Vielzahl der Sequenzierungssonde nach einem der Ansprüche 1 bis 10 umfassen, mit einer Zielnukleinsäure, die an einem Substrat immobilisiert ist, wobei die Zielnukleinsäure an dem Substrat an einer oder mehreren Positionen immobilisiert ist;

(2) Binden eines ersten komplementären Nukleinsäuremoleküls, das eine nachweisbare Markierung umfasst, oder eines ersten komplementären Nukleinsäuremoleküls eines ersten Reporterkomplexes, der eine nachweisbare Markierung umfasst, zu einer ersten Anbringungsposition der mindestens sechs Anbringungspositionen;

(3) Nachweisen der nachweisbaren Markierung des gebundenen ersten komplementären Nukleinsäuremoleküls oder der nachweisbaren Markierung des gebundenen ersten komplementären Nukleinsäuremoleküls des ersten Reporterkomplexes;

(4) Identifizieren der Position und Identität eines ersten Nukleotids in der immobilisierten Zielnukleinsäure;

(5) Binden zu der ersten Anbringungsposition eines ersten hybridisierenden Nukleinsäuremoleküls, dem eine nachweisbare Markierung fehlt, dadurch Lösen des ersten komplementären Nukleinsäuremoleküls, das eine nachweisbare Markierung umfasst, oder des ersten komplementären Nukleinsäuremoleküls des ersten Reporterkomplexes, der eine nachweisbare Markierung umfasst;

(6) Binden eines zweiten komplementären Nukleinsäuremoleküls, das eine nachweisbare Markierung umfasst, oder eines zweiten komplementären Nukleinsäuremoleküls eines zweiten Reporterkomplexes, der eine nachweisbare Markierung umfasst, zu einer zweiten Anbringungsposition der mindestens sechs Anbringungspositionen;

(7) Nachweisen der nachweisbaren Markierung des gebundenen zweiten komplementären Nukleinsäuremoleküls oder der nachweisbaren Markierung des gebundenen zweiten komplementären Nukleinsäuremoleküls des zweiten Reporterkomplexes;

(8) Identifizieren der Position und Identität eines zweiten Nukleotids in der immobilisierten Zielnukleinsäure;

(9) Wiederholen der Schritte (5) bis (8) bis jede Anbringungsposition der mindestens sechs Anbringungspositionen durch ein komplementäres Nukleinsäuremolekül, das eine nachweisbare Markierung umfasst, oder ein komplementäres Nukleinsäuremolekül eines Reporterkomplexes, der eine nachweisbare Markierung umfasst, gebunden wurde und die nachweisbare Markierung des gebundenen komplementären Nukleinsäuremoleküls oder die nachweisbare Markierung des gebundenen komplementären Nukleinsäuremoleküls eines Reporterkomplexes nachgewiesen wurde, dadurch Identifizieren der linearen Reihenfolge von mindestens sechs Nukleotiden für mindestens eine erste Region der immobilisierten Zielnukleinsäure, die durch die Zielbindungsdomäne der Sequenzierungssonde hybridisiert wurde; und

(10) Dehybridisieren der mindestens einen ersten Population von ersten Sequenzierungssonden von der immobilisierten Zielnukleinsäure.

**13.** Verfahren nach Anspruch 12, wobei die Schritte (5) und (6) nacheinander oder gleichzeitig erfolgen.

**14.** Verfahren nach Anspruch 12, wobei das erste komplementäre Nukleinsäuremolekül und das erste hybridisierende Nukleinsäuremolekül, dem eine nachweisbare Markierung fehlt, die gleiche Nukleinsäuresequenz umfassen.

**15.** Verfahren nach Anspruch 12, wobei das erste hybridisierende Nukleinsäuremolekül, dem eine nachweisbare Markierung fehlt, eine Nukleinsäuresequenz umfasst, die einem flankierenden einzelsträngigen Polynukleotid, das benachbart der ersten Anbringungsposition vorliegt, komplementär ist.

**16.** Verfahren nach Anspruch 12, das weiter Folgendes umfasst:

(11) Hybridisieren von mindestens einer zweiten Population von zweiten Sequenzierungssonden, die eine Vielzahl der Sequenzierungssonden nach einem der Ansprüche 1 bis 10 umfassen, mit einer Zielnukleinsäure, die an einem Substrat immobilisiert ist, wobei die Zielnukleinsäure an dem Substrat an einer oder mehreren Positionen immobilisiert ist, wobei die Zielbindungsdomäne der ersten Sequenzierungssonde und der zweiten Sequenzierungssonde unterschiedlich sind und wobei jede Sequenzierungssonde in der zweiten Population von der immobilisierten Zielnukleinsäure unter etwa den gleichen Bedingungen dehybridisiert und von der immobilisierten Zielnukleinsäure unter anderen Bedingungen als die Sequenzierungssonden in der ersten Population dehybridisiert;

(12) Binden eines ersten komplementären Nukleinsäuremoleküls, das eine nachweisbare Markierung umfasst, oder eines ersten komplementären Nukleinsäuremolküls eines ersten Reporterkomplexes, der eine nachweisbare Markierung umfasst, zu einer ersten Anbringungsposition der mindestens sechs Anbringungspositionen;

(13) Nachweisen der nachweisbaren Markierung des gebundenen ersten komplementären Nukleinsäuremolküls oder der nachweisbaren Markierung des gebundenen ersten komplementären Nukleinsäuremolküls des ersten Reporterkomplexes;

(14) Identifizieren der Position und Identität eines ersten Nukleotids in der immobilisierten Zielnukleinsäure;

(15) Binden zu der ersten Anbringungsposition eines ersten hybridisierenden Nukleinsäuremolküls, dem eine nachweisbare Markierung fehlt, dadurch Lösen des ersten komplementären Nukleinsäuremolküls, das eine nachweisbare Markierung umfasst, oder des ersten komplementären Nukleinsäuremolküls des ersten Reporterkomplexes, der eine nachweisbare Markierung umfasst;

(16) Binden eines zweiten komplementären Nukleinsäuremolküls, das eine nachweisbare Markierung umfasst, oder eines zweiten komplementären Nukleinsäuremolküls eines zweiten Reporterkomplexes, der eine nachweisbare Markierung umfasst, zu einer zweiten Anbringungsposition der mindestens sechs Anbringungspositionen;

(17) Nachweisen der nachweisbaren Markierung des gebundenen zweiten komplementären Nukleinsäuremolküls oder der nachweisbaren Markierung des gebundenen zweiten komplementären Nukleinsäuremolküls des zweiten Reporterkomplexes;

(18) Identifizieren der Position und Identität eines zweiten Nukleotids in der immobilisierten Zielnukleinsäure;

(19) Wiederholen der Schritte (15) bis (18) bis jede Anbringungsposition der mindestens sechs Anbringungspositionen durch ein komplementäres Nukleinsäuremolekül, das eine nachweisbare Markierung umfasst, oder ein komplementäres Nukleinsäuremolekül eines Reporterkomplexes, der eine nachweisbare Markierung umfasst, gebunden wurde und die nachweisbare Markierung des gebundenen komplementären Nukleinsäuremolküls oder die nachweisbare Markierung des gebundenen komplementären Nukleinsäuremolküls eines Reporterkomplexes nachgewiesen wurde, dadurch Identifizieren der linearen Reihenfolge von mindestens sechs Nukleotiden für mindestens eine zweite Region der immobilisierten Zielnukleinsäure, die durch die Zielbindungsdomäne der Sequenzierungssonde hybridisiert wurde; und

(20) Dehybridisieren der mindestens einen zweiten Population von zweiten Sequenzierungssonden von der immobilisierten Zielnukleinsäure.

**17.** Verfahren nach Anspruch 16, das weiter Schritte des Zusammenstellens von jeder identifizierten linearen Reihenfolge von Nukleotiden in der mindestens ersten Region und mindestens zweiten Region der immobilisierten Zielnukleinsäure, dadurch Identifizieren einer Sequenz für die immobilisierte Zielnukleinsäure umfasst.

**18.** Kit, der Folgendes umfasst: ein Substrat, eine Vielzahl von Sequenzierungssonden nach einem der Ansprüche 1 bis 10, mindestens zwei Einfangsonden, wobei eine Einfangsonde Folgendes umfasst: eine Nukleinsäuresequenz, die mit einer Region einer Zielnukleinsäure hybridisiert, die sich von der Region der Zielnukleinsäure, die mit einer Sequenzierungssonde hybridisiert, unterscheidet, und einen Affinitäts-Tag,

mindestens sechs komplementäre Nukleinsäuremoleküle, die eine nachweisbare Markierung umfassen, wobei eine komplementäre Nukleinsäure, die eine nachweisbare Markierung umfasst, Folgendes umfasst: eine Nukleinsäuresequenz, die mit einer der mindestens sechs Anbringungspositionen einer Sequenzierungssonde hybridisiert, und eine nachweisbare Markierung,

mindestens sechs komplementäre Nukleinsäuremoleküle, denen eine nachweisbare Markierung fehlt, wobei eine komplementäre Nukleinsäure, der eine nachweisbare Markierung fehlt, eine Nukleinsäuresequenz umfasst, die mit einer der mindestens sechs Anbringungspositionen einer Sequenzierungssonde hybridisiert, und eine Gebrauchsanweisung.

## Revendications

**1.** Sonde de séquençage comprenant un domaine de liaison à une cible et un domaine à code-barres ;

où ledit domaine de liaison à une cible comprend au moins huit nucléotides et est capable de se lier à un acide nucléique cible, et où au moins deux nucléotides du domaine de liaison à une cible sont des bases universelles capables de se lier à n'importe quelle base de l'acide nucléique cible ;

où ledit domaine à code-barres comprend un squelette synthétique, ledit domaine à code-barres comprenant au moins six positions d'attache, chaque position d'attache comprenant une au moins région d'attache, ladite région d'attache comprenant une au moins séquence d'acide nucléique capable d'être liée par une molécule d'un acide nucléique complémentaire,

où chaque position d'attache des au moins six positions d'attache correspond à un nucléotide du domaine de liaison à une cible et où chacune des au moins six positions d'attache a une séquence d'acide nucléique différente et

où ladite séquence d'acide nucléique de chaque position des au moins six positions d'attache détermine la position et l'identité du un nucléotide correspondant dudit acide nucléique cible qui est lié par ledit domaine de liaison à une cible et

où les bases universelles précèdent et suivent les nucléotides correspondant à des positions dans le domaine à code-barres.

2. Sonde de séquençage de la revendication 1, où ledit squelette synthétique comprend un ADN simple brin.

3. Sonde de séquençage de la revendication 1, où ladite sonde de séquençage comprend un ADN double brin espaceur entre le domaine de liaison à une cible et le domaine à code-barres.

4. Sonde de séquençage de la revendication 1, où le nombre de nucléotides d'un domaine de liaison à une cible dépasse de deux au moins le nombre de régions d'attache dans le domaine à code-barres.

5. Sonde de séquençage de la revendication 1, où chaque position dans un domaine à code-barres a : (a) le même nombre de régions d'attache ; (b) une région d'attache ; ou (c) plus d'une région d'attache.

6. Sonde de séquençage de la revendication 1, où chaque molécule d'un acide nucléique complémentaire

(a) comprend une étiquette détectable pour chaque position d'attache ; ou
(b) est indirectement liée à une molécule d'un acide nucléique primaire par l'intermédiaire d'un acide nucléique espaceur ; ou
(c) comprend entre environ 8 nucléotides et environ 20 nucléotides pour chaque position d'attache, préférablement où chaque molécule d'un acide nucléique complémentaire comprend environ 12 nucléotides.

7. Sonde de séquençage de la revendication 6(b), où chaque molécule d'un acide nucléique primaire est hybridée à une, deux, trois, quatre ou cinq au moins molécules d'un acide nucléique secondaire.

8. Sonde de séquençage de la revendication 7, où la molécule ou les molécules d'un acide nucléique secondaire comprennent une au moins étiquette détectable.

9. Sonde de séquençage de la revendication 7, où chaque molécule d'un acide nucléique secondaire est hybridée à une, deux, trois, quatre, cinq, six ou sept au moins molécules d'un acide nucléique tertiaire comprenant une au moins étiquette détectable.

10. Sonde de séquençage de la revendication 1, où une ou plusieurs positions d'attache dans le domaine à code-barres sont adjacentes à un au moins polynucléotide simple brin flanquant.

11. Population de sondes de séquençage comprenant une pluralité de la sonde de séquençage de la revendication 1.

12. Méthode de séquençage d'un acide nucléique comprenant les étapes suivantes :

(1) hybridation de une au moins première population de premières sondes de séquençage comprenant une pluralité de la sonde de séquençage de l'une quelconque des revendications 1 à 10 à un acide nucléique cible qui est immobilisé sur un substrat, où l'acide nucléique cible est immobilisé sur le substrat au niveau de une ou plusieurs positions ;
(2) liaison d'une molécule d'un premier acide nucléique complémentaire comprenant une étiquette détectable ou d'une molécule d'un premier acide nucléique complémentaire d'un premier complexe rapporteur comprenant une étiquette détectable à une première position d'attache des au moins six positions d'attache ;
(3) détection de l'étiquette détectable de la molécule liée du premier acide nucléique complémentaire ou de l'étiquette détectable de la molécule liée du premier acide nucléique complémentaire du premier complexe rapporteur ;
(4) identification de la position et de l'identité d'un premier nucléotide de l'acide nucléique cible immobilisé ;
(5) liaison, au niveau de la première position d'attache, d'une molécule d'un premier acide nucléique hybridant exempt d'une étiquette détectable, détachant ainsi la molécule du premier acide nucléique complémentaire comprenant une étiquette détectable ou la molécule du premier acide nucléique complémentaire du premier complexe rapporteur comprenant une étiquette détectable ;

(6) liaison d'une molécule d'un deuxième acide nucléique complémentaire comprenant une étiquette détectable ou d'une molécule d'un deuxième acide nucléique complémentaire d'un deuxième complexe rapporteur comprenant une étiquette détectable à une deuxième position d'attache des au moins six positions d'attache ;

(7) détection de l'étiquette détectable de la molécule liée du deuxième acide nucléique complémentaire ou de l'étiquette détectable de la molécule liée du deuxième acide nucléique complémentaire du deuxième complexe rapporteur ;

(8) identification de la position et de l'identité d'un deuxième nucléotide de l'acide nucléique cible immobilisé ;

(9) répétition des étapes (5) à (8) jusqu'à ce que chaque position d'attache des au moins six positions d'attache ait été liée par une molécule d'un acide nucléique complémentaire comprenant une étiquette détectable ou une molécule d'un acide nucléique complémentaire d'un complexe rapporteur comprenant une étiquette détectable et jusqu'à ce que l'étiquette détectable de la molécule liée de l'acide nucléique complémentaire ou l'étiquette détectable de la molécule liée de l'acide nucléique complémentaire d'un complexe rapporteur ait a été détectée, identifiant ainsi la séquence linéaire de au moins six nucléotides pour une au moins première région de l'acide nucléique cible immobilisé qui a été hybridé par le domaine de liaison à une cible de la sonde de séquençage ; et

(10) déshybridation de la une au moins première population de premières sondes de séquençage de l'acide nucléique cible immobilisé.

13. Méthode de la revendication 12, où les étapes (5) et (6) sont effectuées en succession ou simultanément.

14. Méthode de la revendication 12, où la molécule du premier acide nucléique complémentaire et la molécule du premier acide nucléique hybridant exempt d'une étiquette détectable comprennent la même séquence d'acide nucléique.

15. Méthode de la revendication 12, où la molécule du premier acide nucléique hybridant exempt d'une étiquette détectable comprend une séquence d'acide nucléique complémentaire à un polynucléotide simple brin flanquant adjacent à la première position d'attache.

16. Méthode de la revendication 12, qui comprend en outre les étapes suivantes :

(11) hybridation de une au moins deuxième population de deuxièmes sondes de séquençage comprenant une pluralité des sondes de séquençage de l'une quelconque des revendications 1 à 10 à un acide nucléique cible qui est immobilisé sur un substrat, où l'acide nucléique cible est immobilisé sur le substrat au niveau de une ou plusieurs positions, où les domaines de liaison à une cible de la première sonde de séquençage et de la deuxième sonde de séquençage sont différents et où chaque sonde de séquençage de la deuxième population est déshybridée de l'acide nucléique cible immobilisé dans des conditions à peu près identiques ; et déshybridée de l'acide nucléique cible immobilisé dans des conditions différentes de celles appliquées pour les sondes de séquençage de la première population ;

(12) liaison d'une molécule d'un premier acide nucléique complémentaire comprenant une étiquette détectable ou d'une molécule d'un premier acide nucléique complémentaire d'un premier complexe rapporteur comprenant une étiquette détectable à une première position d'attache des au moins six positions d'attache ;

(13) détection de l'étiquette détectable de la molécule liée du premier acide nucléique complémentaire ou de l'étiquette détectable de la molécule liée du premier acide nucléique complémentaire du premier complexe rapporteur ;

(14) identification de la position et de l'identité d'un premier nucléotide de l'acide nucléique cible immobilisé ;

(15) liaison, au niveau de la première position d'attache, d'une molécule d'un premier acide nucléique hybridant exempt d'une étiquette détectable, détachant ainsi la molécule du premier acide nucléique complémentaire comprenant une étiquette détectable ou la molécule du premier acide nucléique complémentaire du premier complexe rapporteur comprenant une étiquette détectable ;

(16) liaison d'une molécule d'un deuxième acide nucléique complémentaire comprenant une étiquette détectable ou d'une molécule d'un deuxième acide nucléique complémentaire d'un deuxième complexe rapporteur comprenant une étiquette détectable à une deuxième position d'attache des au moins six positions d'attache ;

(17) détection de l'étiquette détectable de la molécule liée du deuxième acide nucléique complémentaire ou de l'étiquette détectable de la molécule liée du deuxième acide nucléique complémentaire du deuxième complexe rapporteur ;

(18) identification de la position et de l'identité d'un deuxième nucléotide de l'acide nucléique cible immobilisé ;

(19) répétition des étapes (15) à (18) jusqu'à ce que chaque position d'attache des au moins six positions d'attache ait été liée par une molécule d'un acide nucléique complémentaire comprenant une étiquette détectable ou une molécule d'un acide nucléique complémentaire d'un complexe rapporteur comprenant une étiquette détectable et jusqu'à ce que l'étiquette détectable de la molécule liée de l'acide nucléique complémentaire ou l'étiquette détectable de la molécule liée de l'acide nucléique complémentaire d'un complexe rapporteur ait a été détectée, identifiant ainsi la séquence linéaire de au moins six nucléotides pour une au moins deuxième région de l'acide nucléique cible immobilisé qui a été hybridé par le domaine de liaison à une cible de la sonde de séquençage ; et

(20) déshybridation de la une au moins deuxième population de deuxièmes sondes de séquençage de l'acide nucléique cible immobilisé.

17. Méthode de la revendication 16, comprenant en outre des étapes d'assemblage de chaque séquence de nucléotides linéaire identifiée dans la au moins première région et la au moins deuxième région de l'acide nucléique cible immobilisé, identifiant ainsi une séquence pour l'acide nucléique cible immobilisé.

18. Kit comprenant un substrat, une pluralité de sondes de séquençage de l'une quelconque des revendications 1 à 10, au moins deux sondes de capture, où une sonde de capture comprend une séquence d'acide nucléique qui s'hybride à une région d'un acide nucléique cible qui est différente de la région de l'acide nucléique cible qui s'hybride à une sonde de séquençage et une étiquette d'affinité,

au moins six molécules d'un acide nucléique complémentaire comprenant une étiquette détectable, où un acide nucléique complémentaire comprenant une étiquette détectable comprend une séquence d'acide nucléique qui s'hybride à une des au moins six positions d'attache d'une sonde de séquençage et une étiquette détectable,

au moins six molécules d'un acide nucléique complémentaire exempt d'une étiquette détectable, où un acide nucléique complémentaire exempt d'une étiquette détectable comprend une séquence d'acide nucléique qui s'hybride à une des au moins six positions d'attache d'une sonde de séquençage, et

un mode d'emploi.

Figure 1

Figure 2

Figure 3

Figure 4

Figure 5

EP 3 221 469 B1

Figure 6A

$n_1$  $n_2$  1st  2nd  3rd  4th  5th  6th  $n_3$  $n_4$

Figure 6B

EP 3 221 469 B1

Figure 6C

EP 3 221 469 B1

Figure 6D

Figure 7

10 specific bases: $4^{10}$ ~ 1 million probes

8 specific bases: $4^8$ ~ 65 thousand probes

6 specific bases: $4^6$ ~ 4 thousand probes

$U_b$ = universal base (e.g., Inosine and nitro-indole )

## Figure 8A

Sequence complementary to first attachment region

First pool of complementary nucleic acids comprising detectable labels

or

First pool of reporter complexes comprising detectable labels

Base specific detectable label
or
Reporter complex comprising a base specific detectable label

First Sequencing Probe

1st  2nd  3rd  4th  5th  6th  n  n  n  n

T

Target Nucleic Acid
(comprising a T)

Figure 8B

Sequence
complementary to
first attachment
region

First pool of
hybridizing nucleic
acids lacking
Detectable labels

No detectable label

Target Nucleic Acid
(comprising a T)

1st 2nd 3rd 4th 5th 6th n n n n

T

EP 3 221 469 B1

Figure 8C

Second pool of complementary
nucleic acids comprising
detectable labels
or
Second pool of reporter
complexes comprising
detectable labels

Sequence
complementary to
second attachment
region

Base specific
detectable label
or
Reporter complex
comprising a base
specific detectable
label

1st  2nd  3rd  4th  5th  6th  n  n  n  n

T  C

Target Nucleic Acid
(comprising a T - C)

EP 3 221 469 B1

Figure 8D

Sixth pool of complementary nucleic acids comprising detectable labels

or

Sixth pool of reporter complexes comprising detectable labels

Sequence complementary to sixth attachment region

Base specific detectable label

or

Reporter complex comprising a base specific detectable label

1st 2nd 3rd 4th 5th 6th n n n n

T C A G T G

Target Nucleic Acid (comprising a T – C – A- G- T- G)

Figure 8E

Second Sequencing Probe

1st 2nd 3rd 4th 5th 6th n n n n

T C A G T G

(Same) Target Nucleic Acid

EP 3 221 469 B1

Figure 9A

Sample DNA
hybridized to &
immobilized
via biotinylated DNA

Streptavidin-hydrogel on glass slide

Cycle 1: → probe Hyb

Figure 9B

Reporter complex comprising a detectable label

Secondary nucleic
acid molecule

Tertiary nucleic
acid molecule

Detectable Label

Primary nucleic
acid molecule

Complementary
nucleic acid molecule

EP 3 221 469 B1

# Figure 9C

| Attachment Region-1 Reporter Complexes |
|---|

Position 1_Adenine (AR-1_A)  
Position 1_Cytidine (AR-1_C)  
Position 1_Guanine (AR-1_G)  
Position 1_Thymine (AR-1_T)

| Attachment Region-2 Reporter Complexes |
|---|

Position 2_Adenine (AR-2_A)  
Position 2_Cytidine (AR-2_C)  
Position 2_Guanine (AR-2_G)  
Position 2_Thymine (AR-2_T)

| Attachment Region-3 Reporter Complexes |
|---|

Position 3_Adenine (AR-3_A)  
Position 3_Cytidine (AR-3_C)  
Position 3_Guanine (AR-3_G)  
Position 3_Thymine (AR-3_T)

| Attachment Region-4 Reporter Complexes |
|---|

Position 4_Adenine (AR-4_A)  
Position 4_Cytidine (AR-4_C)  
Position 4_Guanine (AR-4_G)  
Position 4_Thymine (AR-4_T)

| Attachment Region-5 Reporter Complexes |
|---|

Position 5_Adenine (AR-5_A)  
Position 5_Cytidine (AR-5_C)  
Position 5_Guanine (AR-5_G)  
Position 5_Thymine (AR-5_T)

| Attachment Region-6 Reporter Complexes |
|---|

Position 6_Adenine (AR-6_A)  
Position 6_Cytidine (AR-6_C)  
Position 6_Guanine (AR-6_G)  
Position 6_Thymine (AR-6_T)

# Figure 9D

Detectable fluorescence
(Position 1 = Thymine)

Cycle 1: → probe Hyb →

Position 1 Spot Hyb
(Image)

Figure 9E

# Figure 9F

Cycle 1: → probe Hyb → Position 1 Spot Hyb → Position 2 Spot Hyb → → → → Position 6 Spot Hyb
(Image)                    (Image)                      (Image)

Figure 9G

Cycle 2: → Second probe Hyb

## Figure 10

| | Target Binding Domain | (Optional) dsDNA spacer | Flank 1 | AR-1 | AR-1/ Flank 2 | AR- 2 | AR-2/ Flank 3 | AR-3 | AR-3/ Flank 4 | AR-4 | AR-4/ Flank 5 |
|---|---|---|---|---|---|---|---|---|---|---|---|
| V2 Design | TTCACTGTAG | CTGTCTCATTTTGCTGCATCCTGTCCGTTCACGTTG | GAGCTT | GTCATC | CGTCCT | CTTTTC | ACTCCT | AGGCAT | TTGCCT | ATTCGG | CGTCCT |

· · ·

Position 2 encodes 'G'  Position 1 encodes 'T'

Step 1:    AR-1 Detect

Step 2:    Lack 1

Step 3:    AR-2 Detect

Flank = Flanking single-stranded polynucleotide
AR = Attachment Region
Detect = Complementary Nucleic Acid Comprising Detectable Label
Lack = Hybridizing Nucleic Acid Lacking Detectable Label

EP 3 221 469 B1

# Figure 11

| | Domain | (Optional) dsDNA spacer | Flank 1 | AR-1 | AR-1/ Flank 2 | AR- 2 | AR-2/ Flank 3 | AR-3 | AR-3/ Flank 4 | AR-4 | AR-4/ Flank 5 |
|---|---|---|---|---|---|---|---|---|---|---|---|
| V2 Design | TTCACTGTAG | CTGTCTCATTTTGCTGCATCCTGTCCGTTCACGTTG | GAGCTT | GTCATC | CGTCCT | CTTTTC | ACTCCT | AGGCAT | TTGCCT | ATTCGG | CGTCCT | ...

Step 1:　　　　　AR-1 Detect

Step 2:　　Lack 1　+　AR-2 Detect

Step 3:　　　　Lack 2　+　AR-3 Detect

Step 4:　　　　　Lack 3　+　AR-4 Detect

........

........

........

Step N:

# Figure 12

# Figure 13

**Complete sequencing work-flow (sample to answer)**

| Extract NA | Target Enrichment | Library Prep | Sequence | Analyze & Report |

*Cancer Panel Example*

**Ion Torrent (AmpliSeq)**

Multiplex PCR (90 minutes) → Partial Digestion (45 minutes) → Adaptor Ligation (45 minutes) → Limited PCR (30 minutes) → Cleanup & Library Concentration Normalization (2 hours) → Library QC (45 minutes) → ePCR & Enrich (5 hours) → Chip Loading (30 minutes)

*Total time: 12 hrs [HOT ~ 3.5 hrs (manual), 0.5 hr (automated on Ion Chef)]*

**Illumina (TruSight)**

Multiplex PCR (90 minutes) → Cleanup (45 minutes) → Indexing PCR (90 minutes) → Cleanup & Library Concentration Normalization (2.5 hours) → Library QC (45 minutes) → Bridge PCR (Clustering on flow-cell) (1.5 hours)

*Total time: 9 hrs [HOT ~ 4 hrs (manual), 0.5 hr (automated on NeoPrep)]*

**Present Invention**

Fragment sample* (30 minutes) → Target Capture to flow-cell (1.5 hours)

*Total time: 2 hrs (HOT ~ 15 minutes, automated)*

*Need for fragmentation will be dependent on sequencing application (i.e. long-read apps such as HLA sequencing will not require this step)

# Figure 14

Complete sequencing work-flow (sample to answer)

Extract NA >> Target Enrichment >> Library Prep >> Sequence* >> Analyze & Report

Run-time calculator based on sequencing application

| | | | |
|---|---|---|---|
| Technical Attributes | Raw Accuracy (%) | 98 | |
| | Read length (bp) | - | |
| Application specific seq requirements | # of Target regions | 100 | |
| | Target region size (bp) | 100 | |
| | Variant detection threshold | 0.01 | |
| | # of Target copies required (X) | 3000 | |
| | Total # of Reads required (M) | 0.3 | |
| Probes detecting 6 nucleotide positions | Total reads per FOV | 250000 | |
| | # of FOV required | 1.2 | |
| | Total number of hyb&seq cycle | 430 | |
| | Hardware motion time (s) | 0.5 | |
| | Exposure time (s) | 0.2 | |
| | Focus time (s) | 0.3 | |
| | Total imaging time per FOV (s) | 1 | |
| | Total # of spot per hyb&seq cycle | 6 | |
| | k-mer probe hyb time (s) | 5 | |
| | Spot exchange time (s) | 3 | |
| | Wash time (s) | 5 | |
| | Total chemistry time per hyb&seq cycle (s) | 28 | |
| | Total seq run time* (hr) | 4.62 | |

Run-times:

100-plex = 4.6 hrs

1000-plex = 16 hrs

* Sequencing run-time includes the time for primary data processing (i.e. basecalling, QV assignment, probe label readout counting & binning)

EP 3 221 469 B1

# Figure 15

Cycling calculator based on probe occupancy

| | | |
|---|---|---|
| Total # of single molecule targets on flow-cell | 30000 | |
| Average length of 'gapped' target (bp) | 100 | |
| | | |
| Barcode occupancy (%) per seq cycle | 0.75 | |
| # of barcode cycle required to 'hit' >95% of targets on flow-cell at least once | 3 | |
| | | |
| Dye occupancy (%) per position | 0.8 | |
| Total number of Dye positions | 6 | |
| Total # of 1 bp encoded read per seq cycle | 6 | 35 |
| Total # of 2 bp encoded read per seq cycle | 23 | 346 |
| Total # of 3 bp encoded read per seq cycle | 92 | 1843 |
| Total # of 4 bp encoded read per seq cycle | 369 | 5530 |
| Total # of 5 bp encoded read per seq cycle | 1475 | 8847 |
| Total # of 6 bp encoded read per seq cycle | 5898 | 5898 |
| Total number of 'usable' read per seq cycle | | 22499 |
| | | |
| # of barcode cycle required to cover given single molecule target with ~3X coverage | 143 | |
| # of barcode cycle required to cover given single molecule target with ~5X coverage | 238 | |
| | | |
| # of barcode cycle required to cover all single molecule target on flow-cell with ~3X coverage | 430 | |
| # of barcode cycle required to cover all single molecule target on flow-cell with ~5X coverage | 713 | |

Figure 16

| | Gigabases | time hrs. | # Reads | Clinical utility |
|---|---|---|---|---|
| Hiseq 2500 | 900Gb | 11 days | 3 Billion I | Genome sequence cancer sequence |
| Next Seq | 120GB | 27hrs | 400M | Genome sequence exome Transcriptome |
| Proton PI | 10Gb | 2-4 | 70M | exome Transcriptome |
| Present Invention | 3 to 12 Gb | 3 | 0.5M | Targeted (Dx, cDx) plus multi-analyte plus sample-to-answer, long-reads |
| MiSeq | 3.5Gb | 24hrs | 25M | transcriptome targeted (Dx) |
| PGM 318 | up to 2Gb | 7.3/4.4 | 4M | Targeted |
| PacBio RS II | 2.2Gb/day | 3 hrs | 50k | Niche genomes, long-reads, high error rate |
| PGM 314 | 0.1Gb | 3.7/2.3 | 400k | Targeted |

EP 3 221 469 B1

# Figure 17

**All 16 possible di-nucleotide combination:**

Template: **C G A T C T G G T T**

**9 of 16 di-nucleotides measured**

**Raw Error**

| | | | | | | | | | | | |
|---|---|---|---|---|---|---|---|---|---|---|---|
| G | C | T | A | G | A | C | C | A | A | 1.5% | 2.3% |
| G | C | T | A | G | A | C | C | A | A | 0.8% | **2.0%** |
| G | C | T | A | G | A | C | C | A | A | **1.7%** | **3.1%** |
| G | C | T | A | G | A | C | C | A | A | **2.1%** | **2.0%** |
| G | C | T | A | G | A | C | C | A | A | **1.0%** | **3.8%** |
| G | C | T | A | G | A | C | C | A | A | **2.0%** | **1.8%** |
| G | C | T | A | G | A | C | C | A | A | **2.2%** | **2.8%** |
| G | C | T | A | G | A | C | C | A | A | **0.6%** | 3.7% |
| G | C | T | A | G | A | C | C | A | A | 3.0% | 32% |

All 10 base positions interrogated by reading di-nucleotides (n+1 sliding window)

(base-calling positions in bold, blue "edge-bases" not called)

| | G | C | T | A | G | A | C | C | A | A |
|---|---|---|---|---|---|---|---|---|---|---|
| (%) | 1.5 | 2.3 1.7 | 3.1 1.0 | 3.8 2.2 | 2.8 3.0 | 32 | | | | |
| | | 0.8 2.0 | 2.1 2.0 | 2.0 1.8 | 0.6 3.7 | | | | | |

**Raw error rate (average: 2.1%, without terminal positions)**

Figure 18

# Figure 19

Position:

| | 1 | 2 | 3 | 4 | 5 | 6 | 7 | 8 | 9 | 10 |
|---|---|---|---|---|---|---|---|---|---|---|
| Template Sequence (3' – 5'): | C | G | A | T | C | T | G | G | T | T |
| 'Perfect Match' → Probe Sequence (5' – 3'): | G | C | T | A | G | A | C | C | A | A |
| Probe Sequence (5' – 3'): | G | C | T | G | G | A | C | C | A | A |
| Probe Sequence (5' – 3'): | G | C | T | C | G | A | C | C | A | A |
| Probe Sequence (5' – 3'): | G | C | T | T | G | A | C | C | A | A |

'Single mis-match'

| | Counts observed* | % of Total valid counts |
|---|---|---|
| | 2543 | 98% |
| | 40 | 1.5% |
| | 13 | 0.5% |
| | 1 | 0% |
| Total: | 2636 | 100% |

* Counts observed value represent counts after background count subtraction
**All hybridization done under buffer condition: 1xSSPE/0.1% Tween20

# Figure 20A

**5x5**

(1 primary nucleic acid molecule, 5 secondary nucleic
acid molecules, 5 tertiary nucleic acid molecule with
detectable label(s))

3'

12
bases

**4x3**

3'

12
bases

**3x4**

3'

12
bases

**Spacer 3x4**

3'

12        Spacer region
bases   (20-40 bases)

# Figure 20B

**Average Counts / FOV**

Categories: 5x5, 4x3, 3x4, 3spx4

⊠ G-Channel Singlets > 16

# Figure 20C

Exemplary recipe for constructing reporter complexes comprising detectable labels

| Volume (uL) | Primary n. a. molecule (10uM) | Secondary n. a. molecule (10uM) | Tertiary n. a. molecule (100uM) | H20 |
|---|---|---|---|---|
| 5x4 | 1 | 4.5 | 2.25 | 92.25 |
| 5x3 | 1 | 4.5 | 1.8 | 92.7 |
| 4x4 | 1.28 | 4.5 | 2.25 | 91.97 |
| 4x3 | 1.28 | 4.5 | 1.8 | 92.42 |
| 3x4 | 1.8 | 4.5 | 2.25 | 91.45 |

Colors in table correspond to colors in diagrams of complexes shown at left

EP 3 221 469 B1

Figure 21A

4x4 'extra-handles'

7x3 'extra-handles'

12mer

12 mer

12 mer

12 mer

12 mer

3'

12 mer

12mer

12mer

3'

12mer

Figure 21B

**Average Counts / FOV**

□ G-Channel Singlets > 16

EP 3 221 469 B1

Figure 22A

'5x3' Kinetics

Figure 22B

'7x3 extra handles' Kinetics

Figure 23

Target Nucleic Acid

Figure 24

Sequencing
Imaging Area

On-Card
Fluidics and
Processing

Off-Card
Reagents

# Figure 25

| code | sequences |
|---|---|
| YGBYGR | GAGACTGTAC - original |
| BGYRGB | GAGACAGTAC |
| YBRYGB | GAGACCGTAC |
| RGBYGR | GAGACGGTAC |
| YRBGYR | GAGAGTGTAC |
| GRYGRB | GAGAATGTAC |
| RGYBRG | GAGATTGTAC |

| code | counts | sequences |
|---|---|---|
| YGBYGR | 16074 | GAGACTGTAC |
| RGYBRG | 1132 | GAGATTGTAC |
| YBRYGB | 998 | GAGACCGTAC |
| YRBGYR | 784 | GAGAGTGTAC |
| YBRYBG | 712 | |
| YBGRBY | 699 | |
| YGRYBG | 692 | |
| YBRGBR | 674 | |
| Background Threshold | 717 | |

Figure 26

EP 3 221 469 B1

| DNA | Sequence | code | count |
|---|---|---|---|
| 12 mer | CTGAGACTGTAC | RYGBRG | 80845 |
| 11 mer | TGAGACTGTAC | GBRYGB | 24474 |
| 10 mer | GAGACTGTAC | YGBYGR | 18339 |
| 9 mer | AGACTGTAC | YRBYRG | 1667 |
| Mirror of "12 mer" | CATGTCAGAGTC | GRBGYR | 1473 |
| Mirror of "11 mer" | CATGTCAGAGT | BGYRBG | 1384 |
| 8 mer | GACTGTAC | GBRYBG | 900 |
| | | Background Threshold | 622 |

Figure 27

**Full length Reporter**

*20 pM* full length (6 spot)  *33* counts/fov

**Single Spot Reporter**

| Single spot [fM] | Counts/FOV |
|---|---|
| 20,000 | Saturated |
| 2,000 | 12000 |
| 200 | 308 |
| *20* | *40* |
| 2 | 11 |

20pM NS reporter $\simeq$ 20fM Single spot

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013055995 A2
- US 7745129 B1
- US 4757141 A
- US 5151507 A
- US 5091519 A
- US 5188934 A
- US 5366860 A
- US 5847162 A
- US 4318846 A
- US 5800996 A
- US 5066580 A, Lee
- US 5688648 A
- US 6322901 B
- US 6576291 B
- US 6423551 B
- US 6251303 B
- US 6319426 B
- US 6426513 B
- US 6444143 B
- US 5990479 A
- US 6207392 B
- US 20020045045 A
- US 20030017264 A
- US 4230558 A
- US 4811218 A
- US 8148512 B
- US 7473767 B
- US 7919237 B
- US 7941279 B
- US 8415102 B
- US 8492094 B
- US 8519115 B
- US 20090220978 A
- US 20090299640 A
- US 20100015607 A
- US 20100261026 A
- US 20110086774 A
- US 20110145176 A
- US 20110201515 A
- US 20110229888 A
- US 20130004482 A
- US 20130017971 A
- US 20130178372 A
- US 20130230851 A
- US 20130337444 A
- US 20130345161 A
- US 20140005067 A
- US 20140017688 A
- US 20140037620 A
- US 20140087959 A
- US 20140154681 A
- US 20140162251 A
- US 62082883 B

**Non-patent literature cited in the description**

- **SUGIMOTO et al.** *Biochemistry,* vol. 34, 11211-6
- **SMALL ; PARTHASARTHY.** Superresolution localization methods. *Annu. Rev. Phys Chem.,* 2014, vol. 65, 107-25
- **LIN et al.** Submicrometre geometrically encoded fluorescent barcodes self-assembled from DNA. *Nature Chemistry,* October 2012, vol. 4 (10), 832-9
- **JUNGMANN et al.** Multiplexed 3D cellular super-resolution imaging with DNA-PAINT and Exchange-PAINT. *Nature Methods,* 2014, vol. 11 (3
- **HAUGLAND.** Handbook of Fluorescent Probes and Research Chemicals. Molecular Probes, Inc, 2002
- **KELLER ; MANAK.** DNA Probes. Stockton Press, 1993
- Oligonucleotides and Analogues: A Practical Approach. IRL Press, 1991
- **WETMUR.** *Critical Reviews in Biochemistry and Molecular Biology,* 1991, vol. 26, 227-259
- **HENEGARIU et al.** *Nature Biotechnol.,* 2000, vol. 18, 345
- **LAKOWICZ et al.** *BioTechniques,* 2003, vol. 34, 62
- **WHEELESS et al.** Flow Cytometry: Instrumentation and Data Analysis. Academic Press, 1985, 21-76
- **SEELING et al.** Catalyzed Relaxation of a Metastable DNA Fuel. *J. Am. Chem. Soc.,* 2006, vol. 128 (37), 12211-12220
- **SAMBROOK ; FRITSCHE ; MANIATIS.** Molecular Cloning A Laboratory Manual. Cold Spring Harbor Press, 1989

- **ANDERSON.** Nucleic Acid Hybridization. BIOS Scientific Publishers Limited, 1999
- **WAJID ; SERPEDIN.** Review of general algorithmic features for genome assemblers for next generation sequencers. *Genomics, proteomics & bioinformatics,* 2012, vol. 10 (2), 58-73
- Toe-Hold. *Probes*
- **OWCZARZY, R.** *Biophys. Chem.,* 2005, vol. 117, 207-215
- **ALLAWI,H. ; SANTALUCIA, J.** *Biochemistry,* vol. 36, 10581
- **XIA,T. et al.** *Biochemistry,* vol. 37, 14719
- **GEISS et al.** Direct multiplexed measurement of gene expression with color-coded probe pairs. *Nature Biotechnology,* 2008, vol. 26, 317-325